# EUROPEAN PATENT APPLICATION

(11) **EP 4 335 866 A1**
(43) Date of publication of application: **13.03.2024**
(21) Application number: 22194874.8
(22) Date of filing: 09.09.2022
(51) Int. Cl.: C07K 14/24, A01N 63/00, C12N 1/06, C12N 1/20, C12N 1/38

(54) **MATERIALS AND METHODS TO PRODUCE INSECTICIDAL TOXINS**

(71) Applicant: Max-Planck-Gesellschaft zur Förderung der Wissenschaften e.V., 80539 München (DE)
(72) Inventor: SITSEL, Oleg, 44202 Dortmund (DE); RAUNSER, Stefan, 44202 Dortmund (DE)
(74) Representative: Vossius & Partner Patentanwälte Rechtsanwälte mbB

(57) **Abstract**

The present invention relates to modified bacteria of the species *Yersinia entomophaga* which are useful to produce toxins and pesticides such as insecticides. The present invention further relates to a toxin composition and a purified insecticidal YenTc toxin susceptible for mass production. The present invention also relates to methods for producing a modified *Yersinia entomophaga* bacterium, an insecticidal toxin composition, a purified insecticidal YenTc toxin and uses thereof.

## Description

The present invention relates to modified bacteria of the species *Yersinia entomophaga* which are useful to produce toxins and pesticides such as insecticides. The present invention further relates to a toxin composition and a purified insecticidal YenTc toxin susceptible for mass production. The present invention also relates to methods for producing a modified *Yersinia entomophaga* bacterium, an insecticidal toxin composition, a purified insecticidal YenTc toxin and uses thereof.

Sprays containing insecticidal toxins have been used for protection of crops since the 1920s, when French farmers started using the entomopathogen *Bacillus thuringiensis* (Bt) with its active Cry toxin ingredient to control flour moths⁷⁸. Unlike many classes of chemical pesticides which have since been developed and phased out due to emerging resistance or health concerns, this biopesticide is still in active use a century later⁷⁹. However, the intense global use of Bt has caused resistant pest populations to emerge, with a large assessment of recent studies identifying 5 of 13 major pest species as having resistant populations⁸⁰. One of the best strategies for mitigating this would be to deploy novel insecticidal biotoxins unrelated to the Bt cry toxin. Unfortunately, very few novel biotoxins can compete with production levels, potency and specificity of the Bt cry toxin.

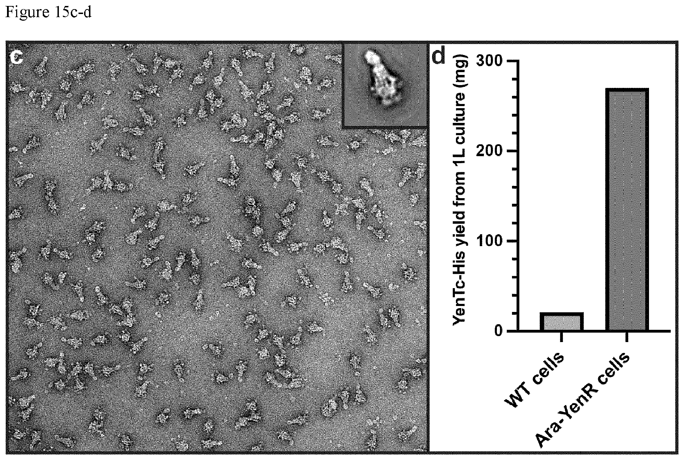

One such example would be the deadly mix of insecticidal toxins produced by the bacterial species *Yersinia entomophaga* (a risk group 1 organism⁸¹ harmless to humans but extremely lethal to insects²⁶). However, *Yersinia entomophaga* naturally produces only a very limited amount of toxin, which greatly limits the potential to produce the toxins in an efficient amount e.g. for pest control. For example, a 100 ml culture of *Yersinia entomophaga* results in only 2 mg of YenTc, which is not sufficient for high throughput production on an industrial scale, e.g. for application as a pesticide, such as insecticide⁸² (see also **Fig. 15d****).**

Thus, there is a need to provide means and methods to increase toxin production of a *Yersinia entomophaga* bacterium. The technical problem underlying the present invention is the improved production of a *Yersinia entomophaga* toxin or toxin composition.

The technical problem is solved by provision of the embodiments characterized in the claims and as provided herein below. Specifically, the technical problem is solved, and the above-mentioned difficulties are overcome by the provision of a *Yersinia entomophaga* bacterium comprising a modified YenR gene.

Accordingly, the present invention provides a *Yersinia entomophaga* bacterium comprising a modified YenR gene.

Furthermore, the invention provides a toxin composition comprising Cbp, Chi1, Chi2, Chi3, NucA, Pil36, PirA, PirB, StcE, Tlh, YenA1, YenA2, YenB, YenC1, YenC2, and RHS2.

Further, the invention provides a method to produce a toxin composition comprising a step of culturing a *Yersinia entomophaga* bacterium of the invention.

The invention also provides toxin composition obtained by or as obtainable by the methods of the present invention.

Furthermore, the invention provides a *Yersinia entomophaga* bacterium of the present invention, further comprising a modified gene encoding a YenTc subunit or a modified YenTc subunit.

The invention also provides a method to produce a purified YenTc toxin comprising a step of culturing a *Yersinia entomophaga* bacterium of the present invention.

Further, the invention provides a purified YenTc toxin obtained by or as obtainable by the methods of the present invention.

The invention also provides a YenTc toxin comprising a purification tag.

The invention also provides a method to induce secretion of a toxin or toxin composition comprising
(i) culturing a *Yersinia entomophaga* bacterium of the present invention, at an initial pH of about 7.0 until a desired number of bacteria is grown; and
(ii) incubating the bacteria of (i) at pH 6.3-10.0, preferably pH 6.9-8.9, or transferring the bacteria to a pH 6.3-10.0 buffer, preferably pH 6.9-8.9, thereby inducing the secretion of the toxin.

The invention also provides a population of soldier cells, wherein the soldier cells are *Yersinia entomophaga* bacteria, wherein the expression of the YenR gene or gene products thereof is increased, optionally, wherein the expression of the YmoA gene or gene products thereof is increased and/or wherein the soldier cells comprise the toxin composition and/or one or more of the toxin(s) according to the invention, preferably, wherein the population comprises the *Yersinia entomophaga* bacterium of the invention,
optionally wherein the expression of the YenR gene or gene products thereof is directly or indirectly increased, further optionally, wherein the expression of the YmoA gene or gene products thereof is directly or indirectly increased. The term "population of soldier cells" refers to a population comprising or consisting (essentially) of soldier cells.

The invention also provides a method to produce a soldier cell comprising,
(i) increasing the expression of the YenR gene or gene products thereof in a *Yersinia entomophaga* bacterium, optionally increasing the expression of the YmoA gene or gene products thereof;
(ii) culturing the *Yersinia entomophaga* bacterium of (i) at an initial pH of about 7.0 until a desired number of bacteria is grown.

The invention also provides a method to kill pests comprising contacting a pest with a *Yersinia entomophaga* bacterium of the present invention or the population of soldier cells of the present invention.

The invention also provides a method to kill pests comprising contacting a pest with a toxin composition of the present invention or a YenTc toxin of the present invention.

The invention also provides a use of a *Yersinia entomophaga* bacterium of the present invention or the population of soldier cells of the present invention as a pesticide.

The invention also provides a use of a toxin composition of the present invention or a YenTc toxin of the present invention as an insecticide.

The invention also provides a use of a *Yersinia entomophaga* bacterium of the present invention or the population of soldier cells of the present invention to produce a toxin composition, such as a pesticide or insecticide.

The invention also provides a use of a *Yersinia entomophaga* bacterium of the present invention or the population of soldier cells of the present invention to produce a YenTc toxin.

The invention also provides a population of cells comprising the *Yersinia entomophaga* bacterium of the present invention.

The invention also provides a modified YenR gene as defined herein, specifically a modified YenR gene comprising the sequence set forth in SEQ ID NO: 2.

The invention also provides a plasmid comprising the sequence set forth in SEQ ID NO: 3.

The invention provides, inter alia, the following advantages:
- A *Yersinia entomophaga* bacterium comprising a modified YenR gene allows to create a population in which all cells produce and optionally secrete a toxin composition and, thus, produces a tremendously increased amount of toxins
- The secretion mechanism of a *Yersinia entomophaga* bacterium is dependent on pH which allows for long time storage and toxin secretion on demand, especially when combined with a *Yersinia entomophaga* bacterium comprising a modified YenR gene which produces an increased amount of toxins
- A *Yersinia entomophaga* bacterium comprising a modified YenR gene can further be modified to comprise a toxin with a purification tag which allows for high yield production of said toxin combined with a high purity
- A *Yersinia entomophaga* bacterium, a toxin composition, or a purified toxin of the invention can be applied on any surfaces where an insecticidal effect is desired

In the following the advantages are described in more detail.

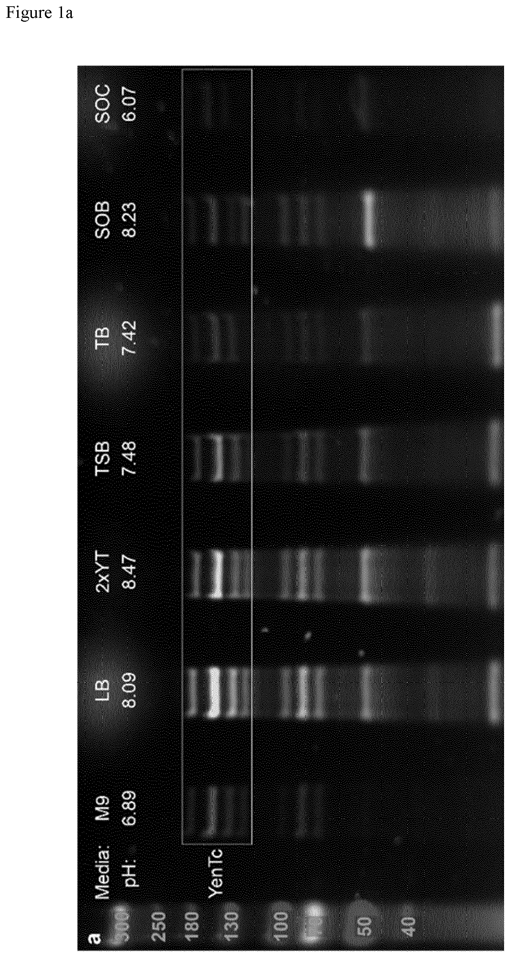

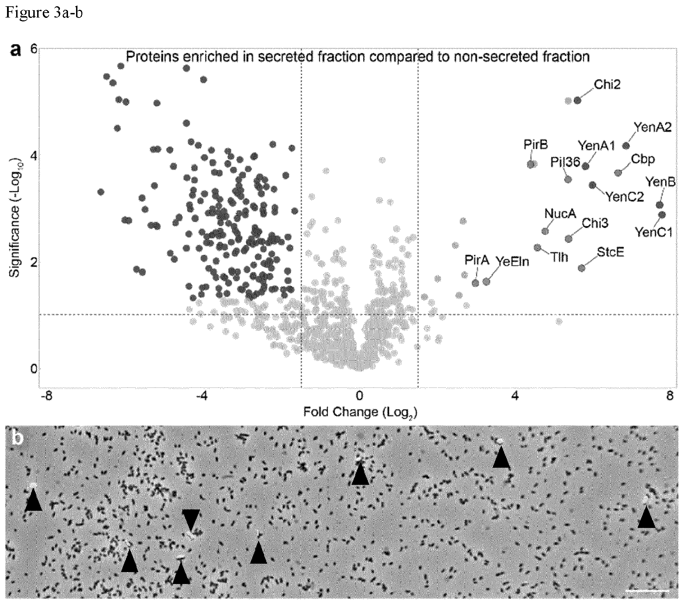

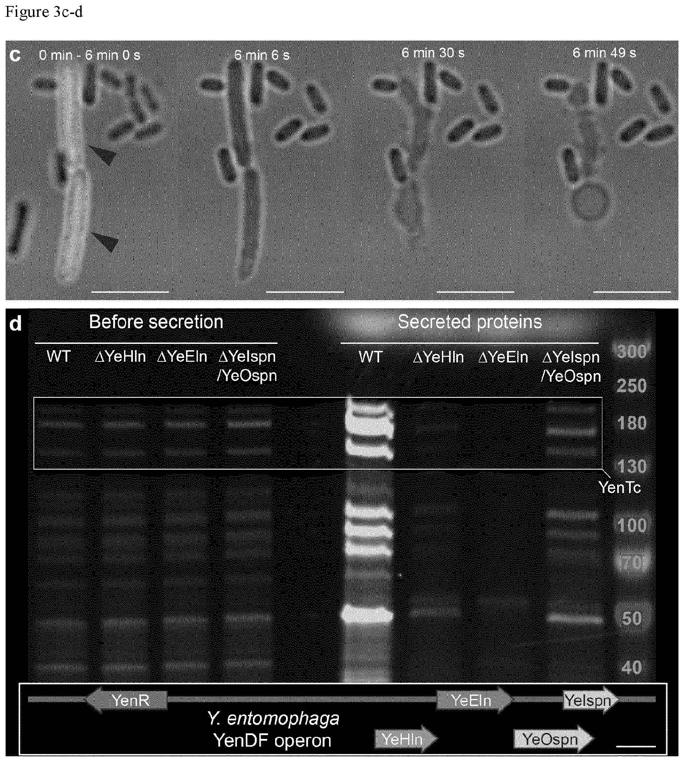

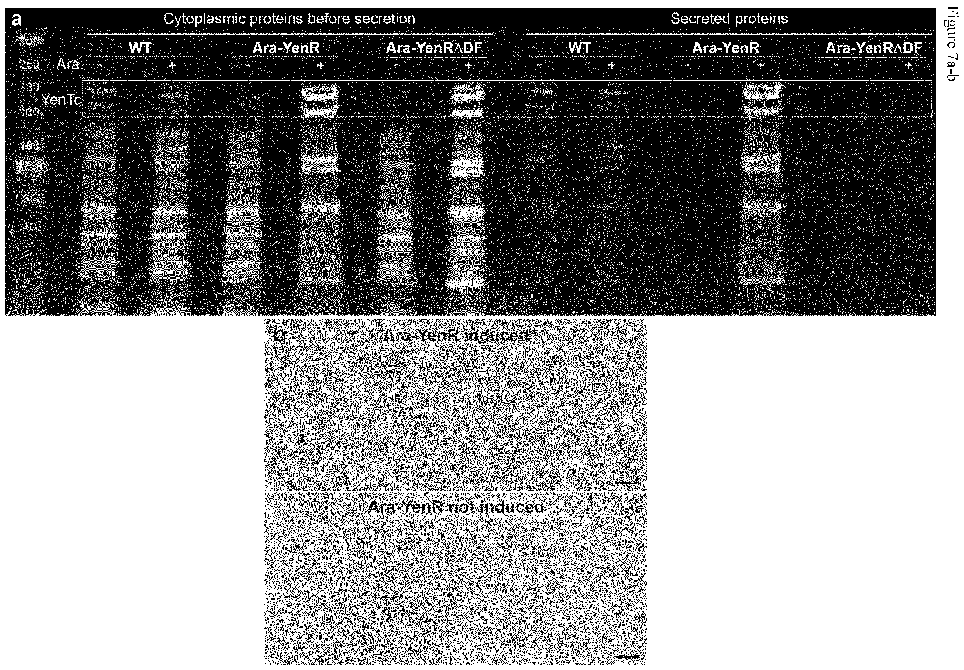

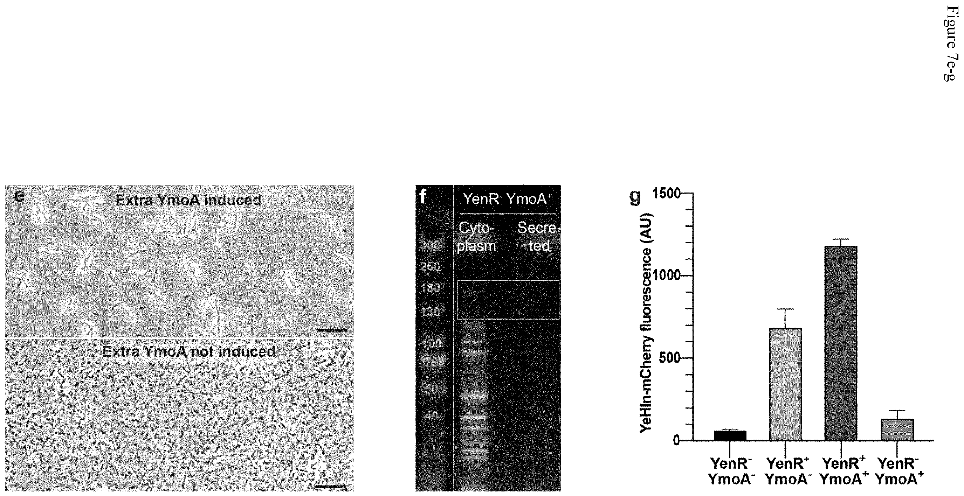

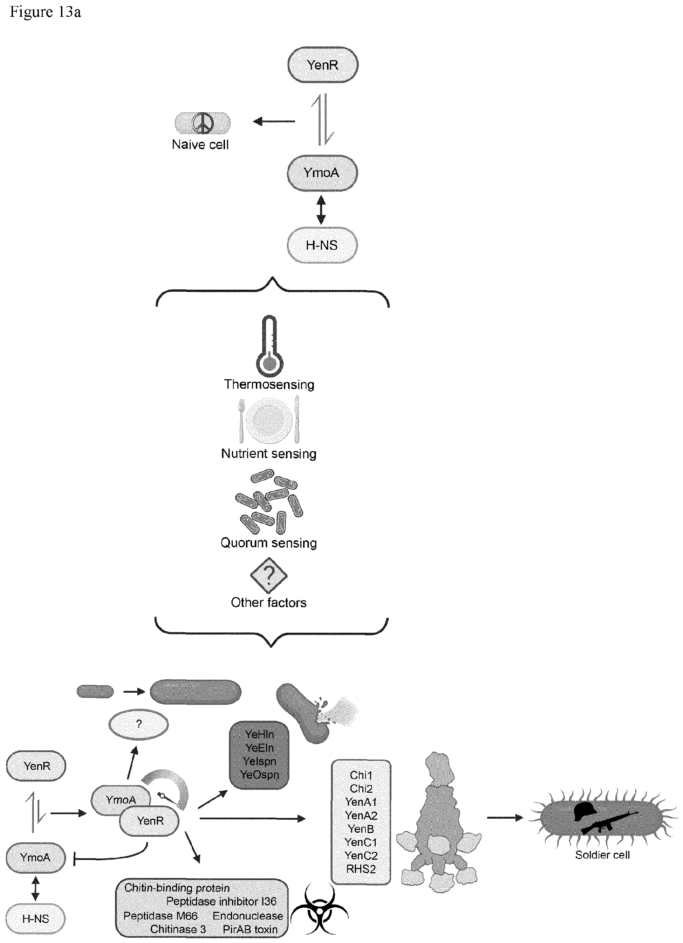

The invention has overcome the above-mentioned difficulties and embodies specific advantages. Especially, the provision of a *Yersinia entomophaga* bacterium comprising a modified YenR gene provides the basis for a novel system for mass production of an insecticidal toxin composition, which can be applied to crops in the form of either intact bacteria, the toxin composition, or individual toxin species purified from this mix, such as a YenTc toxin. It is shown herein that the bacterial species *Yersinia entomophaga* (a risk group 1 organism⁸¹ harmless to humans but extremely lethal to insects²⁶) produces a mix of potent insecticidal toxins **(****Fig. 1a****)** in a small specialized subset of pathogenic cells **(****Fig. 3b** **and** **13a****),** which release this mix into the surrounding environment using a pH-controlled lysis process mediated by a type 10 secretion system (YenDF) **(****Fig. 3c****).** The present invention identified the central switch (YenR) controlling the conversion of normal cells to pathogenic cells. The inventors used targeted genomic editing to create a modified strain (Ara-YenR) where expression of YenR is placed under control of an arabinose-inducible promoter. The inventors could show that the entire population of bacteria can be turned into toxin-producers/releasers upon induction of YenR **(****Fig. 7b****).** As a result, the total production and secretion of *Yersinia entomophaga* insecticidal toxins is boosted tremendously **(****Fig. 7a****).** Inducing an additional regulatory factor (YmoA) can increase production of proteins controlled by YenR even further **(****Fig. 7e****).** Such bacteria can be applied directly to plants, such as crops, in a manner similar to current Bt-derived biopesticides where an insecticidal effect is desired. Also, the toxin composition produced by the modified *Yersinia entomophaga* bacterium, or individual toxin species purified from this composition can be applied directly to plants, such as crops, where an insecticidal effect is desired.

In addition, the invention demonstrated that the extremely dense growth of *Yersinia entomophaga* bacteria in SOC media causes acidification that prevents release of the toxin composition until the pH is raised. During a brief incubation time, the modified *Yersinia entomophaga* cells release the toxin composition directly into the buffer, with no laborious cell lysis steps required. The cell debris can then be separated and the supernatant can be filtered, to obtain a highly concentrated and ready-to-use composition of insecticidal toxins. Alternatively, if secretion is not immediately desired, the bacteria can be stored in pH 5.5 buffer, which will prevent secretion of toxins, until later use.

Additionally, the inventors found that secretion of the toxin composition is controlled by the T10SS (YenDF). A *Yersinia entomophaga* bacterium comprising a functional YenDF can secrete a toxin composition in a pH dependent manner. Secretion can however be avoided by modifying the YenDF, e.g. by disrupting components of YenDF. This creates *Yersinia entomophaga* bacteria that will only release a toxin composition upon an additional induced lysis step which is advantageous in an industrial setting where tightly controlled lysis of a toxin composition may be desired.

Furthermore, if an isolated toxin of the toxin composition is needed, an in-frame affinity tag can be fused to a said toxin, which can then be purified in a high-throughput manner. Using this purification strategy, 270 mg of a pure YenTc was obtained from 1 liter of modified *Yersinia entomophaga* cells (compared to 21 mg using an unmodified *Yersinia entomophaga* strain **Fig. 15d****).**

The above is illustrated in the appended examples.

In the following the invention is described in more detail.

In particular, the invention relates to the following items:
1. A *Yersinia entomophaga* bacterium comprising a modified YenR gene.
2. The *Yersinia entomophaga* bacterium of item 1, wherein the expression of the YenR gene or gene products thereof is transiently or permanently modulated.
3. The *Yersinia entomophaga* bacterium of item 1 or 2, wherein the expression of the YenR gene or gene products thereof is increased, optionally wherein the expression of the YenR gene or gene products thereof is directly or indirectly increased, or
   wherein the *Yersinia entomophaga* bacterium is capable of having the expression of the YenR gene or gene products increased, optionally wherein the *Yersinia entomophaga* bacterium is capable of having the expression of the YenR gene or gene products thereof directly or indirectly increased.
4. The *Yersinia entomophaga* bacterium of any one of items 1-3, wherein the genomic sequence of the YenR gene is modified.
5. The *Yersinia entomophaga* bacterium of any one of items 1-4, wherein the promoter region of the YenR gene is modified.
6. The *Yersinia entomophaga* bacterium of item 4 or 5, wherein the YenR gene and/or the promoter region of the YenR gene comprises a synthetic or artificial sequence.
7. The *Yersinia entomophaga* bacterium of any one of items 1-6, wherein the promoter region of the YenR gene comprises an inducible promoter or a high expressing promoter or regulatory elements.
8. The *Yersinia entomophaga* bacterium of any one of items 1-7, wherein the YenR gene comprises an inducible promoter or inducible regulatory element.
9. The *Yersinia entomophaga* bacterium of any one of items 1-8, wherein the YenR gene comprises an arabinose-inducible promoter or regulatory element, a lactose/IPTG-inducible promoter or regulatory element, an anhydrotetracycline-inducible promoter or regulatory element, or a rhamnose-inducible promoter or regulatory element, preferably an arabinose-inducible promoter or regulatory element.
10. The *Yersinia entomophaga* bacterium of any one of items 1-9, comprising the nucleotide sequence set forth in SEQ ID NO: 1 and/or SEQ ID NO: 2.
11. The *Yersinia entomophaga* bacterium of any one of items 1-10, further comprising a modified YmoA gene.
12. The *Yersinia entomophaga* bacterium of item 11, wherein the expression of the YmoA gene or gene products thereof is transiently or permanently modulated.
13. The *Yersinia entomophaga* bacterium of item 12, wherein the expression of the YmoA gene or gene products thereof is directly or indirectly increased.
14. The *Yersinia entomophaga* bacterium of any one of items 11-13, wherein the genomic sequence of the YmoA gene is modified.
15. The *Yersinia entomophaga* bacterium of any one of items 11-14, wherein the promoter region of the YmoA gene is modified.
16. The *Yersinia entomophaga* bacterium of item 14 or 15, wherein the YmoA gene and/or the promoter region of the YmoA gene comprises a synthetic or artificial sequence.
17. The *Yersinia entomophaga* bacterium of any one of items 11-16, wherein the promoter region of the YmoA gene comprises an inducible promoter or a high expressing promoter or regulatory elements.
18. The *Yersinia entomophaga* bacterium of any one of items 11-17, wherein the YmoA gene comprises an inducible promoter or inducible regulatory element.
19. The *Yersinia entomophaga* bacterium of any one of items 11-18, wherein the YmoA gene comprises an arabinose-inducible promoter or regulatory element, a lactose/IPTG-inducible promoter or regulatory element, an anhydrotetracycline-inducible promoter or regulatory element, or a rhamnose-inducible promoter or regulatory element, preferably an anhydrotetracycline-inducible promoter or regulatory element.
20. The *Yersinia entomophaga* bacterium of any one of items 1-10, comprising the nucleotide sequence set forth in SEQ ID NO: 3.
21. The *Yersinia entomophaga* bacterium of any one of items 1-20, comprising functional YenDF, wherein YenDF comprises YeHln, YeEln, YeIspn and YeOspn.
22. The *Yersinia entomophaga* bacterium of any one of items 1-21, comprising the nucleotide sequence set forth in any one of SEQ ID NOs: 18-21 or 32 and/or the amino acid sequence set forth in any one of SEQ ID NOs: 4-7.
23. The *Yersinia entomophaga* bacterium of any one of items 1-22, further comprising modified YenDF.
24. The *Yersinia entomophaga* bacterium of item 23, wherein the expression of the YeHln, YeEln, YeIspn and/or YeOspn gene or gene products thereof is/are directly or indirectly decreased or increased.
25. The *Yersinia entomophaga* bacterium of item 23 or 24, wherein the genomic sequence of YeHln, YeEln, YeIspn and/or YeOspn is/are modified.
26. The *Yersinia entomophaga* bacterium of any one of items 23-25, comprising a disrupted YenDF, wherein the disruption comprises a deletion of the YeHln, YeEln, YeIspn and/or YeOspn gene or within the YeHln, YeEln, YeIspn and/or YeOspn gene.
27. The *Yersinia entomophaga* bacterium of any one of items 22-26, comprising the nucleotide sequence set forth in SEQ ID NO: 8 or 16.
28. The *Yersinia entomophaga* bacterium of any one of items 1-27, further comprising a modified YenA2 or a modified YenA2 gene.
29. The *Yersinia entomophaga* bacterium of item 28, wherein the modified YenA2 comprises an internal, N-terminal and/or C-terminal purification tag, and/or, wherein the modified YenA2 gene comprises a sequence encoding a purification tag, optionally wherein the modified YenA2 or modified YenA2 gene comprises a linker sequence.
30. The *Yersinia entomophaga* bacterium of item 29, wherein the purification tag is one or more of His-tag, FLAG tag, polycysteine tag and Strep-tag.
31. The *Yersinia entomophaga* bacterium of any one of items 28-30, wherein the modified YenA2 comprises a C-terminal His-tag.
32. The *Yersinia entomophaga* bacterium of any one of items 27-31, comprising the nucleotide sequence set forth in SEQ ID NO: 16, 17 or 27 and/or the amino acid sequence set forth in SEQ ID NO: 9.
33. The *Yersinia entomophaga* bacterium of any one of items 1-32, wherein the bacterium is comprised in a storage buffer at pH 4.5-6.0, preferably pH 5.5.
34. A toxin composition comprising Cbp, Chi1, Chi2, Chi3, NucA, Pil36, PirA, PirB, StcE, Tlh, YenA1, YenA2, YenB, YenC1, YenC2, and RHS2.
35. The toxin composition of item 34, wherein the composition is toxic to invertebrate animals, preferably to invertebrate animals belonging to the class of Insecta.
36. The toxin composition of item 34 or 35, wherein the composition is formulated as a pesticide, such as an insecticide, optionally comprising a suitable carrier.
37. The toxin composition of any one of items 34-36, wherein the composition is not toxic to animals with a body temperature of 30°C or higher.
38. The toxin composition of any one of items 34-37, wherein the composition is not toxic to animals not belonging to the class of Insecta.
39. The toxin composition of any one of items 34-38, wherein the composition is not toxic to mammals.
40. The toxin composition of any one of items 34-39, wherein the composition is not toxic to humans.
41. A method to produce a toxin composition comprising a step of culturing a *Yersinia entomophaga* bacterium of any one of items 1-33.
42. The method of item 41, wherein the toxin composition comprises one or more of Cbp, Chi1, Chi2, Chi3, NucA, Pil36, PirA, PirB, StcE, Tlh, YenA1, YenA2, YenB, YenC1, YenC2, RHS2 or a combination thereof.
43. The method of item 41 or 42, wherein the culturing step comprises culturing the *Yersinia entomophaga* bacterium at an initial pH of about 7.0, further comprising culturing the bacteria until a pH of 5.5-6.0 is reached.
44. The method of any one of items 41-43, wherein the culturing step comprises culturing the *Yersinia entomophaga* bacterium at less than 30°C.
45. The method of any one of items 41-44, wherein the culturing step comprises culturing the *Yersinia entomophaga* bacterium at 10-25°C.
46. The method of any one of items 41-45, wherein the culturing step comprises culturing the *Yersinia entomophaga* bacterium in the presence of markers mimicking the *Yersinia entomophaga* host, such as complex organic compounds.
47. The method of any one of items 41-46, further comprising a secretion step.
48. The method of item 47, wherein the secretion step comprises
   (i) increasing the pH of the initial culture from pH 5.5-6.0 to pH 6.3-10.0 or transferring the bacterium to a pH 6.3-10.0 buffer and/or
   (ii) incubating the *Yersinia entomophaga* bacterium at pH 6.3-10.0, and/or optionally
   (iii) a lysis step comprising mechanical lysis or enzymatic lysis of the *Yersinia entomophaga* bacterium, optionally wherein the lysis step is performed within an insect host.
49. The method of item 47 or 48, wherein the secretion step comprises
   (i) increasing the pH of the initial culture from pH 5.5-6.0 to pH 6.9-8.9 or transferring the bacterium to a pH 6.9-8.9 buffer and
   (ii) incubating the *Yersinia entomophaga* bacterium at pH 6.9-8.9.
50. The method of item 47, wherein the secretion step comprises incubating the *Yersinia entomophaga* bacterium under anaerobic conditions, wherein the anaerobic conditions result in secretion-inducing anaerobic stress.
51. The method of any one of items 41-50, further comprising a purification step.
52. The method of item 51, wherein the purification step comprises
   (i) centrifuging the *Yersinia entomophaga* bacterium, and optionally
   (ii) filtrating the *Yersinia entomophaga* bacterium, and optionally
   (iii) purifying a toxin composition.
53. The method of item 51 or 52, comprising separating a cellular fraction and a fraction comprising a toxin composition from the *Yersinia entomophaga* bacterium culture.
54. The method of any one of items 41-53, further comprising a recovery step.
55. The method of item 54, wherein the recovery step comprises
   (i) recovering one or more of Cbp, Chi1, Chi2, Chi3, NucA, Pi136, PirA, PirB, StcE, Tlh, YenA1, YenA2, YenB, YenC1, YenC2, RHS2 or a combination thereof from a culture of the *Yersinia entomophaga* bacterium and/or
   (ii) recovering a YenTc toxin from a culture of the *Yersinia entomophaga* bacterium and/or optionally
   (iii) concentrating the substances or combinations thereof of (i) and/or the YenTc toxin of (ii).
56. A toxin composition obtained by or as obtainable by the method of any one of items 41-55.
57. A *Yersinia entomophaga* bacterium of any one of items 1-33, further comprising a modified gene encoding a YenTc subunit or a modified YenTc subunit.
58. The *Yersinia entomophaga* bacterium of item 57, wherein the gene encoding a YenTc subunit comprises a sequence encoding a purification tag or wherein the modified YenTc subunit comprises a purification tag.
59. The *Yersinia entomophaga* bacterium of item 57 or 58, wherein the gene encoding a YenTc subunit comprises a sequence encoding the purification tag 5' or 3' or within the coding region, in frame of the coding sequence, optionally comprising a linker sequence.
60. The *Yersinia entomophaga* bacterium of item 57 or 58, wherein the modified YenTc subunit comprises an internal, N-terminal and/or C-terminal purification tag.
61. The *Yersinia entomophaga* bacterium of any one of items 58-60, wherein the purification tag is one or more of His-tag, FLAG tag, polycysteine tag and Strep-tag.
62. The *Yersinia entomophaga* bacterium of any one of items 57-61, wherein the gene encoding a YenTc subunit or the YenTc subunit is one or more of Chi1, Chi2, RHS2, YenA1, YenA2, YenB, YenC1, YenC2 or a combination thereof.
63. The *Yersinia entomophaga* bacterium of any one of items 57-62, wherein the subunit is YenA2 comprising a C-terminal His-tag.
64. The *Yersinia entomophaga* bacterium of any one of items 57-63, wherein the bacterium is comprised in a storage buffer at pH 4.5-6.0, preferably pH 5.5.
65. A method to produce a purified YenTc toxin comprising a step of culturing a *Yersinia entomophaga* bacterium of any one of items 57-64.
66. The method of item 65, wherein the culturing step comprises culturing the *Yersinia entomophaga* bacterium at an initial pH of about 7.0, further comprising culturing the bacteria until a pH of 5.5-6.0 is reached.
67. The method of item 65 or 66, wherein the culturing step comprises culturing the *Yersinia entomophaga* bacterium at less than 30°C.
68. The method of any one of items 65-67, wherein the culturing step comprises culturing the *Yersinia entomophaga* bacterium at 10-25°C.
69. The method of any one of items 65-68, wherein the culturing step comprises culturing the *Yersinia entomophaga* bacterium in the presence of markers mimicking the *Yersinia entomophaga* host, such as complex organic compounds.
70. The method of any one of items 65-69, further comprising a secretion step.
71. The method of item 70, wherein the secretion step comprises
   (i) increasing the pH of the initial culture from pH 5.5-6.0 to pH 6.3-10.0 or transferring the bacterium to a pH 6.3-10.0 buffer and
   (ii) incubating the *Yersinia entomophaga* bacterium at pH 6.3-10.0 and/or
   (iii) a lysis step comprising mechanical lysis or enzymatic lysis of the *Yersinia entomophaga* bacterium, optionally wherein the lysis step is performed within an insect host.
72. The method of item 70 or 71, wherein the secretion step comprises
   (i) incubating the pH of the initial culture from pH 5.5-6.0 to pH 6.9-8.9 or transferring the bacterium to a pH 6.9-8.9 buffer and
   (ii) incubating the *Yersinia entomophaga* bacterium at pH 6.9-8.9.
73. The method of item 70, wherein the secretion step comprises incubating the *Yersinia entomophaga* bacterium under anaerobic conditions, wherein the anaerobic conditions result in secretion-inducing anaerobic stress.
74. The method of any one of items 65-73, further comprising a purification step.
75. The method of item 74, wherein the purification step comprises
   (i) centrifuging the *Yersinia entomophaga* bacterium, and/or optionally
   (ii) filtrating the *Yersinia entomophaga* bacterium, and/or optionally
   (iii) purifying a YenTc toxin via an affinity purification.
76. The method of item 75, wherein the purification step comprises a bead-based purification and wherein the beads show high affinity to the modified YenTc subunit.
77. The method of any one of items 65-76, further comprising a recovery step.
78. The method of item 77, wherein the recovery step comprises
   (i) recovering a purified YenTc toxin from a culture of the *Yersinia entomophaga* bacterium and/or optionally
   (ii) concentrating the YenTc toxin.
79. A purified YenTc toxin obtained by or as obtainable by the method of any one of items 65-78.
80. A YenTc toxin or a subunit of a YenTc toxin comprising a purification tag.
81. The YenTc toxin or subunit of a YenTc toxin of item 80, comprising an internal, N-terminal and/or C-terminal purification tag.
82. The YenTc toxin or subunit of a YenTc toxin of item 80 or 81, comprising one or more of His-tag, FLAG tag, polycysteine tag and Strep-tag.
83. The YenTc toxin or subunit of a YenTc toxin of any one of items 80-82, comprising a YenA2 subunit with a C-terminal His-tag.
84. A method to induce secretion of a toxin or toxin composition comprising
   (i) culturing a *Yersinia entomophaga* bacterium of any one of items 1-33 or 57-64, at an initial pH of about 7.0 until a desired number of bacteria is grown; and
   (ii) incubating the bacteria of (i) at pH 6.3-10.0, preferably pH 6.9-8.9, or transferring the bacteria to a pH 6.3-10.0 buffer, preferably pH 6.9-8.9, thereby inducing the secretion of the toxin.
85. A population of soldier cells, wherein the soldier cells are *Yersinia entomophaga* bacteria, wherein the expression of the YenR gene or gene products thereof is increased, optionally, wherein the expression of the YmoA gene or gene products thereof is increased and/or wherein the soldier cells comprise the toxin composition and/or one or more of the toxin(s) according to items 34-40, preferably, wherein the population comprises the *Yersinia entomophaga* bacterium of any one of items 1-33 or 57-64, optionally wherein the expression of the YenR gene or gene products thereof is directly or indirectly increased, further optionally, wherein the expression of the YmoA gene or gene products thereof is directly or indirectly increased.
86. A method to produce a soldier cell comprising
   (i) increasing the expression of the YenR gene or gene products thereof in a *Yersinia entomophaga* bacterium, optionally increasing the expression of the YmoA gene or gene products thereof;
   (ii) culturing the *Yersinia entomophaga* bacterium of (i) at an initial pH of about 7.0 until a desired number of bacteria is grown.
87. The method of item 86, further comprising storing the *Yersinia entomophaga* bacterium in a storage buffer at pH 4.5-6.0, preferably pH 5.5.
88. A method to kill pests comprising contacting a pest with a *Yersinia entomophaga* bacterium of any one of items 1-33 or 57-64 or the population of soldier cells of item 85.
89. A method to kill pests comprising contacting a pest with a toxin composition of any one of items 34-40 or 56 or a YenTc toxin of any one of items 79, or 80-83.
90. The method of item 88 or 89, wherein the pest is an animal belonging to the class of Insecta, such as an insect.
91. The method of any one of items 88-90, wherein the pest is contacted on or near a plant or in an area where plants are grown.
92. The method of any one of items 88-91, wherein the pest is contacted on a plant in an agricultural area or a garden area or park area.
93. The method of any one of items 88-92, wherein the bacterium, population of soldier cells or the toxin composition or YenTc toxin is administered to plants infested with a pest, such as an insect pest.
94. The method of item 93, wherein the bacterium, population of soldier cells or the toxin composition or YenTc toxin is repeatedly administered to plants infested with a pest, such as an insect pest.
95. Use of a *Yersinia entomophaga* bacterium of any one of items 1-33 or 57-64 or the population of soldier cells of item 85 as a pesticide, preferably an insecticide.
96. Use of a toxin composition of any one of items 34-40 or 56, or a YenTc toxin of any one of items 79 or 80-83 as a pesticide, preferably an insecticide.
97. Use of a *Yersinia entomophaga* bacterium of any one of items 1-33 or 57-64 or the population of soldier cells of item 85 to produce a toxin composition, optionally wherein the toxin composition is formulated as a pesticide composition, preferably insecticide composition.
98. Use of a *Yersinia entomophaga* bacterium of any one of items 1-33 or 57-65 or the population of soldier cells of item 85 to produce a YenTc toxin.
99. A population of cells comprising the *Yersinia entomophaga* bacterium of any one of items 1-33 or 57-64.
100. A modified YenR gene comprising the sequence set forth in SEQ ID NO: 2.
101. A plasmid comprising the sequence set forth in SEQ ID NO: 3.

It is envisaged herein that the YenR of the bacterium *Yersinia entomophaga* is modified in particular to allow/provide for increased expression of the YenR gene or gene product. In other words, a *Yersinia entomophaga* bacterium comprising a modified YenR gene is capable of an increased expression of the YenR gene or gene product or a *Yersinia entomophaga* bacterium comprising a modified YenR gene has an increased expression of the YenR gene or gene product. Specifically, it is envisaged herein that the YenR of the bacterium *Yersinia entomophaga* is modified to allow/provide for inducible expression of the YenR gene. As mentioned above and shown herein, in wild type *Yersinia entomophaga* bacteria only a small subpopulation induces the expression of YenR upon certain stimuli and transforms into so called soldier cells that produce and secrete a toxin composition. Thus, the capacity of wild type *Yersinia entomophaga* bacteria to produce the toxin/toxin composition is limited.

However, in the examples of the present application it is demonstrated that forced/induced expression (e.g. via an inducible promoter) of the YenR gene in all bacteria of a population allows to transform all bacteria of the population into so called soldier cells that produce and secrete a toxin composition. Accordingly, it is particular envisaged herein that the YenR gene of the bacterium *Yersinia entomophaga* is so modified that the expression of the YenR gene can be forced/induced.

Accordingly, in one aspect, the present invention provides a *Yersinia entomophaga* bacterium comprising a modified YenR gene. A *Yersinia entomophaga* bacterium is a bacterium belonging to the species of *Yersinia entomophaga.* A *Yersinia entomophaga* bacterium of the present invention in general is a modified *Yersinia entomophaga* bacterium, i.e. that it is different from a wild type *Yersinia entomophaga* bacterium. In the sense of the present invention a "wild type *Yersinia entomophaga* bacterium" relates to bacteria isolated from nature, such as the *Yersinia entomophaga* strain MH96 (available via the DSMZ-German Collection of Microorganisms and Cell Cultures GmbH; DSMNo.: 22339 or from ATCC under accession number ATCC BAA-1678). Generally, any "wild type *Yersinia entomophaga* bacterium" can be modified as described herein, particularly in relation to a modified YenR gene. Such "wild type *Yersinia entomophaga* bacterium" are readily available and can, for example, be obtained from depository institutions, e.g. the *Yersinia entomophaga* strain MH96 is available from the DSMZ-German Collection of Microorganisms and Cell Cultures GmbH; DSM No.: 22339 or from ATCC under accession number ATCC BAA-1678).

The YenR gene encodes a OmpR/PhoB-type helix-turn-helix fold protein UniProt accession number (A0A3S6F5G2). The YenR gene may be modified in a *Yersinia entomophaga* bacterium. Accordingly, the present invention relates to a *Yersinia entomophaga* bacterium comprising a modified YenR gene.

In the sense of the present invention "modified *Yersinia entomophaga* bacterium" means that the *Yersinia entomophaga* wild type strain has been altered. The term "modified" is not particularly limited to any modifications in the *Yersinia entomophaga* wild type strain, but rather relates to any modification that distinguishes the modified the *Yersinia entomophaga* bacterium from a wild type bacterium structurally or functionally. The skilled person is aware that such modifications may be for example permanent or transient changes in the genome, transient changes in gene expression, transient or permanent modulation of the activity of a gene or gene product. In the sense of the present invention "modulate" or "modulation" means that an expression or activity is modified or controlled by precise tools, such as genome editing or provision of a compound such as a drug. The expression of the YenR gene or gene products thereof may be modulated in a modified *Yersinia entomophaga* bacterium. Accordingly, the invention provides a *Yersinia entomophaga* bacterium comprising a modified YenR gene wherein the expression of the YenR gene or gene products thereof is modulated. Such a modulation of the YenR gene or gene products thereof may be transient or permanent. Accordingly, the invention provides a *Yersinia entomophaga* bacterium comprising a modified YenR gene wherein the expression of the YenR gene or gene products thereof is transiently or permanently modulated.

Transient modulation in gene expression can be achieved e.g. by a compound which increases or decreases the expression of a gene, such as the YenR gene. Transient modulation can also be achieved e.g. by a compound which increases or decreases the activity of a gene product. A compound can be any compound that is able to modulate the expression or activity of the YenR gene or gene product, such as a drug.

Accordingly, the invention provides a *Yersinia entomophaga* bacterium comprising a modified YenR gene wherein the expression of the YenR gene or gene products thereof is transiently modulated by a drug.

The invention provides a *Yersinia entomophaga* bacterium comprising a modified YenR gene, wherein the expression of the YenR gene or gene product is transiently modulated by a drug.

Preferably, the invention provides a *Yersinia entomophaga* bacterium comprising a modified YenR gene, wherein the expression of the YenR gene or gene product is transiently increased by a drug.

A transient modulation may also be achieved by contacting a *Yersinia entomophaga* bacterium comprising a modified YenR gene with positive or negative regulator of the YenR gene or gene products thereof. In the sense of the present invention a positive regulator of the YenR gene or gene products thereof increases the expression of the YenR gene or gene products thereof. In general, autoinducer-1 type molecules, complex organic compounds and temperatures below 30°C can also increase YenR expression in the sense of a positive regulation. A positive regulator in the sense of the present invention may be autoinducer-1 type molecules. In the sense of the present invention a negative regulator of the YenR gene or gene products thereof decreases the expression of the YenR gene or gene products thereof. Accordingly, the invention provides a *Yersinia entomophaga* bacterium comprising a modified YenR gene, wherein the expression of the YenR gene or gene products thereof is transiently modulated. A positive or negative regulator of the YenR gene or gene products can be any compound, such as a drug. The invention provides a *Yersinia entomophaga* bacterium comprising a modified YenR gene, wherein the expression of the YenR gene or gene products thereof is transiently modulated by a positive or negative regulator of the YenR gene.

Preferably, the invention provides a *Yersinia entomophaga* bacterium comprising a modified YenR gene, wherein the expression of the YenR gene or gene products thereof is transiently increased.

Accordingly, the invention provides a *Yersinia entomophaga* bacterium comprising a modified YenR gene, wherein the expression of the YenR gene or gene products thereof is transiently increased by a positive regulator of the YenR gene.

In the sense of the present invention, the expression of the YenR gene or gene products thereof may also be modulated by contacting a *Yersinia entomophaga* bacterium comprising a modified YenR gene with a nucleic acid. In particular, the expression of the YenR gene or gene products thereof may be modulated by contacting a *Yersinia entomophaga* bacterium comprising a modified YenR gene with a nucleic acid encoding a positive or negative regulator. A nucleic acid encoding a positive or negative regulator can be DNA or RNA. A nucleic acid encoding a positive or negative regulator can be a plasmid. In preferred embodiments the plasmid encodes a YmoA gene. A YmoA gene in the sense of the present invention relates to a YmoA gene with the UniProt accession number A0A3S6EV06. The YmoA gene encodes a histone-like thermosensitive virulence regulator which is affected by YenR induction. As demonstrated in the examples YmoA overexpression boosts production of YenR-controlled proteins when YenR is present.

Accordingly, the invention provides a *Yersinia entomophaga* bacterium comprising a modified YenR gene, comprising a nucleic acid encoding a regulator of the YenR gene, wherein the expression of the YenR gene or gene products thereof is transiently modulated.

Preferably, the invention provides a *Yersinia entomophaga* bacterium comprising a modified YenR gene, comprising a nucleic acid encoding a positive regulator of the YenR gene, wherein the expression of the YenR gene or gene products thereof is transiently increased.

Preferably, the invention provides a *Yersinia entomophaga* bacterium comprising a modified YenR gene, comprising a DNA plasmid encoding a YmoA gene, wherein the expression of the YenR gene or gene products thereof is transiently increased.

Preferably, a "modified YenR gene" is a YenR gene that has been modified resulting in an increased expression of the YenR gene or gene product and/or allowing/providing for an increased expression of the YenR gene or gene product. An increase of expression can be achieved by direct or indirect means. Direct means of increasing expression are e.g. a modification of the YenR gene by genome editing or targeting the YenR gene with a compound such as a drug. Thus, a direct increase of expression will target the YenR gene per se. Indirect means of increasing expression are e.g. a modification of a positive or negative regulator of the YenR gene or a modification of any YenR pathway components resulting in increased expression of the YenR gene or gene products. The skilled person is aware that a modification of a negative regulator of the YenR gene or gene products can increase the expression or decrease the expression if downregulated or upregulated, respectively. Accordingly, a modification of a positive regulator of the YenR gene or gene products can increase the expression or decrease the expression if upregulated or downregulated, respectively. In the sense of the invention "YenR pathway" comprises anything that can directly or indirectly result in a modulated expression of YenR gene or gene products. For example, a negative or positive regulator of YenR can be a YenR pathway component.

Accordingly, the invention provides a *Yersinia entomophaga* bacterium comprising a modified YenR gene, wherein the expression of the YenR gene or gene products thereof is (e.g. directly or indirectly) increased. The invention also provides a *Yersinia entomophaga* bacterium comprising a modified YenR gene, wherein the *Yersinia entomophaga* bacterium is capable of increased expression of the YenR gene or gene products thereof (e.g. wherein the expression is directly or indirectly increased).

Accordingly, the invention provides a *Yersinia entomophaga* bacterium comprising a modified YenR gene, wherein the expression of the YenR gene or gene products thereof is increased. The term "increased expression of the YenR gene or gene products thereof" or the like as used herein refers, in particular, to an expression level (or an amount of the gene product) sufficient to induce or allow production of the toxin composition or toxin as defined and provided herein. The same explanation applies, mutatis mutandis, to the term "increased expression of the YmoA gene or gene products thereof".

The invention provides a *Yersinia entomophaga* bacterium comprising a modified YenR gene, wherein the expression of the YenR gene or gene products thereof is directly or indirectly increased.

In one aspect, the invention provides a *Yersinia entomophaga* bacterium comprising a modified YenR gene, wherein the *Yersinia entomophaga* bacterium is capable of having the expression of the YenR gene or gene products increased, optionally wherein the *Yersinia entomophaga* bacterium is capable of having the expression of the YenR gene or gene products thereof directly or indirectly increased.

The invention provides a *Yersinia entomophaga* bacterium comprising a modified YenR gene, wherein the expression of the YenR gene or gene products thereof is directly increased.

As mentioned, a direct increase in YenR expression can be achieved by altering the genome of the *Yersinia entomophaga* bacterium by genome editing. Such changes can be permanent or transient.

Accordingly, the present invention provides a *Yersinia entomophaga* bacterium comprising a modified YenR gene, wherein the expression of the YenR gene or gene products thereof is permanently modulated.

For example, a permanent change in the genome can be achieved by contacting a *Yersinia entomophaga* bacterium with a positive regulator of YenR or a nucleic acid encoding the same. Such a nucleic acid can be permanently incorporated into the *Yersinia entomophaga* bacterium. An example for such a nucleic acid being permanently incorporated into the *Yersinia entomophaga* bacterium is a DNA plasmid, preferably encoding a YmoA gene.

Accordingly, the invention provides a *Yersinia entomophaga* bacterium comprising a modified YenR gene, comprising a nucleic acid encoding a regulator of the YenR gene, wherein the expression of the YenR gene or gene products thereof is permanently modulated.

Preferably, the invention provides a *Yersinia entomophaga* bacterium comprising a modified YenR gene, comprising a nucleic acid encoding a positive regulator of the YenR gene, wherein the expression of the YenR gene or gene products thereof is permanently modulated. Preferably, the invention provides a *Yersinia entomophaga* bacterium comprising a modified YenR gene, comprising a DNA plasmid encoding a YmoA gene, wherein the expression of the YenR gene or gene products thereof is permanently modulated.

In a particular aspect, the present invention provides a *Yersinia entomophaga* bacterium comprising a modified YenR gene, wherein the expression of the YenR gene can be induced. In the sense of the present invention "induced" means that an expression of a gene is modulated depending on a signal. The "signal" can be a chemical, light or temperature. Inducible systems that can be used in the present invention are known in the art. An inducible system can comprise an inducible promoter or inducible regulatory elements. An example of a chemically inducible system is the negative inducible prokaryotic promoter araBAD. This system is dependent on L-arabinose as a chemical signal. When L-arabinose is absent, regulatory protein araC binds O and I1 sites upstream of araBad, blocking transcription. The addition of L-arabinose causes araC to bind I1 and I2 sites, allowing transcription to begin. In addition to L-arabinose, cAMP complexed with cAMP activator protein (CAP) can also stimulate araC binding to I1 and I2 sites.

The arabinose inducible system can be introduced into a promoter region of any gene of interest to directly modulate the expression of the same in a transient, inducible manner. Introducing the arabinose inducible system is routine for the person skilled in the art and can be realised by common genome editing methods such as the λ-RED recombination system or CRISPR/Cas system. Briefly, when using the CRISPR/Cas system, the promoter region of the gene of interest is targeted with specific spacer sequences having homology to a target region where an insertion is desired. Further a donor polynucleotide comprising the arabinose inducible system with homology arms left and right of the desired insertion site is provided and incorporated into the promoter region by homology dependent repair (HDR). When using the λ-RED recombination system, a bacterium can be contacted with a DNA donor template is which is then inserted in the bacterium via homologous recombination facilitated by the λ-RED recombineering proteins. One non-limiting way of facilitating an insertion of an arabinose inducible system into the YenR promoter using the λ-RED recombination system is described in detail in the appended examples. In addition, the insertion can be combined with a selection marker to efficiently select for strains carrying a desired insertion. This selection maker can be excised from the strain at a later stage when desired. One non-limiting way of excising a selection marker using the CRISPR/Cas system is described in detail in the appended examples. Accordingly, the present invention provides a *Yersinia entomophaga* bacterium comprising a modified YenR gene, wherein the genomic sequence of the YenR gene is modified.

In one aspect, the present invention provides a *Yersinia entomophaga* bacterium comprising a modified YenR gene, wherein the promoter region of the YenR gene is modified.

In one aspect, the present invention provides a *Yersinia entomophaga* bacterium comprising a modified YenR gene, wherein the YenR gene and/or the promoter region of the YenR gene comprises a synthetic or artificial sequence.

In one aspect, the present invention provides a *Yersinia entomophaga* bacterium comprising a modified YenR gene, wherein the promoter region of the YenR gene comprises an inducible promoter or a high expressing promoter or regulatory elements.

In one aspect, the present invention provides a *Yersinia entomophaga* bacterium comprising a modified YenR gene, wherein the YenR gene comprises an inducible promoter or inducible regulatory element.

The present invention is not particularly limited to any inducible systems or inducible promoters or inducible regulatory elements. Any system, promoter or regulatory element can be used that results in modulation of the expression of the YenR gene or gene products. Examples of inducible systems that can be used are the arabinose inducible promoter araBAD, the tetracycline ON system, the Lac promoter, the AlcA promoter, the LexA promoter, the heat shock-inducible Hsp70 or Hsp90-derived promoters, the FixK2 promoter, R FixK2 promoter OR2-OR1-PR promoter, LtetO promoter, LlacO promoter, PesaR promoter, Tac promoter (hybrid of trp and lac promoters with higher expression upon induction), PrpB promoter, anhydrotetracycline inducible promoters, red light inducible inverter promoter, LtetO-1 promoter, PcpcG2 promoter. In the sense of the present invention a lactose/IPTG-inducible promoter or regulatory element, an anhydrotetracycline-inducible promoter or regulatory element, or a rhamnose-inducible promoter or regulatory element can also be used to induce expression of a gene of interest, such as YenR. In preferred embodiments the arabinose inducible promoter araBAD is used as an inducible system to increase YenR expression. Accordingly, the invention provides a *Yersinia entomophaga* bacterium comprising a modified YenR gene, wherein the YenR gene comprises an arabinose-inducible promoter or regulatory element, a lactose/IPTG-inducible promoter or regulatory element, an anhydrotetracycline-inducible promoter or regulatory element, or a rhamnose-inducible promoter or regulatory element, preferably an arabinose-inducible promoter or regulatory element.

The nucleic acid sequence set forth in SEQ ID NO: 1 encodes the genome of a *Yersinia entomophaga* bacterium comprising a modified YenR gene. In particular, the SEQ ID NO: 1 encodes the genome of a *Yersinia entomophaga* bacterium comprising a modified YenR gene comprising an arabinose inducible promoter.

Accordingly, the invention provides a *Yersinia entomophaga* bacterium comprising the nucleotide sequence set forth in SEQ ID NO: 1.

The nucleic acid sequence set forth in SEQ ID NO: 2 encodes a modified YenR gene. In particular, the SEQ ID NO: 2 encodes a modified YenR gene comprising an arabinose inducible promoter.

Accordingly, the invention also provides a *Yersinia entomophaga* bacterium, wherein the YenR gene comprises the nucleotide sequence set forth in SEQ ID NO: 2.

The invention also provides a *Yersinia entomophaga* bacterium, wherein the YenR gene consists of the nucleotide sequence set forth in SEQ ID NO: 2.

It is envisaged herein that the *Yersinia entomophaga* bacterium according to the invention comprises an additional modification that modulates (preferably increases) YenR expression and/or modulates (preferably increases) expression of YenR-controlled genes. As described above and in the examples YmoA overexpression boosts production of YenR-controlled proteins when YenR is present. Thus, in another aspect, the invention provides a *Yersinia entomophaga* bacterium comprising a modified YenR gene, which further comprises a modified YmoA gene. Increasing the expression of the YmoA gene or gene products can further increase YenR expression. Accordingly, the YmoA gene can be modified using the same means as described for a modification of the YenR gene above. Preferably, a "modified YmoA gene" is a YmoA gene that has been modified resulting in an increased expression of the YmoA gene or gene product and/or allowing/providing for an increased expression of the YmoA gene or gene product.

Accordingly, the invention provides a *Yersinia entomophaga* bacterium comprising a modified YenR gene, further comprising a modified YmoA gene.

In one aspect, the invention provides a *Yersinia entomophaga* bacterium comprising a modified YenR gene, further comprising a modified YmoA gene, wherein the expression of the YmoA gene or gene products thereof is transiently or permanently modulated.

In one aspect, the invention provides a *Yersinia entomophaga* bacterium comprising a modified YenR gene, further comprising a modified YmoA gene, wherein the expression of the YmoA gene or gene products thereof is directly or indirectly increased.

In one aspect, the invention provides a *Yersinia entomophaga* bacterium comprising a modified YenR gene, further comprising a modified YmoA gene, wherein the genomic sequence of the YmoA gene is modified.

In one aspect, the invention provides a *Yersinia entomophaga* bacterium comprising a modified YenR gene, further comprising a modified YmoA gene, wherein the promoter region of the YmoA gene is modified.

In one aspect, the invention provides a *Yersinia entomophaga* bacterium comprising a modified YenR gene, further comprising a modified YmoA gene, wherein the YmoA gene and/or the promoter region of the YmoA gene comprises a synthetic or artificial sequence.

In one aspect, the invention provides a *Yersinia entomophaga* bacterium comprising a modified YenR gene, further comprising a modified YmoA gene, wherein the promoter region of the YmoA gene comprises an inducible promoter or a high expressing promoter or regulatory elements.

In one aspect, the invention provides a *Yersinia entomophaga* bacterium comprising a modified YenR gene, further comprising a modified YmoA gene, wherein the YmoA gene comprises an inducible promoter or inducible regulatory element.

In one aspect, the invention provides a *Yersinia entomophaga* bacterium comprising a modified YenR gene, further comprising a modified YmoA gene, wherein the YmoA gene comprises an arabinose-inducible promoter or regulatory element, a lactose/IPTG-inducible promoter or regulatory element, an anhydrotetracycline-inducible promoter or regulatory element, or a rhamnose-inducible promoter or regulatory element, preferably an anhydrotetracycline-inducible promoter or regulatory element.

Expression of the YmoA gene can also be modulated by administering a nucleic acid to a *Yersinia entomophaga* bacterium comprising a modified YenR gene. In this regard the nucleic acid can be a nucleic acid encoding a YmoA gene. A nucleic acid encoding a YmoA gene can be a DNA or an RNA, such as an mRNA. A nucleic acid encoding a YmoA gene is preferably a DNA plasmid.

In context of the present invention the YmoA gene under control of an inducible promoter may be introduced into a *Yersinia entomophaga* bacterium in form of a plasmid. The plasmid may have the nucleotide sequence set forth in SEQ ID NO: 3. The nucleotide sequence set forth in SEQ ID NO: 3 encodes a plasmid comprising a YmoA gene under the control of an anhydrotetracycline-inducible promoter.

Accordingly, the present invention provides a *Yersinia entomophaga* bacterium comprising a modified YenR gene, further comprising the nucleotide sequence set forth in SEQ ID NO: 3.

In a preferred embodiment, the invention relates to a *Yersinia entomophaga* bacterium comprising a YenR gene under an arabinose-inducible promoter and a YmoA gene under control of an anhydrotetracycline-inducible promoter. In another preferred embodiment the invention relates to *Yersinia entomophaga* bacterium comprising the nucleotide sequence set forth in SEQ ID NO: 1 and the nucleotide sequence set forth in SEQ ID NO: 3. In another preferred embodiment the invention relates to *Yersinia entomophaga* bacterium comprising the nucleotide sequence set forth in SEQ ID NO: 2 and the nucleotide sequence set forth in SEQ ID NO: 3.

It is evident for the skilled person that if the *Yersinia entomophaga* bacterium is supposed to secrete the toxin composition it is necessary that mechanism(s) for secretion are functional. In other words, it may be necessary that the *Yersinia entomophaga* bacterium comprises functional genes or proteins involved in toxin secretion.

Thus, in a further aspect of the invention, it can be beneficial for a *Yersinia entomophaga* bacterium to comprise functional genes or proteins involved in toxin secretion. In particular, endolysins, holins and spanins can be involved in a secretion mechanism. Secretion for example can be reduced or abolished when one or more of the endolysin YeEln, the holin YeHln or the spanins YeIspn and YeOspn are disrupted in a *Yersinia entomophaga* bacterium. YeEln relates to UniProt accession number A0A3S6F4L4. YeHln relates to UniProt accession number A0A3S6F4H8. YeIspn relates to UniProt accession number A0A3S6F4Q6. YeOspn is unannotated in Uniprot. The ORF of YeOspn is half-integrated into the ORF of Yelspn, and the automated genome annotation of *Yersinia entomophaga* missed this unusual arrangement. Together, YeHln, YeEln, YeIspn and YeOspn form the *Yersinia entomophaga* death factor YenDF, which is also termed T10SS. To disrupt YenDF function one or more of YeHln, YeEln, YeIspn and YeOspn can be modified, such as disrupted or deleted, i.e. knocked out or knocked down. To enhance YenDF expression of one or more of YeHln, YeEln, YeIspn and YeOspn can be modified, such as increased. A functional YenDF in the sense of the present invention may comprise functional YeHln, YeEln, YeIspn and/or YeOspn genes. A functional YenDF in the sense of the present invention may comprise wild type YeHln, YeEln, YeIspn and/or YeOspn genes.

Accordingly, the present invention provides a *Yersinia entomophaga* bacterium comprising a modified YenR gene, comprising functional YenDF, wherein YenDF comprises YeHln, YeEln, YeIspn and YeOspn.

The nucleic acid sequence set forth in SEQ ID NO: 18 encodes an unmodified YeHln gene as found for example in the *Yersinia entomophaga* strain MH96.

The nucleic acid sequence set forth in SEQ ID NO: 19 encodes an unmodified YeEln gene as found for example in the *Yersinia entomophaga* strain MH96.

The nucleic acid sequence set forth in SEQ ID NO: 20 encodes an unmodified YeIspn gene as found for example in the *Yersinia entomophaga* strain MH96.

The nucleic acid sequence set forth in SEQ ID NO: 21 encodes an unmodified YeOspn gene as found for example in the *Yersinia entomophaga* strain MH96.

The amino acid sequence set forth in SEQ ID NO: 4 encodes an unmodified YeHln polypeptide as found for example in the *Yersinia entomophaga* strain MH96.

The amino acid sequence set forth in SEQ ID NO: 5 encodes an unmodified YeEln polypeptide as found for example in the *Yersinia entomophaga* strain MH96.

The amino acid sequence set forth in SEQ ID NO: 6 encodes an unmodified YeIspn polypeptide as found for example in the *Yersinia entomophaga* strain MH96.

The amino acid sequence set forth in SEQ ID NO: 7 encodes an unmodified YeOspn polypeptide as found for example in the *Yersinia entomophaga* strain MH96.

The present invention provides a *Yersinia entomophaga* bacterium comprising a modified YenR gene, further comprising the nucleotide sequence set forth in any one of SEQ ID NOs: 18-21 or 32 and/or the amino acid sequence set forth in any one of SEQ ID NOs: 4-7.

The present invention also provides a *Yersinia entomophaga* bacterium comprising a modified YenR gene, further comprising a modified YmoA gene, further comprising a functional YenDF, wherein YenDF comprises YeHln, YeEln, YeIspn and YeOspn.

The present invention provides a *Yersinia entomophaga* bacterium comprising a modified YenR gene, further comprising a modified YmoA gene, further comprising the nucleotide sequence set forth in any one of SEQ ID NOs: 18-21 or 32 and/or the amino acid sequence set forth in any one of SEQ ID NOs: 4-7.

The present invention provides a *Yersinia entomophaga* bacterium comprising a modified YenR gene, further comprising SEQ ID NO: 3, further comprising the nucleotide sequence set forth in any one of SEQ ID NOs: 18-21 or 32 and/or the amino acid sequence set forth in any one of SEQ ID NOs: 4-7.

In a preferred embodiment the invention provides a *Yersinia entomophaga* bacterium comprising SEQ ID NO: 1 and/or SEQ ID NO: 2, further comprising SEQ ID NO: 3, further comprising the nucleotide sequence set forth in any one of SEQ ID NOs: 18-21 or 32 and/or the amino acid sequence set forth in any one of SEQ ID NOs: 4-7.

In yet another aspect, the *Yersinia entomophaga* bacterium of the present invention can comprise a modified YenDF gene. *Yersinia entomophaga* death factor (YenDF) encodes the type 10 secretion system (T10SS) of *Yersinia entomophaga.* Upon activation of the T10SS the *Yersinia entomophaga* bacterium lyses and secretes any toxins produced. In the present invention the YenDF can be modified, such as downregulated or knocked out. This avoids secretion of toxins which can be desirable for example when storing or transporting the *Yersinia entomophaga* bacterium comprising a modified YenR gene. Expression of the YenDF can be modulated in the same way as described for YenR above. Since YenDF encodes several components, namely YeHln, YeEln, YeIspn and YeOspn any of them can be downregulated or knocked out. All components except YeIspn and YeOspn may be downregulated or knocked out. The YenDF may also be downregulated by a single disruption, due to the overlapping arrangement of YeHln, YeEln, YeIspn and YeOspn, they may be transcribed as a polycistronic mRNA. A modified YenDF can also comprise upregulated YeHln, YeEln, YeIspn and YeOspn, which can be useful if secretion on demand or an increased secretion is desired. Specifically, YeHln, YeEln, YeIspn and YeOspn can be controlled by inducible systems to allow for secretion on demand. In particular, YenDF can be modified to comprise an inducible system to modulate YenDF expression similar to YenR as described herein, e.g. by replacing the native YenDF promoter with an inducible system. This allows to decouple toxin production from toxin secretion which can be desired when secretion is to be avoided or to be temporally separated from induced toxin production. In another aspect, it is preferred herein that a *Yersinia entomophaga* bacterium of the present invention, e.g. when used as a pesticide/insecticide or in a pesticide/insecticide composition, is capable of secreting a toxin or toxin composition as defined herein.

Accordingly, the present invention provides a *Yersinia entomophaga* bacterium comprising a modified YenR gene, further comprising a modified YenDF.

The present invention provides a *Yersinia entomophaga* bacterium comprising a modified YenR gene, further comprising a modified YenDF, wherein the expression of the YeHln, YeEln, YeIspn and/or YeOspn gene or gene products thereof is/are directly or indirectly decreased or increased.

The present invention provides a *Yersinia entomophaga* bacterium comprising a modified YenR gene, further comprising a modified YenDF, wherein the genomic sequence of YeHln, YeEln, YeIspn and/or YeOspn is/are modified.

The present invention provides a *Yersinia entomophaga* bacterium comprising a modified YenR gene, further comprising a disrupted YenDF.

The present invention provides a *Yersinia entomophaga* bacterium comprising a modified YenR gene, further comprising a disrupted YenDF, wherein the disruption comprises a deletion of the YeHln, YeEln, YeIspn and/or YeOspn gene or within the YeHln, YeEln, YeIspn and/or YeOspn gene.

The present invention provides a *Yersinia entomophaga* bacterium comprising a modified YenR gene, further comprising the nucleotide sequence set forth in SEQ ID NO: 32. Accordingly, the present invention provides a *Yersinia entomophaga* bacterium comprising a modified YenR gene, a modified YmoA gene, one or more functional YeHln, YeEln, YeIspn and YeOspn genes and further comprising a modified YenDF, or any combination thereof.

As mentioned above, *Yersinia entomophaga* produces a toxin composition that is lethal for insects. Also, individual components of the toxin composition may be lethal or harmful for insects. Accordingly, it is evident for the skilled person that individual components of the toxin composition may be isolated/purified from the toxin composition. For example, the multiprotein complex YenTc (also termed YenTc toxin herein) is lethal or harmful for insects. Accordingly, it is evident for the skilled person that the YenTc toxin of the toxin composition may be isolated/purified from the toxin composition. The skilled person knows that a subunit of the YenTc toxin may be modified (e.g. fused to an affinity tag) to simplify isolation/purification of the YenTc toxin. A non-limiting example of a YenTc toxin subunit that may be modified is YenA2. A YenTc toxin can be purified using any subunit. YenTc is a multisubunit structure which is fully assembled in the cytoplasm of *Yersinia entomophaga.* thus, when using a modified YenTc subunit for purification any subunit can be used to purify the whole YenTc toxin. For example, an affinity purification of a tagged YenA2 subunit will results in recovery of the whole YenTc instead of the subunit only. YenTc comprises the subunits Chi1, Chi2, YenA1, YenA2, YenB, YenC1, YenC2 and RHS2 which form the fully assembled YenTc holotoxin. Accordingly, in the sense of the present invention a YenTc preferably comprises Chi1, Chi2, YenA1, YenA2, YenB, YenC1, YenC2 and RHS2. A YenTc may also consist of Chi1, Chi2, YenA1, YenA2, YenB, YenC1, YenC2 and RHS2. Accordingly, any one of these YenTc subunits (e.g. Chi1, Chi2, YenA1, YenA2, YenB, YenC1, YenC2 and/or RHS2) can be modified and then such (a) modified YenTc subunit(s) can be used for to purify the whole YenTc toxin. The explanations given herein above to modified YenA2 (e.g. tagged YenA2) apply, mutatis mutandis, to any other YenTc subunits (e.g. Chi1, Chi2, YenA1, YenA2, YenB, YenC1, YenC2 and/or RHS2).

Accordingly, in yet another aspect, the *Yersinia entomophaga* bacterium comprising a modified YenR gene of the present invention can further comprise a modified YenA2 or YenA2 gene. YenA2 relates to the UniProt accession number B6A878 and is a component/subunit of the YenTc toxin. Modification of the YenA2 subunit or YenA2 gene is particularly beneficial in the present invention, as the YenA2 subunit can be modified to allow for purification of a YenTc toxin. In general, the invention is not limited to any modification of a YenTc subunit. Rather any YenTc subunit can be modified to comprise a modification allowing for purification of the YenTc toxin. A modification of a subunit preferably does not interfere with the toxicity of the YenTc toxin. A YenTc subunit or a gene encoding the same can be one or more of or a Chi1, Chi2, RHS2, YenA1, YenA2, YenB, YenC1, YenC2 or a combination thereof. A YenTc subunit or gene encoding the same can also comprise multiple modifications. A modification of a YenTc subunit or a gene encoding the same can be one or more of His-tag, FLAG tag, polycysteine tag and strep tag. A modification of a YenTc subunit or gene encoding the same can also comprise multiple copies of one or more of His-tag, FLAG tag, polycysteine tag and strep tag or any combinations thereof.

Accordingly, the present invention provides a *Yersinia entomophaga* bacterium comprising a modified YenR gene, further comprising a modified YenA2 or a modified YenA2 gene.

In general a tag can be introduced at any position of a YenTc subunit or gene encoding the same, such as internal, N-terminal, and/or C-terminal or internal, 5' and/or 3' of the coding sequence. The position of a tag can be chosen based on preferred parameters such as affinity, accessibility, and functionality, i.e. toxicity of the YenTc toxin. A tag can be fused to a YenTc subunit or introduced into a gene encoding a YenTc subunit using a linker sequence, such as a peptide linker. The invention is not particularly limited to a linker sequence of any size, the skilled person is rather aware that any linker providing good affinity of the corresponding tag and which does not decrease the potency of the YenTc subunit or the YenTc toxin can be used. Accordingly, the present invention provides a *Yersinia entomophaga* bacterium comprising a modified YenR gene, further comprising a modified YenA2 or a modified YenA2 gene, wherein the modified YenA2 comprises an internal, N-terminal and/or C-terminal purification tag, and/or, wherein the modified YenA2 gene comprises a sequence encoding a purification tag, optionally wherein the modified YenA2 or modified YenA2 gene comprises a linker sequence.

In the sense of the present invention a tag is preferably a purification tag. A purification tag is an affinity tag that can be used to separate a protein of interest from other compounds. The invention is not particularly limited to any affinity tags for purification of e.g. a toxin. Any affinity tags that allow for high yield purification of a toxin, such as a YenTc toxin can be used. Exemplary affinity tags for purification that can be used in the sense of the present invention are one or more of His-tag, FLAG tag, polycysteine tag and strep tag.

Accordingly, the present invention provides a *Yersinia entomophaga* bacterium comprising a modified YenR gene, further comprising a modified YenA2 or a modified YenA2 gene, wherein the purification tag is one or more of His-tag, FLAG tag, polycysteine tag and strep tag.

In a preferred embodiment the subunit comprising the affinity tag is the YenA2 subunit of the YenTc toxin. Fusing the YenA2 subunit with an affinity tag is especially useful to produce a high yield of pure YenTc toxin.

Accordingly, the present invention provides a *Yersinia entomophaga* bacterium comprising a modified YenR gene, further comprising a modified YenA2, wherein the modified YenA2 comprises a C-terminal His-tag.

Preferably, the present invention provides a *Yersinia entomophaga* bacterium comprising a modified YenR gene, further comprising the nucleotide sequence set forth in SEQ ID NO: 18-21 or 32 and/or the amino acid sequence set forth in SEQ ID NO: 4-7.

The *Yersinia entomophaga* bacterium comprising a modified YenR gene, further comprising a modified YenA2 or a modified YenA2 gene of the present invention can be combined with any of the *Yersinia entomophaga* bacteria described herein. In particular, the *Yersinia entomophaga* bacterium comprising a modified YenR gene and further comprising a modified YenA2 or a modified YenA2 gene of the present invention can further comprise a modified YmoA gene, a functional YenDF, wherein YenDF comprises YeHln, YeEln, YeIspn and YeOspn and a modified YenDF or any combinations thereof.

Accordingly, the present invention provides a *Yersinia entomophaga* bacterium comprising a modified YenR gene, a modified YmoA gene, a functional YenDF, wherein YenDF comprises YeHln, YeEln, YeIspn and YeOspn, a modified YenDF, and a modified YenA2 or a modified YenA2 gene.

The present invention also provides a *Yersinia entomophaga* bacterium comprising a modified YenR gene, a plasmid expressing YmoA, a functional YenDF, wherein YenDF comprises YeHln, YeEln, YeIspn and YeOspn, a modified YenDF, and a modified YenA2 or a modified YenA2 gene.

The present invention also provides a *Yersinia entomophaga* bacterium comprising a modified YenR gene, the nucleotide sequence set forth in SEQ ID NO: 3, a functional YenDF, wherein YenDF comprises YeHln, YeEln, YeIspn and YeOspn, a modified YenDF, and a modified YenA2 or a modified YenA2 gene.

The present invention also provides a *Yersinia entomophaga* bacterium comprising the nucleotide sequence set forth in SEQ ID NO: 1 and/or 2, the nucleotide sequence set forth in SEQ ID NO: 3, the amino acid sequence set forth in SEQ ID NOs: 4-7, the amino acid sequence set forth in SEQ ID NO: 9.

The amino acid sequence set forth in SEQ ID NO: 9 encodes a modified YenA2. In particular, SEQ ID NO: 9 encodes a YenA2 comprising a C-terminal His-tag.

The features of a modified YenR gene, a modified YmoA gene or a YmoA plasmid, a functional YenDF, wherein YenDF comprises YeHln, YeEln, YeIspn and YeOspn, and a modified YenA2 or a modified YenA2 gene can be combined at will in the sense of the present invention to generate a modified *Yersinia entomophaga* bacterium. Different combinations of said features can be used to achieved different properties. For example, a modified YenDF can be used to avoid secretion of a toxin through lysis of the *Yersinia entomophaga* bacterium. Accordingly, the skilled person understands that an appropriate combination of features is desired for different applications.

The *Yersinia entomophaga* bacterium as described herein is especially useful for toxin production. Secretion of the produced toxins can be induced by increasing the pH of the bacteria culture or by transferring the bacteria to a higher pH environment, such as a buffer. In some cases, it is not desired that the bacteria secrete the toxins. Secretion can be avoided by maintaining the bacteria in a lower pH environment, such as a culture or buffer. Thus, the *Yersinia entomophaga* bacterium as described herein can be stored in a storage buffer which prevents secretion. In general, the pH and the ingredients of the storage buffer are not particularly limited. Any buffer which prevents the secretion of toxins can be used according to the present invention. However, it has been found that a storage buffer with a pH of about 4.5-6.0 efficiently prevents secretion of a toxin. In a preferred embodiment a storage buffer with a pH of about 5.5 prevents secretion of a toxin. The bacteria can be stored until a secretion of toxins is desired, which can be induced by increasing the pH of the buffer or transferring the bacteria to a higher pH environment, such as another buffer.

Accordingly, the invention provides a *Yersinia entomophaga* bacterium comprising a modified YenR gene, wherein the bacterium is comprised in a storage buffer at pH 4.5-6.0.

The invention also provides a *Yersinia entomophaga* bacterium comprising a modified YenR gene, wherein the bacterium is comprised in a storage buffer at pH 4.5, 4.6, 4.7, 4.8, 4.9, 5, 5.1, 5.2, 5.3, 5.4, 5.5, 5.6, 5.7, 5.8, 5.9 or pH 6.0.

Preferably, the invention provides a *Yersinia entomophaga* bacterium comprising a modified YenR gene, wherein the bacterium is comprised in a storage buffer at pH 5.5.

The buffer of the present invention is not particularly limited, rather any suitable buffer can be used. An exemplary buffer in the sense of the present invention can comprise 20 - 100 mM MES-NaOH, 100 mM NaCl at a variable pH of about 5-12. As described herein the pH can depend on whether culturing or secretion is desired.

In another aspect, the present invention provides a toxin composition. A toxin is a naturally occurring organic poison produced by metabolic activities of living cells. In the sense of the present invention a toxin can also be modified to comprise one or more compounds not occurring naturally. Toxins occur especially as a protein or conjugated protein. In the present invention a toxin is preferably a *Yersinia entomophaga* toxin. *Yersinia entomophaga* bacteria produce and secrete a highly devastating toxin cocktail comprising Cbp, Chi1, Chi2, Chi3, NucA, Pil36, PirA, PirB, StcE, Tlh, YenA1, YenA2, YenB, YenC1, YenC2, RHS2. The above-mentioned compounds comprised in the toxin cocktail relate to the following UniProt accession numbers in parenthesis: Cbp (A0A3S6EXR6), Chi1 (B6A876), Chi2 (B6A879), Chi3 (A0A3S6F1Q8), NucA (A0A3S6F4M5), PiI36 (A0A3S6F569), PirA (A0A3S6F007), PirB (A0A3S6F043), StcE (A0A3S6EYX4), Tlh (A0A3S6F052), YenA1 (B6A877), YenA2 (B6A878), YenB (B6A880), YenC1 (B6A881), YenC2 (B6A882), RHS2 (A0A2D0TC51). Accordingly, the present invention provides a toxin composition comprising Cbp, Chi1, Chi2, Chi3, NucA, Pil36, PirA, PirB, StcE, Tlh, YenA1, YenA2, YenB, YenC1, YenC2, RHS2. The invention also provides a toxin composition comprising one or more of Cbp, Chi1, Chi2, Chi3, NucA, Pil36, PirA, PirB, StcE, Tlh, YenA1, YenA2, YenB, YenC1, YenC2, RHS2 or any combination thereof. In particular, the invention provides a toxin composition comprising 2 or more, 3 or more, 4 or more, 5 or more, 6 or more, 7 or more, 8 or more, 9 or more, 10 or more, 11 or more, 12 or more, 13 or more, 14 or more, 15 or more, or 16 components selected from Cbp, Chi1, Chi2, Chi3, NucA, Pil36, PirA, PirB, StcE, Tlh, YenA1, YenA2, YenB, YenC1, YenC2, RHS2. Accordingly, the invention provides a toxin composition comprising 1, 2, 3, 4, 5, 6, 7, 8, 9, 10, 11, 12, 13, 14, 15, or 16 components selected from Cbp, Chi1, Chi2, Chi3, NucA, Pil36, PirA, PirB, StcE, Tlh, YenA1, YenA2, YenB, YenC1, YenC2, RHS2. It is preferred herein that a toxin composition comprises all of Cbp, Chi1, Chi2, Chi3, NucA, Pil36, PirA, PirB, StcE, Tlh, YenA1, YenA2, YenB, YenC1, YenC2, RHS2. YenTc is assembled by the subunits Chi1, Chi2, YenA1, YenA2, YenB, YenC1, YenC2 and RHS2. Thus, it is preferred herein, that a toxin composition comprises at least Chi1, Chi2, YenA1, YenA2, YenB, YenC1, YenC2 and RHS2. Accordingly, the invention provides a toxin composition comprising at least Chi1, Chi2, YenA1, YenA2, YenB, YenC1, YenC2 and RHS2. A toxin composition in the sense of the present invention can also comprise further toxins or virulence factors such as Cbp, Chi3, NucA, Pil36, PirA, PirB, StcE and Tlh. Accordingly, the invention provides a toxin composition comprising at least Chi1, Chi2, YenA1, YenA2, YenB, YenC1, YenC2 and RHS2, and optionally Cbp, Chi3, NucA, Pil36, PirA, PirB, StcE and Tlh. The invention provides a toxin composition comprising the YenTc components Chi1, Chi2, YenA1, YenA2, YenB, YenC1, YenC2 and RHS2 and the toxin or virulence components Cbp, Chi3, NucA, Pil36, PirA, PirB, StcE and Tlh.

In another aspect the invention also provides a toxin composition consisting of Cbp, Chi1, Chi2, Chi3, NucA, Pil36, PirA, PirB, StcE, Tlh, YenA1, YenA2, YenB, YenC1, YenC2, RHS2 and optionally a suitable carrier. All toxin compositions provided herein can optionally comprise a suitable carrier.

A toxin composition as described herein preferably comprises at least the fully assembled YenTc toxin.

*Yersinia entomophaga* is a close relative of the deadly human pathogen *Yersinia pestis* that has instead evolved to target insects. The toxin composition secreted by a *Yersinia entomophaga* bacterium is extremely lethal to insects.

In the sense of the present invention insects are pancrustacean hexapod invertebrate animals of the class Insecta, especially beetles and moths (e.g. plaintain moths, porina moths), and specifically insect species of the order Coleoptera (e.g. *Costelytra zealandica, Heteronychus arator*) and/or Lepidoptera (e.g. *Plutella xylostella, Scopula rubraria, Epyaxa rosearia, Wiseana spp.*)*.* In the sense of the present invention an insect may be one or more of *Plutella xylostella, Helicoverpa amigera, Cydia pomonella, Spodoptera litura, Epiphyas postvittana, Pieris rapae, Costelytra zealandica, Adoryphorus couloni, Acrossidius tasmaniae, Odontria sp., Locusta migratoria, Wiseana spp (e.g. Wiseana copularis), Galleeria mellonella, Teia anartoides, Planotortrix notophaea, Planotortrix excessana, Ctenoptusis spp., Pericoptus truncatus, Hylastes ater, Otiorhynchus sulcatus, Sitona lepidus, Doleromyrma darwiniana, Vespula vulgaris, Pratylenchus penetrans, Scopula rubraria, Epyaxa rosearia, Heteronychus arator* or larvae/caterpillar thereof. The term "insect" incudes any developmental stage thereof, including the larval stage/caterpillar stage and adult stage.

In the sense of the present invention an insect is preferably the plantain moth *Scopula rubraria* and/or *Epyaxa rosearia.* These plantain moths are native to New Zealand and Australia and infest plantain crops in large numbers. It is reported that *Epyaxa* also feeds and damages clover. The *Yersinia entomophaga* bacterium, toxin composition, YenTc toxin or population of soldier cells of the present invention can be used to contact the caterpillars of *Scopula rubraria* and/or *Epyaxa rosearia* and therefore control infestation by killing them avoiding infestation of plantain crops at the caterpillar and consequently adult stage after metamorphosis. In addition, killing the caterpillars of *Scopula rubraria* and/or *Epyaxa rosearia* has the advantage that eating damage to plants, such as crops can be avoided. For example, the *Scopula rubraria* and/or *Epyaxa rosearia* caterpillars can be contracted with a spray comprising *Yersinia entomophaga* bacterium, toxin composition, YenTc toxin or population of soldier cells of the present invention. *Scopula rubraria* and/or *Epyaxa rosearia* caterpillars can be contacted directly on any surface, such as on the ground or on a plant such as a plantain crop and/or clover. In general, *Scopula rubraria* and/or *Epyaxa rosearia* caterpillars are surface dwellers which allows for contacting the same e.g. with a spray composition.

In another preferred aspect the insect can be the black beetle *Heteronychus arator. Heteronychus arator* is a species of beetle in the subfamily Dynastinae (the rhinoceros beetles). It is commonly called African black beetle or black lawn beetle. It is native to Africa, and it is an introduced species in Australia and on the North Island of New Zealand. *Heteronychus arator* damages lawns, pastures, turf, crops, garden flowers, trees and shrubs by their root feeding larvae. Accordingly, the *Yersinia entomophaga* bacterium, toxin composition, YenTc toxin or population of soldier cells of the present invention can be used to contact *Heteronychus arator* or larvae thereof. In general, it is preferred to contact the adult *Heteronychus arator* since the larvae are underground dwellers and thus are difficult to contact, e.g. with a spray composition. However, by reducing the adult population the larvae population is indirectly reduced as well resulting in efficient pest control. The underground dwelling larvae of *Heteronychus arator* can for example be contacted with a composite bait comprising the *Yersinia entomophaga* bacterium, toxin composition, YenTc toxin or population of soldier cells of the present invention as described herein.

In another preferred aspect an insect can be a moth or larvae of the genus *Wiseana.* Accordingly, an insect can be *Wiseana cervinata, Wiseana copularis, Wiseana fuliginea, Wiseana jocosa Wiseana mimica, Wiseana signata, Wiseana umbraculata.* Larvae of *Wiseana* feed on various grasses and clover and are thus a major threat to agricultural areas such as pastures. Accordingly, the *Yersinia entomophaga* bacterium, toxin composition, YenTc toxin or population of soldier cells of the present invention can be used to contact and kill moths or larvae of the genus *Wiseana.*

Generally, the herein provided *Yersinia entomophaga* bacterium, toxin composition, YenTc toxin or population of soldier cells of the present invention can be used alone for the purposes indicated herein, such as killing pests/pesticide etc. (i.e. as sole active agent, e.g. in a pesticide composition). It is however also envisaged that the herein provided *Yersinia entomophaga* bacterium, toxin composition, YenTc toxin or population of soldier cells of the present invention can be used in combination with other techniques to control pests/insects, e.g. in combination with other pesticides/insecticides, such as commercially available pesticides/insecticides, in particular biopesticides and/or beneficial organisms.

For example, in case of black beetle, the herein provided *Yersinia entomophaga* bacterium, toxin composition, YenTc toxin or population of soldier cells of the present invention can be used in combination with sowing insecticide-coated seed to kill black beetle adults when grass seed is germinating. Also, ryegrass cultivars containing the endophyte AR37 might be used in combination with the *Yersinia entomophaga* bacterium, toxin composition, YenTc toxin or population of soldier cells of the present invention in this context.

Accordingly, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is toxic to invertebrate animals, preferably to invertebrate animals belonging to the class of Insecta as defined above.

As described herein, the *Yersinia entomophaga* bacterium comprising a modified YenR gene (as well as a population thereof), soldier cells (or a population thereof), the toxin composition and/or YenTc toxin can be used as a pesticide, specifically insecticide.

When the *Yersinia entomophaga* bacterium comprising a modified YenR gene (as well as a population thereof) or the soldier cells (or a population thereof) are to be used as pesticide, the expression of the YenR gene or gene product thereof is to be increased as described herein, specifically when/before the bacteria / soldier cells are applied/contacted/administered to the plants/crops (and/or pests/insects) as insecticide. It is envisaged that the increased YenR expression results in production of the toxin / toxin composition by the bacteria /soldier cells so that the bacteria will exert their (toxic) effect when applied to the plants/crops (and/or pests/insects) and subsequently being taken up by the pests/insects.

For example, the *Yersinia entomophaga* bacterium comprising a modified YenR gene (as well as a population thereof) might be packaged (for sale) as dry powder or in storage buffer (i.e. without increased expression of the YenR gene or gene product thereof). Prior to applying/contacting/administering the bacteria (e.g. in dry powder form or in storage buffer) the expression of the YenR gene or gene product thereof is induced (as described herein), resulting in production of the toxin composition/toxin in the bacteria in an amount sufficient to induce a toxic effect on the insects, specifically when a suitable amount of the bacteria is applied/contacted/administered to the pest/insect.

Alternatively, the *Yersinia entomophaga* bacterium comprising a modified YenR gene (as well as a population thereof) or the soldier cells (or a population thereof) having increased expression of the YenR gene or gene product might be packaged (for sale), e.g. as dry powder or in storage buffer, wherein the bacteria comprise the toxin composition/toxin in an amount sufficient to have a toxic effect on the insects, specifically when a suitable amount of the bacteria is applied/contacted/administered to the pest/insect.

In general, the *Yersinia entomophaga* bacterium, toxin composition, YenTc toxin or population of soldier cells of the present invention can be used to contact a pest in any form. For example, the *Yersinia entomophaga* bacterium, toxin composition, YenTc toxin or population of soldier cells of the present invention can be used in a method to kill pests combined with other techniques to control pests. Other techniques can for example include an additional treatment with an insecticidal drug or chemical. The *Yersinia entomophaga* bacterium, toxin composition, YenTc toxin or population of soldier cells of the present invention can be used to coat the seeds of plants before growing them. This has the advantage that pathogens that may harm the seed as such or during the early stage of growth can be killed. An example of a pathogen that can be contacted with a spray and e.g. a seed coating comprising the *Yersinia entomophaga* bacterium, toxin composition, YenTc toxin or population of soldier cells of the present invention is the black beetle *Heteronychus arator.* It is envisioned that the adult beetles are targeted with e.g. a spray and the underground dwelling larvae can be targeted with e.g. a seed coating or the like. The *Yersinia entomophaga* bacterium, toxin composition, YenTc toxin or population of soldier cells of the present invention can also be combined with other means of pest control, such as endophytes. For example, ryegrass cultivars containing the novel endophyte AR37 have been shown to give protection against other pests such as *Listronotus bonariensis.* When using the *Yersinia entomophaga* bacterium, toxin composition, YenTc toxin or population of soldier cells of the present invention in pest control the combination with such a ryegrass cultivars containing the novel endophyte AR37 may further increase the desired insecticidal effects.

In another aspect, the *Yersinia entomophaga* bacterium, toxin composition, YenTc toxin or population of soldier cells of the present invention can be applied together with a bait. The advantage of application with a bait is that a pathogen, such as an insect or larvae thereof will be attracted to the bait and preferably ingest the bait including the active ingredient. After ingesting the *Yersinia entomophaga* bacterium, or population of soldier cells of the present invention including the bait, the bacteria will infest the pathogen and multiply eventually killing the host pathogen. Thus, the *Yersinia entomophaga* bacterium, or population of soldier cells of the present invention has the advantage when being ingested by a pathogen, such as an insect host, that it will multiply, resulting in an increased number of bacteria and increased effectiveness of pest control. After ingesting the toxin composition or YenTc toxin of the present invention including the bait the pathogen, such as an insect, will be killed by disintegration of the gut. Thus, the formulation of the *Yersinia entomophaga* bacterium, toxin composition, YenTc toxin or population of soldier cells of the present invention as a composite bait is advantageous.

Accordingly, the invention provides the *Yersinia entomophaga* bacterium, toxin composition, YenTc toxin or population of soldier cells of the present invention formulated as a composite bait.

Generally, the *Yersinia entomophaga* bacterium, toxin composition, YenTc toxin or population of soldier cells of the present invention can be used in any form. For example, *Yersinia entomophaga* bacterium (or population thereof) and/or the population of soldier cells (or population thereof) can be formulated/used (and hence packaged/sold) as dried powder which is dissolved in water prior to application to the pests/insects and/or crops/plants and is applied as a (liquid) spray. As another, non-limiting example, the toxin / toxin composition can be formulated/used (and hence packaged/sold) in crystalline form which is dissolved in water prior to application to the pests/insects and/or crops/plants and is applied as a (liquid) spray.

Without being bound by theory, the *Yersinia entomophaga* bacterium comprising a modified YenR gene (as well as a population thereof) and/or the soldier cells (or a population thereof) provided herein might effect their toxicity by the following mechanism: It is documented in the art that the pH in the gut of insects is alkaline (often in the range of 8.2 to 11). Upon being taken up by the insects the *Yersinia entomophaga* bacterium comprising a modified YenR gene (as well as a population thereof) and/or the soldier cells (or a population thereof) provided herein secrete the toxin composition into the gut. The released toxin / toxin composition then effects its toxic effect, e.g. by destroying the gut epithelium.

When the toxin composition / toxin (e.g. packaged (for sale) as a pesticide/insecticide composition) is directly applied/contacted/administered to the plants/crops (and/or pests/insects), the toxin composition / toxin is subsequently taken up by the pests/insects and will exert its toxic effects.

Many insects are plant pathogens that result in an infestation killing or reducing the yield of a plant, such as a crop. Plant pathogenic insects may also infest plants that are not crops and are grown for aesthetic or other reasons e.g. shade in a park or garden area. In this case it is desire to avoid an infestation that may result in an aesthetically unpleasing plant or killing the plant. An aesthetically unpleasing plant is for example a plant that lost all green tissue such as leaves. Thus, a plant in the sense of the present invention can be a plant in an agricultural environment, such as a crop. A plant in the sense of the present invention can also be a plant in a park or garden environment e.g. bushes or trees.

Accordingly, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is toxic to plant pathogens belonging to the class of Insecta.

As described herein above, the toxin composition of the invention is lethal to insects. As such the toxin composition is especially useful for killing pests such as insects. For this, the toxin composition can be formulated as a pesticide composition. Pesticides are substances that are meant to control pests and include insecticides. Accordingly, the toxin composition can also be formulated as an insecticide composition. When formulated as a pesticide composition, specifically an insecticide composition, the composition can be in form of or further comprise a suspension capsule for the treatment of seeds or a bait in any of its forms, a combi pack in any of its forms, such as a concentrate, encapsulated, miscible, and ultra-low volume; tablets in any of their forms or in the form of ultra-low volume, such as oil-in-water or water-in-oil emulsions for the treatment of seeds in the form of gel or concentrate paste, emulsifiable gel, and gel for the treatment of seeds; in the form or dispersible, encapsulated, fine, soluble, and emulsifiable granules; in the form of macro and micro granules; in the form of paste; in the form of contact, wettable, and dispersible powder in the treatment of seeds; in the form of a solution for seed treatment, without excluding other possible forms applicable in the technical area of this application. The formula can optionally include carriers for the said microorganisms, such as clay, kaolin, talcum, zeolite, water, vegetable oils, and paraffinic or nonparaffinic minerals.

When formulated as a pesticide composition, specifically an insecticide composition, the composition can further comprise any suitable excipient. Suitable excipients include, for example, carrier molecules that include large, slowly metabolized macromolecules such as proteins, polysaccharides, polylactic acids, polyglycolic acids, polymeric amino acids and amino acid copolymers. Other exemplary excipients can include antioxidants (for example and without limitation, ascorbic acid), chelating agents (for example and without limitation, EDTA), carbohydrates (for example and without limitation, dextrin, hydroxyalkylcellulose, and hydroxyalkylmethylcellulose), stearic acid, liquids (for example and without limitation, oils, water, saline, glycerol and ethanol), wetting or emulsifying agents, pH buffering substances, and the like.

Accordingly, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is formulated as a pesticide composition.

The invention also provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is formulated as an insecticide composition.

The toxin composition of the invention can be formulated into a suitable carrier which allows for storage and application of the toxin composition. The invention is not particularly limited to any carrier, rather any carrier that allows for storage without significantly decreasing the activity, such as toxicity of the toxin composition can be used. When the toxin composition is applied to a plant it is desired that the carrier confers a certain level of viscosity to the toxin composition. A more viscous composition allows the toxin to remain on the plant for a longer period of time. A suitable carrier can comprise for example any of the excipients described herein.

Accordingly, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is formulated as a pesticide composition, optionally comprising a suitable carrier.

The invention also provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is formulated as an insecticide composition, optionally comprising a suitable carrier.

Since *Yersinia entomophaga* has evolved to target insects, the toxins derived from the same do not show toxicity to organisms other than insects. This is especially useful since the toxin composition of the present invention is safe, i.e. not toxic to other animals, such as mammals. When a toxin is used e.g. in pest control is its desirable to avoid toxicity towards other animals, such as mammals. In particular, pest control is applied in agricultural areas or garden or park areas where other animals are present. Thus, the toxin composition is safe for other animals not belonging to the class of Insecta. In particular, the toxin composition is also safe and does not harm beneficial organism even within the class of Insecta. Such beneficial organisms can be for example earthworms and/or honey bees. In the sense of the invention a bee or honey bee may be the western honey bee *Apis mellifera.*

The toxin composition is safe for mammals, such as humans. Accordingly, the toxin composition can be safely applied as a pest control, such as an insecticide in an area where humans are present, such as an agricultural, garden or park area. Another advantage of the invention is that toxin production and secretion does not occur at a temperature above 30°C which indicates an incompatible host environment.

Accordingly, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to animals with a body temperature of 30°C or higher.

The invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to animals not belonging to the class of Insecta.

The invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to mammals.

Preferably, the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to humans.

In another aspect the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to beneficial organisms within the class of Insecta.

In another aspect the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to earthworms or bees.

In another aspect the invention provides a toxin composition comprising Cbp, Chi1,Chi2, Chi3, NucA, Pil36, PirA, PirB, StcE, Tlh, YenA1, YenA2, YenB, YenC1, YenC2, RHS2, wherein the composition is not toxic to bees.

In another aspect the invention provides a toxin composition comprising Cbp, Chi1, Chi2, Chi3, NucA, Pil36, PirA, PirB, StcE, Tlh, YenA1, YenA2, YenB, YenC1, YenC2, RHS2, wherein the composition is not toxic to *Apis mellifera.*

In another aspect, the invention provides a method to produce a toxin composition, optionally further comprising formulating the toxin composition as pesticide composition, specifically insecticide composition. Formulating the toxin composition as pesticide composition, specifically insecticide composition, can comprise adding antioxidants (for example and without limitation, ascorbic acid), chelating agents (for example and without limitation, EDTA), carbohydrates (for example and without limitation, dextrin, hydroxyalkylcellulose, and hydroxyalkylmethylcellulose), stearic acid, liquids (for example and without limitation, oils, water, saline, glycerol and ethanol), wetting or emulsifying agents, pH buffering substances, and the like to the toxin composition.

As described herein above, a *Yersinia entomophaga* bacterium produces a highly devastating toxin cocktail comprising Cbp, Chi1, Chi2, Chi3, NucA, Pil36, PirA, PirB, StcE, Tlh, YenA1, YenA2, YenB, YenC1, YenC2, RHS2. The production of the toxins can further be increased by modifying the YenR gene in a *Yersinia entomophaga* bacterium as described herein above. Further modification of the YmoA gene, a functional YenDF, wherein YenDF comprises YeHln, YeEln, YeIspn and YeOspn, modification of the YenDF and modification of the YenA2 or YenA2 gene can further contribute to toxin production. Thus, the *Yersinia entomophaga* bacterium of the present invention is especially useful in a method to produce a high amount of a toxin composition.

Accordingly, the invention provides a method to produce a toxin composition comprising, a step of culturing a *Yersinia entomophaga* bacterium of the present invention.

The method comprises a step of culturing a *Yersinia entomophaga* bacterium of the present invention. Any of the *Yersinia entomophaga* bacteria described herein can be used in the method to produce a toxin composition. In particular, a *Yersinia entomophaga* bacterium comprising one or more of a modified YenR gene, a modified YmoA gene or a YmoA plasmid, a functional YenDF, wherein YenDF comprises YeHln, YeEln, YeIspn and YeOspn, a modified YenDF and a modified YenA2 or a modified YenA2 gene or any combination of the aforementioned.

Accordingly, the invention provides a method to produce a toxin composition comprising, a step of culturing a *Yersinia entomophaga* bacterium of the present invention, wherein the *Yersinia entomophaga* bacterium comprises a modified YenR gene.

The invention provides a method to produce a toxin composition comprising, a step of culturing a *Yersinia entomophaga* bacterium of the present invention, wherein the *Yersinia entomophaga* bacterium comprises a modified YenR gene and a modified YmoA gene or a YmoA plasmid. The invention also provides a method to produce a toxin composition comprising, a step of culturing a *Yersinia entomophaga* bacterium of the present invention, wherein the *Yersinia entomophaga* bacterium comprises a modified YenR gene and a modified YmoA gene or a YmoA plasmid and a functional YenDF, wherein YenDF comprises YeHln, YeEln, YeIspn and YeOspn. The method can optionally further comprise formulating the toxin composition as pesticide composition, specifically insecticide composition. The explanations and definitions provided above apply here mutatis mutandis.

The invention also provides a method to produce a toxin composition comprising, a step of culturing a *Yersinia entomophaga* bacterium of the present invention, wherein the *Yersinia entomophaga* bacterium comprises a modified YenR gene, and a modified YmoA gene or a YmoA plasmid, and a functional YenDF, wherein YenDF comprises YeHln, YeEln, YeIspn and YeOspn, and a modified YenDF.

The invention also provides a method to produce a toxin composition comprising, a step of culturing a *Yersinia entomophaga* bacterium of the present invention, wherein the *Yersinia entomophaga* bacterium comprises a modified YenR gene, and a modified YmoA gene or a YmoA plasmid, and a functional YenDF, wherein YenDF comprises YeHln, YeEln, YeIspn and YeOspn, and a modified YenDF, and a modified YenA2 or a modified YenA2 gene. Any of the aforementioned modifications can also be optional. It is however preferred that the *Yersinia entomophaga* bacterium comprises at least a modified YenR gene as described herein. Accordingly, the present invention also provides a method to produce a toxin composition comprising, a step of culturing a *Yersinia entomophaga* bacterium of the present invention, wherein the *Yersinia entomophaga* bacterium comprises a modified YenR gene, and optionally a modified YmoA gene or a YmoA plasmid, and optionally a functional YenDF, wherein YenDF comprises YeHln, YeEln, YeIspn and YeOspn, and optionally a modified YenDF, and optionally a modified YenA2 or a modified YenA2 gene.

As described herein a *Yersinia entomophaga* bacterium produces a highly devastating toxin cocktail. Thus, a *Yersinia entomophaga* bacterium of the present invention can be used to produce said in a method comprising a step of culturing a *Yersinia entomophaga* bacterium of the invention.

Accordingly, the invention provides a method to produce a toxin composition comprising, a step of culturing a *Yersinia entomophaga* bacterium of the present invention, wherein the toxin composition comprises one or more of Cbp, Chi1, Chi2, Chi3, NucA, Pil36, PirA, PirB, StcE, Tlh, YenA1, YenA2, YenB, YenC1, YenC2, RHS2 or a combination thereof.

The culturing step is not particularly limited to any culturing conditions. In general, any culturing conditions resulting in an effective growth of a *Yersinia entomophaga* bacterium of the present invention are envisioned. The culturing step can preferably comprise culturing a *Yersinia entomophaga* growth at an initial pH of about 7.0. However, lower pH values can also be used in the culturing steps.

Accordingly, the invention provides a method to produce a toxin composition comprising, a step of culturing a *Yersinia entomophaga* bacterium of the present invention, wherein the culturing step comprises culturing the *Yersinia entomophaga* bacterium at an initial pH of about 5.5, 5.6, 5.7, 5.8, 5.9, 6, 6.1, 6.2, 6.3, 6.4, 6.5, 6.6, 6.7, 6.8, 6.9, or 7.0.

The invention provides a method to produce a toxin composition comprising, a step of culturing a *Yersinia entomophaga* bacterium of the present invention, wherein the culturing step comprises culturing the *Yersinia entomophaga* bacterium at an initial pH of about 5.5-7.0. While grown in culture, the *Yersinia entomophaga* bacterium of the present invention can naturally acidify the culture media resulting in a pH of about 5.5-6.0. In this case the secretion step will commence with an initial culture pH of about 5.5-6.0 after the culturing step. Preferably, the invention provides a method to produce a toxin composition comprising, a step of culturing a *Yersinia entomophaga* bacterium of the present invention, wherein the culturing step comprises culturing the *Yersinia entomophaga* bacterium at an initial pH of about 7.0, further comprising culturing the bacteria until a pH of 5.5-6.0 is reached.

*Yersinia entomophaga* has evolved to target insects. Thus, the bacterium is bacteria is preferentially grown at less than about 30-40°C. Without necessarily being bound by theory any higher temperatures may mimic an incompatible host (e.g. a mammal) and thus impede bacterial growth. Preferably, the *Yersinia entomophaga* of the present invention is grown at less than about 30°C.

Accordingly, the invention provides a method to produce a toxin composition comprising, a step of culturing a *Yersinia entomophaga* bacterium of the present invention, wherein the culturing step comprises culturing the *Yersinia entomophaga* bacterium at less than about 30, 30.5, 31, 31.5, 32, 32.5, 33, 33.5, 34, 34.5, 35, 35.5, 36, 36.5, 37, 37.5, 38, 38.5, 39, 39.5, or 40°C.

The invention also provides a method to produce a toxin composition comprising, a step of culturing a *Yersinia entomophaga* bacterium of the present invention, wherein the culturing step comprises culturing the *Yersinia entomophaga* bacterium at less than about 30-40°C. Preferably, the invention provides a method to produce a toxin composition comprising, a step of culturing a *Yersinia entomophaga* bacterium of the present invention, wherein the culturing step comprises culturing the *Yersinia entomophaga* bacterium at less than about 30°C.

A good culture growth of the *Yersinia entomophaga* bacterium of the present invention can be realised if the bacteria is cultures at about 10-25°C, preferably at about 16-20°C.

Accordingly, the invention provides a method to produce a toxin composition comprising, a step of culturing a *Yersinia entomophaga* bacterium of the present invention, wherein the culturing step comprises culturing the *Yersinia entomophaga* bacterium at about 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, or 25°C, preferably at about 16, 16.5, 17, 17.5, 18, 18.5, 19, 19.5, or 20°C.

The invention also provides a method to produce a toxin composition comprising, a step of culturing a *Yersinia entomophaga* bacterium of the present invention, wherein the culturing step comprises culturing the *Yersinia entomophaga* bacterium at about 10-25°C, preferably at about 16-20°C.

Preferably, the invention provides a method to produce a toxin composition comprising, a step of culturing a *Yersinia entomophaga* bacterium of the present invention, wherein the culturing step comprises culturing the *Yersinia entomophaga* bacterium at about 10-25°C, preferably at about 16-20°C.

In general, any media which enables sufficient growth can be used to grow the *Yersinia entomophaga* bacterium of the present invention. Exemplary media that can be used to culture the *Yersinia entomophaga* bacterium of the present invention are lysogeny broth (LB). LB media is commonly used and typically comprises peptone 140, yeast extract and sodium chloride in a ratio of 2:1:1, respectively. For example, SOC media can also be used to culture the *Yersinia entomophaga* bacterium of the present invention. SOC media is commonly used and typically comprises 2% tryptone, 0.5% yeast extract, 10 mM NaCl, 2.5 mM KCl, 10 mM MgC12, 10 mM MgSO4, and 20 mM glucose. SOC media is preferred herein as it acidifies naturally during bacterial growth allowing for on demand pH dependent release. When using other media that do not acidify naturally, the bacteria may secrete the toxin composition consistently during growth. However, the toxin composition can still be harvested after growth since the composition is stable in growth media.

When a *Yersinia entomophaga* bacterium enters a natural host, i.e. an insect, host markers, such as complex organic compounds are present in the host environment. Thus, a *Yersinia entomophaga* bacterium of the present invention is preferably grown in the presence of complex organic compounds. Exemplary complex organic compounds used for culturing a *Yersinia entomophaga* bacterium of the present invention are tryptone and its constituents, yeast extract and its constituents, and sugars, such as glucose.

Accordingly, the invention provides a method to produce a toxin composition comprising, a step of culturing a *Yersinia entomophaga* bacterium of the present invention, wherein the culturing step comprises culturing the *Yersinia entomophaga* bacterium in the presence of markers mimicking the *Yersinia entomophaga* host, such as complex organic compounds.

A *Yersinia entomophaga* bacterium of the present invention can produce a substantial amount of toxins when grown in a culture. The toxins produced by a *Yersinia entomophaga* bacterium of the present invention are localized in the cytoplasm of the bacterium. Thus, the toxins have to be secreted to be accessible and recover the toxins.

Accordingly, the present invention provides a method to produce a toxin composition comprising, a step of culturing a *Yersinia entomophaga* bacterium of the present invention, further comprising a secretion step.

There are multiple means how the toxins can be secreted. Naturally, the T10SS can be activated by an increase in the pH of the environment which can occur for example in the more alkaline posterior midgut of an insect host. Thus, secretion of a toxin produced by a *Yersinia entomophaga* bacterium of the present invention can be induced by increasing the pH of the bacterial culture or transferring the bacteria to a high pH environment. A high pH environment in the sense of the present invention can be a buffer, such as a secretion buffer. In general, the pH of the initial culture of a *Yersinia entomophaga* bacterium of the present invention will change through the growth phase from about pH 7.0 to a final pH of about pH 5.5-6.0. To induce the secretion, the pH is raised from the final culture pH of about pH 5.5-6.0 to about pH 6.3-10.0. pH induced secretion by cell lysis will start immediately after increasing the pH or transferral of the bacteria to a high pH environment. In general, the pH can be increased to a pH of about 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or about 10 to induce secretion. The *Yersinia entomophaga* bacterium of the present invention can be incubated at the increased pH or in the high pH environment between 5-30 minutes or until a toxin composition is efficiently secreted. Accordingly, a *Yersinia entomophaga* bacterium of the present invention can be incubated at pH 6.3-10 for 5, 6, 7, 8, 9, 10, 11, 12, 13, 14, 15, 16, 17, 18, 19, 20, 21, 22, 23, 24, 25, 26, 27, 28, 29, or 30 minutes. Preferably, a *Yersinia entomophaga* bacterium of the present invention can be incubated at pH 6.9-8.9 for 5, 6, 7, 8, 9, 10, 11, 12, 13, 14, 15, 16, 17, 18, 19, 20, 21, 22, 23, 24, 25, 26, 27, 28, 29, or 30 minutes.

Accordingly, the present invention provides a method to produce a toxin composition comprising, a step of culturing a *Yersinia entomophaga* bacterium of the present invention, further comprising a secretion step, wherein the secretion step comprises
(i) increasing the pH of the initial culture from pH 5.5, 5.6, 5.7, 5.8, 5.9, or 6 to pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 and
(ii) incubating the *Yersinia entomophaga* bacterium at pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10.

The present invention also provides a method to produce a toxin composition comprising, a step of culturing a *Yersinia entomophaga* bacterium of the present invention, further comprising a secretion step, wherein the secretion step comprises
(i) increasing the pH of the initial culture from pH 5.5-6.0 to pH 6.3-10.0 and
(ii) incubating the *Yersinia entomophaga* bacterium at pH 6.3-10.0.

The present invention provides a method to produce a toxin composition comprising, a step of culturing a *Yersinia entomophaga* bacterium of the present invention, further comprising a secretion step, wherein the secretion step comprises
(i) transferring the bacterium to a pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 buffer and
(ii) incubating the *Yersinia entomophaga* bacterium at pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10.

The present invention also provides a method to produce a toxin composition comprising, a step of culturing a *Yersinia entomophaga* bacterium of the present invention, further comprising a secretion step, wherein the secretion step comprises
(i) transferring the bacterium to a pH 6.3-10.0 buffer and
(ii) incubating the *Yersinia entomophaga* bacterium at pH 6.3-10.0.

Preferably, the invention provides a method to produce a toxin composition comprising, a step of culturing a *Yersinia entomophaga* bacterium of the present invention, further comprising a secretion step, wherein the secretion step comprises
(i) increasing the pH of the initial culture from pH 5.5, 5.6, 5.7, 5.8, 5.9, or 6.0 to pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9 and
(ii) incubating the *Yersinia entomophaga* bacterium at pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9.

Preferably, the invention provides a method to produce a toxin composition comprising, a step of culturing a *Yersinia entomophaga* bacterium of the present invention, further comprising a secretion step, wherein the secretion step comprises
(i) increasing the pH of the initial culture from pH 5.5-6.0 to pH 6.9-8.9 and
(ii) incubating the *Yersinia entomophaga* bacterium at pH 6.9-8.9.

Preferably, the invention provides a method to produce a toxin composition comprising, a step of culturing a *Yersinia entomophaga* bacterium of the present invention, further comprising a secretion step, wherein the secretion step comprises
(i) transferring the bacterium to a pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9 buffer and
(ii) incubating the *Yersinia entomophaga* bacterium at pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9.

Preferably, the invention provides a method to produce a toxin composition comprising, a step of culturing a *Yersinia entomophaga* bacterium of the present invention, further comprising a secretion step, wherein the secretion step comprises
(i) transferring the bacterium to a pH 6.9-8.9 buffer and
(ii) incubating the *Yersinia entomophaga* bacterium at pH 6.9-8.9.

Lysis of the *Yersinia entomophaga* bacterium of the present invention can also be realized by other means such as mechanical lysis, chemical lysis, physical lysis or biological lysis such as enzymatic lysis. An enzymatic lysis can be performed by and/or within an insect host. A mechanical or enzymatic lysis is especially useful if the YenDF has been modified according to the present invention. In this case, pH induced lysis might not be efficient or desired, and the toxins can be secreted by a secretion step comprising mechanical lysis or enzymatic lysis. In the sense of the present invention any means for mechanical or enzymatic lysis can be used as long as a toxin is efficiently secreted and/or recovered from a lysed *Yersinia entomophaga* bacterium. Exemplary means for mechanical lysis are disruption by sonication, bead milling/beating, and high pressure homogenization. Exemplary means for non-mechanical lysis are osmotic shock and use of detergents. Exemplary means for enzymatic lysis are treatment with lysozyme.

Accordingly, the present invention provides a method to produce a toxin composition comprising, a step of culturing a *Yersinia entomophaga* bacterium of the present invention, further comprising a secretion step, wherein the secretion step comprises a lysis step comprising mechanical lysis or enzymatic lysis of the *Yersinia entomophaga* bacterium, optionally wherein the lysis step is performed within an insect host.

The present invention also provides a method to produce a toxin composition comprising, a step of culturing a *Yersinia entomophaga* bacterium of the present invention, further comprising a secretion step, wherein the secretion step comprises a lysis step comprising mechanical lysis or enzymatic lysis of the *Yersinia entomophaga* bacterium, wherein the lysis step is performed within an insect host.

The above described means for mechanical lysis, chemical lysis, physical lysis or biological lysis such as enzymatic lysis can be combined with a pH induced secretion step as described herein.

Secretion of a toxin can also be induced by anaerobic culture conditions such anaerobic stress. The *Yersinia entomophaga* bacterium of the present invention undergoes controlled lysis under sudden anaerobic stress which causes a loss in the proton motive force (PMF) due to decrease of cytoplasmic pH.

Accordingly, the present invention provides a method to produce a toxin composition comprising, a step of culturing a *Yersinia entomophaga* bacterium of the present invention, further comprising a secretion step, wherein the secretion step comprises incubating the *Yersinia entomophaga* bacterium under anaerobic conditions, wherein the anaerobic conditions result in secretion-inducing anaerobic stress.

The present invention allows to obtain a toxin composition of high purity. Accordingly, the invention provides a method to produce a toxin composition comprising, a step of culturing a *Yersinia entomophaga* bacterium of the present invention, further comprising a purification step. The purification step can be combined with a preceding secretion step as described herein above. Accordingly, the invention provides a method to produce a toxin composition comprising, a step of culturing a *Yersinia entomophaga* bacterium of the present invention, further comprising a secretion step and a purification step.

In general, it is desired to obtain a toxin composition as pure as possible. For this, any remnants from the cell lysis are to be removed. In particular, such remnants may be cell debris of a *Yersinia entomophaga* bacterium which has secreted a toxin composition according to the secretion step of the present invention. Cell debris can be removed by centrifuging the bacterial solution at a speed and for a time sufficient to separate the cellular fraction from the toxin fraction. In general, the invention is not particularly limited to any centrifugation speed or time parameters, rather any parameters can be used by the skilled person that effectively result in a separation of a cellular fraction and a toxin fraction. A culture of the *Yersinia entomophaga* bacterium of the present invention can be centrifuged at 2000-6000g for 1-10 minutes. A culture of the *Yersinia entomophaga* bacterium of the present invention can be centrifuged at 2000, 2500, 3000, 3500, 4000, 4500, 5000, 5500, or 6000g for 1, 2, 3, 4, 5, 6, 7, 8, 9, or 10 minutes. Preferably, a culture of the *Yersinia entomophaga* bacterium of the present invention can be centrifuged at 4000g for 5 minutes.

After a toxin fraction has been separated, it can be further purified by a filtration step. By filtration any remaining debris or remnants from the previous secretion steps can be removed. In general, the invention is not particularly limited to any filtration parameters such as exclusion or filter or pore size. Any filter can be used that results in a toxin composition of desired purity without compromising the amount and activity, i.e. toxicity of the toxins therein. Accordingly, the *Yersinia entomophaga* bacterium of the present invention can be filtrated with a 0.2-0.22 µm, preferably 0.22 µm pore size filter.

The above-described centrifuging and filtrating steps can be combined with each other and with further purification steps and the secretion steps as described herein.

Accordingly, the invention provides a method to produce a toxin composition comprising, a step of culturing a *Yersinia entomophaga* bacterium of the present invention, further comprising a purification step, wherein the purification step comprises centrifuging the *Yersinia entomophaga* bacterium.

Preferably, the invention provides a method to produce a toxin composition comprising, a step of culturing a *Yersinia entomophaga* bacterium of the present invention, further comprising a purification step, wherein the purification step comprises centrifuging the *Yersinia entomophaga* bacterium at 4000g for 5 minutes.

The invention provides a method to produce a toxin composition comprising, a step of culturing a *Yersinia entomophaga* bacterium of the present invention, further comprising a purification step, wherein the purification step comprises filtrating the *Yersinia entomophaga* bacterium. The invention provides a method to produce a toxin composition comprising, a step of culturing a *Yersinia entomophaga* bacterium of the present invention, further comprising a purification step, wherein the purification step comprises filtrating the *Yersinia entomophaga* bacterium with a 0.22 µm pore size filter.

The invention provides a method to produce a toxin composition comprising, a step of culturing a *Yersinia entomophaga* bacterium of the present invention, further comprising a purification step, wherein the purification step comprises centrifuging and filtrating the *Yersinia entomophaga* bacterium.

The invention provides a method to produce a toxin composition comprising, a step of culturing a *Yersinia entomophaga* bacterium of the present invention, further comprising a purification step, wherein the purification step comprises
(i) centrifuging the *Yersinia entomophaga* bacterium at 4000g for 5 minutes and
(ii) filtrating the *Yersinia entomophaga* bacterium with a 0.22 µm pore size filter.

The above-described purification steps can be combined with a secretion step as described herein above that precedes the purification steps.

The method to produce a toxin composition of the present invention can further comprise a recovery step. A recovery step is used to harvest the compounds of the toxin composition. Accordingly, a recovery step can comprise recovering one or more of Cbp, Chi1, Chi2, Chi3, NucA, Pil36, PirA, PirB, StcE, Tlh, YenA1, YenA2, YenB, YenC1, YenC2, RHS2 or a combination thereof from a culture of the *Yersinia entomophaga* bacterium. A recovery step can also comprise recovering a YenTc toxin from a culture of the *Yersinia entomophaga* bacterium. A recovery step can also comprise concentrating one or more of Cbp, Chi1, Chi2, Chi3, NucA, Pil36, PirA, PirB, StcE, Tlh, YenA1, YenA2, YenB, YenC1, YenC2, RHS2 or a combination thereof. A recovery step can also comprise concentrating a YenTc toxin. The above-mentioned recovery steps can be combined with any one of or all of a culturing step, a secretion step and a purification step of the present invention. The above-mentioned recovery step can also be combined with each over in on recovery step. Such a recovery step is especially useful if a toxin composition containing certain compounds is desired. For example, a recovery step can only recover a YenTc toxin from the toxin composition. In general, any desired compound from the toxin composition or all compounds thereof can be recovered in the sense of the present invention. It is preferred that a YenTc toxin is recovered from the toxin compound. A recovery step can comprise recovery of a toxin or subunit thereof comprising an affinity tag.

Accordingly, the present invention provides a method to produce a toxin composition comprising, a step of culturing a *Yersinia entomophaga* bacterium of the present invention, further comprising a recovery step, wherein the recovery step comprises
(i) recovering one or more of Cbp, Chi1, Chi2, Chi3, NucA, Pil36, PirA, PirB, StcE, Tlh, YenA1, YenA2, YenB, YenC1, YenC2, RHS2 or a combination thereof from a culture of the *Yersinia entomophaga* bacterium and/or
(ii) recovering a YenTc toxin from a culture of the *Yersinia entomophaga* bacterium and/or optionally
(iii) concentrating the substances or combinations thereof of (i) and/or the YenTc toxin of (ii).

In another aspect the invention also provides a toxin composition obtained by or as obtainable by the methods of the present invention.

In another aspect, the invention provides a *Yersinia entomophaga* bacterium of the present invention further comprising a modified gene encoding a YenTc subunit or a modified YenTc subunit. YenTc is a multimeric pore-forming toxin comprising several subunits. In general, the invention is not limited to any modification of a YenTc subunit or gene encoding the same. Rather any YenTc subunit or gene encoding the same can be modified to comprise a modification. Such a modification can for example allow purification or recovery of entire YenTc toxin. A modification of a subunit preferably does not interfere with the toxicity of the YenTc toxin. A YenTc subunit or a gene encoding the same can be one or more of or a Chi1, Chi2, RHS2, YenA1, YenA2, YenB, YenC1, YenC2 or a combination thereof. A YenTc subunit or gene encoding the same can also comprise multiple modifications. A modification of a YenTc subunit or gene encoding the same can preferably be an affinity tag or purification tag. A modification of a YenTc subunit or a gene encoding the same can be one or more of His-tag, FLAG tag, polycysteine tag and strep tag. A modification of a YenTc subunit or gene encoding the same can also comprise multiple copies of one or more of His-tag, FLAG tag, polycysteine tag and strep tag or any combinations thereof.

Accordingly, the present invention provides a *Yersinia entomophaga* bacterium of the invention, further comprising a modified gene encoding a YenTc subunit or a modified YenTc subunit.

In the sense of the present invention a tag is preferably a purification tag. A purification tag is an affinity tag that can be used to separate a protein of interest from other compounds. The invention is not particularly limited to any affinity tags for purification of e.g. a toxin. Any affinity tags that allow for high yield purification of a toxin, such as a YenTc toxin can be used. Exemplary affinity tags for purification that can be used in the sense of the present invention are one or more of His-tag, FLAG tag, polycysteine tag and strep tag.

Accordingly, the invention provides a *Yersinia entomophaga* bacterium of the invention, further comprising a modified gene encoding a YenTc subunit or a modified YenTc subunit, wherein the gene encoding a YenTc subunit comprises a sequence encoding a purification tag or wherein the modified YenTc subunit comprises a purification tag.

In general, a tag can be introduced at any position of a YenTc subunit or gene encoding the same, such as internal, N-terminal, and/or C-terminal or internal, 5' and/or 3' of the coding sequence. The position of a tag can be chosen based on preferred parameters such as affinity, accessibility, and functionality, i.e. toxicity of the YenTc toxin. A tag can be fused to a YenTc subunit or introduced into a gene encoding a YenTc subunit using a linker sequence, such as a peptide linker. The invention is not particularly limited to a linker sequence of any size, the skilled person is rather aware that any linker providing good affinity of the corresponding tag and which does not decrease the potency of the YenTc subunit can be used.

Accordingly, the present invention provides a *Yersinia entomophaga* bacterium of the invention, further comprising a modified gene encoding a YenTc subunit, wherein the gene encoding a YenTc subunit comprises a sequence encoding the purification tag 5' or 3' or within the coding region, in frame of the coding sequence, optionally comprising a linker sequence. The present invention also provides a *Yersinia entomophaga* bacterium of the invention, further comprising a modified YenTc subunit, wherein the modified YenTc subunit comprises an internal, N-terminal and/or C-terminal purification tag.

The present invention provides a *Yersinia entomophaga* bacterium of the invention, further comprising a modified gene encoding a YenTc subunit, wherein the gene encoding a YenTc subunit comprises a sequence encoding the purification tag 5' or 3' or within the coding region, in frame of the coding sequence, optionally comprising a linker sequence, wherein the purification tag is one or more of His-tag, FLAG tag, polycysteine tag and Strep-tag.

The present invention also provides a *Yersinia entomophaga* bacterium of the invention, further comprising a modified YenTc subunit, wherein the modified YenTc subunit comprises an internal, N-terminal and/or C-terminal purification tag, wherein the purification tag is one or more of His-tag, FLAG tag, polycysteine tag and Strep-tag.

As described above, any subunit of the YenTc toxin or gene encoding the same can be modified according to the present invention.

Accordingly, the invention provides a *Yersinia entomophaga* bacterium of the invention, further comprising one or more modified Chi1, Chi2, RHS2, YenA1, YenA2, YenB, YenC1, YenC2 or a combination thereof.

The invention also provides a *Yersinia entomophaga* bacterium of the invention, further comprising one or more modified Chi1, Chi2, RHS2, YenA1, YenA2, YenB, YenC1, YenC2 genes or a combination thereof.

In general, any of the components of the toxin composition of the present invention, namely Cbp, Chi1, Chi2, Chi3, NucA, Pil36, PirA, PirB, StcE, Tlh, YenA1, YenA2, YenB, YenC1, YenC2, RHS2, can be modified, e.g. with a purification tag, to allow for efficient production and/or purification.

Accordingly, the present invention provides a *Yersinia entomophaga* bacterium comprising one or more modified Cbp, Chi1, Chi2, Chi3, NucA, Pil36, PirA, PirB, StcE, Tlh, YenA1, YenA2, YenB, YenC1, YenC2, RHS2.

The present invention provides a *Yersinia entomophaga* bacterium comprising one or more modified Cbp, Chi1, Chi2, Chi3, NucA, Pil36, PirA, PirB, StcE, Tlh, YenA1, YenA2, YenB, YenC1, YenC2, RHS2, wherein the one or more modified Cbp, Chi1, Chi2, Chi3, NucA, Pil36, PirA, PirB, StcE, Tlh, YenA1, YenA2, YenB, YenC1, YenC2, RHS2 comprises a purification tag.

The present invention also provides a *Yersinia entomophaga* bacterium comprising one or more modified Cbp, Chi1, Chi2, Chi3, NucA, Pil36, PirA, PirB, StcE, Tlh, YenA1, YenA2, YenB, YenC1, YenC2, RHS2 comprising an internal, N-terminal and/or C-terminal purification tag. The present invention also provides a *Yersinia entomophaga* bacterium comprising one or more modified Cbp, Chi1, Chi2, Chi3, NucA, Pil36, PirA, PirB, StcE, Tlh, YenA1, YenA2, YenB, YenC1, YenC2, RHS2 comprising one or more of His-tag, FLAG tag, polycysteine tag and Strep-tag.

In another aspect the invention provides one or more Cbp, Chi1, Chi2, Chi3, NucA, Pil36, PirA, PirB, StcE, Tlh, YenA1, YenA2, YenB, YenC1, YenC2, RHS2 comprising a purification tag.

The *Yersinia entomophaga* bacterium comprising a modified gene encoding a YenTc subunit or a modified YenTc subunit of the present invention can be combined with any of the *Yersinia entomophaga* bacteria described herein. In particular, the *Yersinia entomophaga* bacterium comprising a modified YenR gene and further comprising a modified YenA2 or a modified YenA2 gene of the present invention can further comprise a modified YmoA gene, a functional YenDF, wherein YenDF comprises YeHln, YeEln, YeIspn and YeOspn, and a modified YenDF or any combinations thereof.

In some preferred embodiments the modified YenTc subunit or gene encoding the same is YenA2. YenA2 relates to the UniProt accession number B6A878 and is a component/subunit of the YenTc toxin. Modification of the YenA2 subunit or YenA2 gene is particularly beneficial in the present invention, as the YenA2 subunit can be modified with a purification or affinity tag to allow for high yield production and purification of a YenTc toxin.

Accordingly, the invention provides a *Yersinia entomophaga* bacterium of the present invention further comprising a modified YenA2 subunit comprising a C-terminal His-tag.

The features of a modified YenR gene, a modified YmoA gene or a YmoA plasmid, a functional YenDF, a modified YenDF and a modified YenTc subunit or gene encoding the same can be combined at will in the sense of the present invention to generate a modified *Yersinia entomophaga* bacterium. Different combinations of said features can be used to achieved different properties. For example, a modified YenDF can be used to avoid secretion of a toxin through lysis of the *Yersinia entomophaga* bacterium and a modified YenTc subunit can be used to recover a high purity YenTc. Accordingly, the skilled person understands that an appropriate combination of features is desired for different applications.

In another aspect, the invention provides a method to produce a purified YenTc toxin comprising a step of culturing a *Yersinia entomophaga* bacterium of the present invention. A method to produce a purified YenTc toxin is especially useful since the YenTc toxin is highly potent and specific to insects. Accordingly producing a high amount of pure YenTc is desirable. In general, the method to produce a purified YenTc toxin comprises a step of culturing a *Yersinia entomophaga* bacterium comprising a modified YenTc subunit or a modified YenTc of the present invention. The modification of a YenTc subunit, such as the addition of a purification or affinity tag, as described herein above allows for efficient purification of a YenTc toxin. The *Yersinia entomophaga* bacterium comprising a modified YenTc subunit or a modified YenTc of the present invention can be cultured using the means and parameters as described for a *Yersinia entomophaga* bacterium comprising a modified YenR herein above. Accordingly, the invention provides a method to produce a purified YenTc toxin comprising a step of culturing a *Yersinia entomophaga* bacterium comprising a modified gene encoding a YenTc subunit or a modified YenTc subunit

The invention provides a method to produce a purified YenTc toxin comprising a step of culturing a *Yersinia entomophaga* bacterium of the present invention, wherein the culturing step comprises culturing the *Yersinia entomophaga* bacterium at an initial pH of about 7.0, further comprising culturing the bacteria until a pH of 5.5-6.0 is reached.

The invention provides a method to produce a purified YenTc toxin comprising a step of culturing a *Yersinia entomophaga* bacterium of the present invention, wherein the culturing step comprises culturing the *Yersinia entomophaga* bacterium at less than about 30, 30.5, 31, 31.5, 32, 32.5, 33, 33.5, 34, 34.5, 35, 35.5, 36, 36.5, 37, 37.5, 38, 38.5, 39, 39.5, or 40°C.

The invention provides a method to produce a purified YenTc toxin comprising a step of culturing a *Yersinia entomophaga* bacterium of the present invention, wherein the culturing step comprises culturing the *Yersinia entomophaga* bacterium at less than about 30-40°C. Preferably the invention provides a method to produce a purified YenTc toxin comprising a step of culturing a *Yersinia entomophaga* bacterium of the present invention, wherein the culturing step comprises culturing the *Yersinia entomophaga* bacterium at less than about 30°C. The invention provides a method to produce a purified YenTc toxin comprising a step of culturing a *Yersinia entomophaga* bacterium of the present invention, wherein the culturing step comprises culturing the *Yersinia entomophaga* bacterium at about 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5 or 25°C, preferably at about 16, 16.5, 17, 17.5, 18, 18.5, 19, 19.5, or 20°C.

The invention provides a method to produce a purified YenTc toxin comprising a step of culturing a *Yersinia entomophaga* bacterium of the present invention, wherein the culturing step comprises culturing the *Yersinia entomophaga* bacterium at about 10-25°C, preferably at about 16-20°C.

The invention provides a method to produce a purified YenTc toxin comprising a step of culturing a *Yersinia entomophaga* bacterium of the present invention, wherein the culturing step comprises culturing the *Yersinia entomophaga* bacterium in the presence of markers mimicking the *Yersinia entomophaga* host, such as complex organic compounds.

The invention provides a method to produce a purified YenTc toxin comprising a step of culturing a *Yersinia entomophaga* bacterium of the present invention, further comprising a secretion step.

The invention provides a method to produce a purified YenTc toxin comprising a step of culturing a *Yersinia entomophaga* bacterium of the present invention, further comprising a secretion step, wherein the secretion step comprises
(i) increasing the pH of the initial culture from pH 5.5-6.0 to pH 6.3-10.0 and
(ii) incubating the *Yersinia entomophaga* bacterium at pH 6.3-10.0.

The invention provides a method to produce a purified YenTc toxin comprising a step of culturing a *Yersinia entomophaga* bacterium of the present invention, further comprising a secretion step, wherein the secretion step comprises
(i) increasing the pH of the initial culture from pH 5.5, 5.6, 5.7, 5.8, 5.9, or 6.0 to pH 6.3-10.0 and
(ii) incubating the *Yersinia entomophaga* bacterium at pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10.

The invention provides a method to produce a purified YenTc toxin comprising a step of culturing a *Yersinia entomophaga* bacterium of the present invention, further comprising a secretion step, wherein the secretion step comprises
(i) transferring the bacterium to a pH 6.3-10.0 buffer and
(ii) incubating the *Yersinia entomophaga* bacterium at pH 6.3-10.0.

The invention provides a method to produce a purified YenTc toxin comprising a step of culturing a *Yersinia entomophaga* bacterium of the present invention, further comprising a secretion step, wherein the secretion step comprises
(i) transferring the bacterium to a pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 buffer and
(ii) incubating the *Yersinia entomophaga* bacterium at pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10.

The invention provides a method to produce a purified YenTc toxin comprising a step of culturing a *Yersinia entomophaga* bacterium of the present invention, further comprising a secretion step, wherein the secretion step comprises
(i) increasing the pH of the initial culture from pH 5.5-6.0 to pH 6.9-8.9 and
(ii) incubating the *Yersinia entomophaga* bacterium at pH 6.9-8.9.

The invention provides a method to produce a purified YenTc toxin comprising a step of culturing a *Yersinia entomophaga* bacterium of the present invention, further comprising a secretion step, wherein the secretion step comprises
(i) increasing the pH of the initial culture from pH 5.5, 5.6, 5.7, 5.8, 5.9, or 6.0 to pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9 and
(ii) incubating the *Yersinia entomophaga* bacterium at pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9.

The invention provides a method to produce a purified YenTc toxin comprising a step of culturing a *Yersinia entomophaga* bacterium of the present invention, further comprising a secretion step, wherein the secretion step comprises
(i) transferring the bacterium to a pH 6.9-8.9 buffer and
(ii) incubating the *Yersinia entomophaga* bacterium at pH 6.9-8.9.

The invention provides a method to produce a purified YenTc toxin comprising a step of culturing a *Yersinia entomophaga* bacterium of the present invention, further comprising a secretion step, wherein the secretion step comprises
(i) transferring the bacterium to a pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9 buffer and
(ii) incubating the *Yersinia entomophaga* bacterium at pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9.

To purify a YenTc toxin a *Yersinia entomophaga* bacterium comprising a modified gene encoding a YenTc subunit or a modified YenTc subunit can be centrifuged and filtered to separate a cellular fraction from a YenTc comprising fraction. The means of centrifugation steps and filtration steps as described herein above regarding a *Yersinia entomophaga* bacterium comprising a modified YenR gene can also be applied in the method to produce a purified YenTc toxin comprising a step of culturing a *Yersinia entomophaga* bacterium comprising a modified gene encoding a YenTc subunit or a modified YenTc subunit.

In particular, a *Yersinia entomophaga* bacterium comprising a modified gene encoding a YenTc subunit or a modified YenTc subunit can further be purified by an affinity purification. The modified YenTc subunit or gene encoding the same can for example comprise one or more purification and/or affinity tags. Exemplary tags that can be used in the sense of the present invention are one or more of His-tag, FLAG tag, polycysteine tag and Strep-tag. Accordingly, a respectively tagged YenTc subunit can be purified by an affinity purification step wherein a tagged YenTc in purified from a culture or buffer. In general, the invention is not limited to any affinity purification, rather any affinity purification that results in a desired purity can be used in the method to produce a purified YenTc. Exemplary affinity purification that can be used in the sense of the present invention are immobilized metal ion affinity chromatography, antibody-based affinity chromatography, streptavidin-based affinity chromatography, thiopropyl-sepharose based affinity chromatography. One non-limiting Ni²⁺-NTA bead-based purification of a modified YenTc toxin is described in detail in the appended examples.

The affinity purification step can be combined with any of a culturing step, a secretion step, and/or a recovery step. These steps can be combined at will in the method to produce a purified YenTc toxin to achieve a desired yield of a desired purity.

Accordingly, the invention provides a method to produce a purified YenTc toxin comprising a step of culturing a *Yersinia entomophaga* bacterium of the present invention, further comprising a purification step.

The invention provides a method to produce a purified YenTc toxin comprising a step of culturing a *Yersinia entomophaga* bacterium of the present invention, further comprising a purification step, wherein the purification step comprises centrifuging the *Yersinia entomophaga* bacterium.

The invention provides a method to produce a purified YenTc toxin comprising a step of culturing a *Yersinia entomophaga* bacterium of the present invention, further comprising a purification step, wherein the purification step comprises filtrating the *Yersinia entomophaga* bacterium.

The invention provides a method to produce a purified YenTc toxin comprising a step of culturing a *Yersinia entomophaga* bacterium of the present invention, further comprising a purification step, wherein the purification step comprises purifying a YenTc toxin via an affinity purification.

The skilled person is aware that the steps of purification can also be combined depending on desired purity and on the impurities of the initial *Yersinia entomophaga* culture.

Accordingly, the invention provides a method to produce a purified YenTc toxin comprising a step of culturing a *Yersinia entomophaga* bacterium of the present invention, further comprising a purification step, wherein the purification step comprises
(i) centrifuging the *Yersinia entomophaga* bacterium, and/or optionally
(ii) filtrating the *Yersinia entomophaga* bacterium, and/or optionally
(iii) purifying a YenTc toxin via an affinity purification.

The invention provides a method to produce a purified YenTc toxin comprising a step of culturing a *Yersinia entomophaga* bacterium of the present invention, further comprising a purification step, wherein the purification step comprises
(i) centrifuging the *Yersinia entomophaga* bacterium, and
(ii) filtrating the *Yersinia entomophaga* bacterium, and
(iii) purifying a YenTc toxin via an affinity purification.

The invention also provides a method to produce a purified YenTc toxin comprising a step of culturing a *Yersinia entomophaga* bacterium of the present invention, further comprising a purification step, wherein the purification step comprises a bead-based purification and wherein the beads show high affinity to the modified YenTc subunit.

The invention also provides a method to produce a purified YenTc toxin comprising a step of culturing a *Yersinia entomophaga* bacterium of the present invention, further comprising a purification step, wherein the purification step comprises a bead-based purification and wherein the beads show high affinity to the amino acid sequence set forth in SEQ ID NO: 9.

The method to produce to produce a purified YenTc toxin of the present invention can further comprise a recovery step. A recovery step is used to harvest the compounds of the toxin composition. A recovery step can be used in accordance with the means and parameters of the recovery step of the method to produce a toxin composition of the present invention as described herein above. However, in the sense of a method to produce to produce a purified YenTc toxin of the present invention it is preferred that only a YenTc toxin is recovered. Thus, it is preferred that toxin other than YenTc that may be present in a culture or buffer of the present invention are not recovered in the recovery step of a method to produce to produce a purified YenTc toxin of the present invention.

Accordingly, the invention provides a method to produce a purified YenTc toxin comprising a step of culturing a *Yersinia entomophaga* bacterium of the present invention, further comprising a recovery step.

The invention provides a method to produce a purified YenTc toxin comprising a step of culturing a *Yersinia entomophaga* bacterium of the present invention, further comprising a recovery step wherein the recovery step comprises recovering a purified YenTc toxin from a culture of the *Yersinia entomophaga* bacterium.

The invention also provides a method to produce a purified YenTc toxin comprising a step of culturing a *Yersinia entomophaga* bacterium of the present invention, further comprising a recovery step wherein the recovery step comprises recovering a purified YenTc toxin from a buffer comprising the *Yersinia entomophaga* bacterium.

The invention provides a method to produce a purified YenTc toxin comprising a step of culturing a *Yersinia entomophaga* bacterium of the present invention, further comprising a recovery step wherein the recovery step comprises
(i) recovering a purified YenTc toxin from a culture of the *Yersinia entomophaga* bacterium and/or optionally
(ii) concentrating the YenTc toxin.

The invention provides a method to produce a purified YenTc toxin comprising a step of culturing a *Yersinia entomophaga* bacterium of the present invention, further comprising a recovery step wherein the recovery step comprises
(i) recovering a purified YenTc toxin from a buffer comprising the *Yersinia entomophaga* bacterium and/or optionally
(ii) concentrating the YenTc toxin.

In another aspect the present invention provides a purified YenTc toxin obtained by or as obtainable by the method to produce a purified YenTc toxin.

In another aspect the present invention provides a YenTc toxin or a subunit of a YenTc toxin comprising a purification tag. As described herein a YenTc toxin comprising a purification or an affinity tag is especially useful to obtain a high amount of YenTc with a high purity. The purification or affinity tag can be used in a step of affinity purification which enables to recover a tremendous amount of YenTc. The purification or affinity purification tag of the invention is preferably chosen to not interfere with the structure or function, i.e. toxicity. of the YenTc. However, the purification or affinity purification tag of the invention can also be cut off after producing a purified YenTc. This has the advantage that a YenTc can be obtained with a high yield and high purity that does not comprise any artificial sequence. This can be realized by adding a cleavable linker sequence between the subunit of interest and the purification tag. This linker can then be cleaved by treating the tagged protein/subunit with an enzyme cleaving said linker. Exemplary enzymes that can be used are TEV protease, 3C protease or thrombin. As described herein above a purification or affinity tag can be attached anywhere in the YenTc or YenTc subunit. In general, it is preferred that the location of the tag does not interfere with structure, function or production of the YenTc. Thus, a purification or affinity tag of the present invention can be internal, N-termina and/or C-terminal. A purification or affinity tag is preferably in frame with the coding region of a YenTc subunit. A purification or affinity tag in the sense of the present invention can be one or more of a His-tag, FLAG tag, polycysteine tag and Strep-tag or any combinations thereof. One or more YenTc subunits can also comprise multiple of the same tags or multiple different tags. Accordingly, a YenTc can also comprise multiple of the same tags or multiple different tags. It is preferred that a YenTc subunit or a YenTc comprises one or more His-tags. In particular it is preferred that the YenTc subunit YenA2 comprises one or more His-tag, preferably at the C-terminus of the YenA2. Accordingly, the invention provides a YenTc toxin or a subunit of a YenTc toxin comprising a purification tag.

The invention provides a YenTc toxin or a subunit of a YenTc toxin comprising an internal, N-terminal and/or C-terminal purification tag.

The invention also provides a YenTc toxin or a subunit of a YenTc toxin, comprising one or more of His-tag, FLAG tag, polycysteine tag and Strep-tag.

Preferably, the invention provides a YenTc toxin comprising a YenA2 subunit with a C-terminal His-tag.

Preferably, the invention provides a YenA2 subunit with a C-terminal His-tag.

In another aspect the invention provides a method to induce secretion of a toxin or toxin composition. As described herein above, secretion of a toxin from a *Yersinia entomophaga* bacterium can be induced by increasing the pH of the culture or buffer comprising the bacteria. Often secretion of toxin or toxin composition may not be desired or required right away but at a later stage of production. Thus, it is especially useful to provide means for precise control for the secretion of a toxin or toxin composition.

Accordingly, the invention provides a method to induce secretion of a toxin or toxin composition comprising
(i) culturing a *Yersinia entomophaga* bacterium of the present invention, at an initial pH of about 7.0 until a desired number of bacteria is grown; and
(ii) incubating the bacteria of (i) at pH 6.3-10.0, preferably pH 6.9-8.9, thereby inducing the secretion of the toxin.

The invention provides a method to induce secretion of a toxin or toxin composition comprising
(i) culturing a *Yersinia entomophaga* bacterium of the present invention, at an initial pH of about 7.0 until a desired number of bacteria is grown; and
(ii) transferring the bacteria of (i) to a pH 6.3-10.0 buffer, preferably pH 6.9-8.9, thereby inducing the secretion of the toxin.

The invention also provides a method to induce secretion of a toxin or toxin composition comprising
(i) culturing a *Yersinia entomophaga* bacterium of the present invention, at an initial pH of about 7.0 until a desired number of bacteria is grown; and
(ii) incubating the bacteria of (i) at pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10, preferably pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9, thereby inducing the secretion of the toxin.

In other words, the invention provides a method to induce secretion of a toxin or toxin composition comprising
(i) culturing a *Yersinia entomophaga* bacterium of the present invention, at an initial pH of about 7.0 until a desired number of bacteria is grown; and
(ii) transferring the bacteria of (i) to a pH 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, or 10 buffer, preferably pH 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, or 8.9, thereby inducing the secretion of the toxin.

In another aspect, the invention provides a (isolated) population of soldier cells. In the sense of the present invention "soldier cells" are *Yersinia entomophaga* cells where the production and/or secretion of a toxin or toxin composition is induced. These cells naturally occur as a subset of pathogenic cells with a distinct phenotype in a population of *Yersinia entomophaga.* Soldier cells may comprise wild type *Yersinia entomophaga* cells, such as cells of the *Yersinia entomophaga* strain MH96, wherein expression of YenR is increased. A soldier cell is a *Yersinia entomophaga* cell comprising/having increased YenR expression and/or comprising a toxin composition or toxin as described herein. Accordingly, the invention provides a population of soldier cells comprising/having increased YenR expression and/or comprising a toxin or toxin composition. The term "population of soldier cells" refers to a population comprising or consisting (essentially) of soldier cells. Accordingly, the invention provides a a population of soldier cells comprising (or consisting (essentially) of) a *Yersinia entomophaga* bacterium comprising/having increased YenR expression and/or comprising a toxin or toxin composition. In other words, the invention provides a population comprising or consisting (essentially) of soldier cells, wherein the soldier cells are *Yersinia entomophaga* bacteria, specifically wherein the expression of the YenR gene or gene products thereof is increased (in the soldier cell(s)), optionally, wherein the expression of the YmoA gene or gene products thereof is increased (in the soldier cell(s)) and/or wherein the soldier cells comprise the toxin composition and/or one or more of the toxin(s) as provided herein, preferably, wherein the population comprises the *Yersinia entomophaga* bacterium comprising the modified YenR gene as provided herein, optionally wherein the expression of the YenR gene or gene products thereof is directly or indirectly increased (in the soldier cell(s)), further optionally, wherein the expression of the YmoA gene or gene products thereof is directly or indirectly increased (in the soldier cell(s)).

In this context, it is particularly preferred that the soldier cell or a population thereof is a wild type *Yersinia entomophaga* as described herein, e.g. *Yersinia entomophaga* strain MH96. The soldier cell or population thereof comprises/has an increased YenR expression compared to an appropriate control, e.g. a wild type *Yersinia entomophaga* (or population thereof) which is/are not a soldier cell. As described herein, "soldier cells" can be readily distinguished from wild type *Yersinia entomophaga* (or population thereof) which is/are not a soldier cell e.g. by morphological analysis.

It is envisaged herein that the population of soldier cells does not comprise (detectable amounts of) other cells, specifically not wild type *Yersinia entomophaga* bacteria which are not soldier cells. In this sense, the above population is a population of soldier cells consisting of *Yersinia entomophaga* bacteria comprising/having increased YenR expression and/or comprising a toxin or toxin composition.

Alternatively, the population of soldier cells can comprise only minimal amounts of other cells, specifically wild type *Yersinia entomophaga* bacteria which are not soldier cells. For example, the percentage of such other cells (specifically wild type *Yersinia entomophaga* bacteria which are not soldier cells) is below 20 %, 10 %, 5 %, 4 %, 3 %, 2 % or even below 1% of the total cell population of soldier cells. In this sense, the above population (below 20 %) is a population of soldier cells consisting essentially of *Yersinia entomophaga* bacteria comprising/having increased YenR expression and/or comprising a toxin or toxin composition.

In accordance with the above, a population comprising (or consisting (essentially) of) soldier cells may be obtained/isolated from a population of wild type *Yersinia entomophaga* by any suitable means, such as manual sorting of soldier cells with a distinct phenotype, FACS and the like. Soldier cells are heavier than their counterparts (non-soldier cells of *Yersinia entomophaga*) and thus allow for separation by low-speed centrifugation. For example, a population comprising (or consisting (essentially) of) soldier cells may be obtained/isolated by low-speed centrifugation, such as centrifugation at about 50, 100, 150, 200, 250, 300, 350, 400, 450, 500, 550, 600, 650, 700, 750, 800, 850, 900, 950, or 1000 g. In particular, soldier cells are bigger than their counterparts and thus allow for separation by filtration. For example, a population comprising (or consisting (essentially) of) soldier cells may be obtained/isolated by filtration with a filter of about 0.45 µm pore size. The soldier cells will not be able to pass the filter and can be efficiently separated from their non-soldier cell counterparts.

The present invention provides the means to produce a population of *Yersinia entomophaga* wherein all cells are pathogenic toxin producers, i.e. wherein all cells are soldier cells. This can be realized e.g. by modifying the YenR gene. In general, the term "soldier cell" refers to a *Yersinia entomophaga* cell that produces or is capable of producing the toxin composition or toxin(s) as defined and provided herein. A population of soldier cells is especially useful since all cells of the population produce a toxin or toxin composition. The population of soldier cells can be used in the methods to produce a toxin or toxin composition as described herein. A population of soldier cells can comprise any of the modifications described herein. However, it is preferred that a population of soldier cells comprises at least modified YenR gene. Thus, a population of solider cells can comprise a modified YenR gene and can further comprise one or more of a modified YmoA gene or YmoA plasmid, a functional YenDF, a modified YenDF, a modified YenTc subunit or gene encoding the same and a modified YenA2 or gene encoding the same or any combination thereof. A population of soldier cells preferably comprises a *Yersinia entomophaga* bacterium of the present invention.

Accordingly, the invention provides a population comprising soldier cells, wherein the soldier cells are *Yersinia entomophaga* bacteria, wherein the expression of the YenR gene or gene products thereof is increased.

The invention provides a population consisting essentially of soldier cells, wherein the soldier cells are *Yersinia entomophaga* bacteria, wherein the expression of the YenR gene or gene products thereof is increased.

The invention also provides a population consisting of soldier cells, wherein the soldier cells are *Yersinia entomophaga* bacteria, wherein the expression of the YenR gene or gene products thereof is increased.

The above defined population of soldier cells can further comprise increased expression of the YmoA gene or gene products thereof.

In another aspect, the invention provides a population comprising of soldier cells, wherein the soldier cells comprise the toxin composition and/or one or more of the toxin(s) of the present invention.

The invention provides a population consisting essentially of soldier cells, wherein the soldier cells comprise the toxin composition and/or one or more of the toxin(s) of the present invention. The invention provides a population consisting of soldier cells, wherein the soldier cells comprise the toxin composition and/or one or more of the toxin(s) of the present invention. The features of a population of soldier cells may also be combined. Accordingly, the invention provides a population comprising or consisting (essentially) of soldier cells, wherein the soldier cells are *Yersinia entomophaga* bacteria, wherein the expression of the YenR gene or gene products thereof is increased, optionally, wherein the expression of the YmoA gene or gene products thereof is increased and/or wherein the soldier cells comprise the toxin composition and/or one or more of the toxin(s) according to the present invention, preferably, wherein the population comprises the *Yersinia entomophaga* bacterium of the present invention, optionally wherein the expression of the YenR gene or gene products thereof is directly or indirectly increased, further optionally, wherein the expression of the YmoA gene or gene products thereof is directly or indirectly increased.

The term "increased expression of the YenR gene or gene products thereof' or the like as used herein refers, in particular, to an expression level (or an amount of the gene product) sufficient to induce or allow production of the toxin composition or toxin as defined and provided herein. The same explanation applies, mutatis mutandis, to the term "increased expression of the YmoA gene or gene products thereof'.

The invention provides a population of soldier cells comprising *Yersinia entomophaga* bacteria, wherein the expression of the YenR gene or gene products thereof is directly or indirectly increased.

The invention provides a population of soldier cells comprising *Yersinia entomophaga* bacteria, wherein the expression of the YenR gene or gene products thereof is directly or indirectly increased, wherein the expression of the YmoA gene or gene products thereof is directly or indirectly increased.

The invention provides a population of soldier cells comprising *Yersinia entomophaga* bacteria, wherein the expression of the YenR gene or gene products thereof is directly or indirectly increased, further comprising a YmoA plasmid.

The invention provides a population of soldier cells comprising *Yersinia entomophaga* bacteria, wherein the expression of the YenR gene or gene products thereof is directly or indirectly increased, further comprising a YmoA plasmid and a functional YenDF.

The invention provides a population of soldier cells comprising *Yersinia entomophaga* bacteria, wherein the expression of the YenR gene or gene products thereof is directly or indirectly increased, further comprising a YmoA plasmid and a modified YenDF.

The invention provides a population of soldier cells comprising *Yersinia entomophaga* bacteria, wherein the expression of the YenR gene or gene products thereof is directly or indirectly increased, further comprising a YmoA plasmid and a modified YenTc subunit.

The invention provides a population of soldier cells comprising *Yersinia entomophaga* bacteria, wherein the expression of the YenR gene or gene products thereof is directly or indirectly increased, further comprising a YmoA plasmid and a modified YenDF and a modified YenTc subunit.

The invention provides a population of soldier cells comprising *Yersinia entomophaga* bacteria, wherein the expression of the YenR gene or gene products thereof is directly or indirectly increased, further comprising a YmoA plasmid and a modified YenDF and a modified YenA2 subunit.

The invention also provides a population of soldier cells comprising a *Yersinia entomophaga* bacterium of the present invention.

The invention also provides a method to produce the soldier cells described herein. As described above, a soldier cell can be produced by increasing the expression of a YenR gene in a *Yersinia entomophaga* bacterium and culturing said *Yersinia entomophaga* bacterium.

Accordingly, the present invention provides a method to produce a soldier cell comprising,
(i) increasing the expression of the YenR gene or gene products thereof in a *Yersinia entomophaga* bacterium, optionally increasing the expression of the YmoA gene or gene products thereof;
(ii) culturing the *Yersinia entomophaga* bacterium of (i) at an initial pH of about 7.0 until a desired number of bacteria is grown.

The present invention also provides a method to produce a soldier cell comprising,
(i) increasing the expression of the YenR gene or gene products thereof in a *Yersinia entomophaga* bacterium, and increasing the expression of the YmoA gene or gene products thereof;
(ii) culturing the *Yersinia entomophaga* bacterium of (i) at an initial pH of about 7.0 until a desired number of bacteria is grown.

The method to produce a soldier cell can further comprise in step (i) increasing or decreasing YenDF expression. As described herein above, the soldier cells can be stored in a storage buffer at pH 4.5-6.0, such as 4.5, 4.6, 4.7, 4.8, 4.9, 5, 5.1, 5.2, 5.3, 5.4, 5.5, 5.6, 5.7, 5.8, 5.9, or 6, preferably pH 5.5.

In another aspect the invention provides methods to kill pests. As described herein a toxin composition or YenTc toxin of the present invention is lethal to pests such as insects. Thus, the *Yersinia entomophaga* bacteria and the toxin composition and YenTc toxin of the present invention is especially useful in a method to kill pests such as insects.

Accordingly, the invention provides a method to kill pests comprising contacting a pest with a *Yersinia entomophaga* bacterium of the present invention or a population of soldier cells of the present invention.

The invention also provides a method to kill pests comprising contacting a pest with a toxin composition of any one of the present invention or a YenTc toxin of the present invention.

In the sense of the present invention, it is preferred that a pest belongs to the class of Insecta, such as an insect.

Accordingly, the invention provides a method to kill pests comprising contacting a pest with a *Yersinia entomophaga* bacterium of the present invention or a population of soldier cells of the present invention, wherein the pest is an animal belonging to the class of Insecta, such as an insect.

The invention also provides a method to kill pests comprising contacting a pest with a toxin composition of any one of the present invention or a YenTc toxin of the present invention, wherein the pest is an animal belonging to the class of Insecta, such as an insect.

In general, the bacteria or toxins of the present invention can be directly applied to insects, i.e. used to contact insects directly. An insect can be contacted with the bacteria, toxin composition and YenTc toxin of the present invention on a plant. In general, a plant can be any plant, such as a crop. A plant can be a plant in an agricultural area, garden area or park area.

In the sense of the present invention a plant can be a plantain. A plant can be a plantain crop. A plant can be a plant of the genus *Musa.* A plant can also be ryegrass, clover, or chicory. In the sense of the presentation a plant can also be in fact a mix of different species e.g. being grown in the same area. Thus, a plant can also be a mix of plantain, ryegrass, clover and chicory or the like.

The bacteria or toxin composition or YenTc toxin of the present invention can also be applied to plants, such as plants infested with a pest. Accordingly, a bacterium, toxin composition or YenTc toxin of the intention can be applied to a plant infested with an insect. A bacterium, toxin composition or YenTc toxin of the intention can directly be applied to plants that are acutely infested with an insect. The toxin composition can also be applied to plants that are at risk of being infested with an insect. The toxin composition can also be applied to plants to prevent a risk of being invested with an insect, i.e. prophylactic application. The toxin composition can be applied to a plant once or more than once, such as twice or multiple times. In preferred embodiments the toxin composition can be applied multiple times until an insect infestation is abolished or a risk of an infestation is abolished. The toxin composition can be applied repeatedly until a desired effect is achieved.

Accordingly, the invention provides a method to kill pests comprising contacting a pest with a *Yersinia entomophaga* bacterium of the present invention or a population of soldier cells of the present invention, wherein the pest is contacted on or near a plant or in an area where plants are grown.

The invention also provides a method to kill pests comprising contacting a pest with a toxin composition of any one of the present invention or a YenTc toxin of the present invention, wherein the pest is contacted on or near a plant or in an area where plants are grown. Accordingly, the invention provides a method to kill pests comprising contacting a pest with a *Yersinia entomophaga* bacterium of the present invention or a population of soldier cells of the present invention, wherein the pest is contacted on a plant in an agricultural area or a garden area or park area.

The invention also provides a method to kill pests comprising contacting a pest with a toxin composition of any one of the present invention or a YenTc toxin of the present invention, wherein the pest is contacted on a plant in an agricultural area or a garden area or park area. Accordingly, the invention provides a method to kill pests comprising contacting a pest with a *Yersinia entomophaga* bacterium of the present invention or a population of soldier cells of the present invention, wherein the bacterium or population of soldier cells is administered to plants infested with a pest, such as an insect pest.

The invention also provides a method to kill pests comprising contacting a pest with a toxin composition of any one of the present invention or a YenTc toxin of the present invention, wherein the toxin composition or YenTc toxin is administered to plants infested with a pest, such as an insect pest.

Accordingly, the invention provides a method to kill pests comprising contacting a pest with a *Yersinia entomophaga* bacterium of the present invention or a population of soldier cells of the present invention, wherein the bacterium or population of soldier cells is repeatedly administered to plants infested with a pest, such as an insect pest.

The invention also provides a method to kill pests comprising contacting a pest with a toxin composition of any one of the present invention or a YenTc toxin of the present invention, wherein the toxin composition or YenTc toxin is repeatedly administered to plants infested with a pest, such as an insect pest.

In another aspect, the present invention provides uses of the *Yersinia entomophaga* bacteria or the toxin composition or YenTc toxin of the invention as a pesticide or insecticide.

In another aspect, the present invention provides uses of the *Yersinia entomophaga* bacteria or the toxin composition or YenTc toxin of the invention as a pesticide composition or insecticide composition.

The *Yersinia entomophaga* bacteria or the population of soldier cells of the invention can also be used to produce a toxin composition, a YenTc toxin, a pesticide or an insecticide.

The *Yersinia entomophaga* bacteria or the population of soldier cells of the invention can also be used to produce a toxin composition, a YenTc toxin, a pesticide composition or an insecticide composition.

Accordingly, the invention provides a use of a *Yersinia entomophaga* bacterium of the invention or the population of soldier cells of the invention as a pesticide.

The invention also provides a use of a *Yersinia entomophaga* bacterium of the invention or the population of soldier cells of the invention as a pesticide composition.

The invention provides a use of a toxin composition of the invention, or a YenTc toxin of the invention as an insecticide.

The invention provides a use of a toxin composition of the invention, or a YenTc toxin of the invention as an insecticide composition.

The invention also provides a use of a *Yersinia entomophaga* bacterium of the invention or the population of soldier cells of the invention to produce a toxin composition, such as a pesticide or insecticide.

The invention also provides a use of a *Yersinia entomophaga* bacterium of the invention or the population of soldier cells of the invention to produce a toxin composition, such as a pesticide composition or insecticide composition.

The invention also provides a use of a *Yersinia entomophaga* of the invention or the population of soldier cells of the invention to produce a YenTc toxin.

The invention further provides a population of cells comprising the *Yersinia entomophaga* bacterium of the invention.

The invention further provides a modified YenR gene comprising the sequence set forth in SEQ ID NO: 2.

The invention also provides a modified YenR gene. The invention provides a modified YenR gene, wherein the expression of the YenR gene or gene products thereof is transiently or permanently modulated. The invention provides a modified YenR gene, wherein the expression of the YenR gene or gene products thereof is directly or indirectly increased. The invention provides a modified YenR gene, wherein the genomic sequence of the YenR gene is modified. The invention provides a modified YenR gene, wherein the promoter region of the YenR gene is modified. The invention provides a modified YenR gene, wherein the YenR gene and/or the promoter region of the YenR gene comprises a synthetic or artificial sequence. The invention provides a modified YenR gene, wherein the promoter region of the YenR gene comprises an inducible promoter or a high expressing promoter or regulatory elements. The invention provides a modified YenR gene, wherein the YenR gene comprises an inducible promoter or inducible regulatory element. The invention provides a modified YenR gene, wherein the YenR gene comprises an arabinose-inducible promoter or regulatory element, a lactose/IPTG-inducible promoter or regulatory element, an anhydrotetracycline-inducible promoter or regulatory element, or a rhamnose-inducible promoter or regulatory element, preferably an arabinose-inducible promoter or regulatory element.

The invention further provides a plasmid comprising the sequence set forth in SEQ ID NO: 3.

The invention also provides a *Yersinia entomophaga* bacterium comprising a modified YenR gene, wherein the expression of the YenR gene or gene products thereof is increased.

The invention also provides a *Yersinia entomophaga* bacterium comprising a modified YenR gene, wherein the expression of the YenR gene or gene products thereof is increased compared to a *Yersinia entomophaga* bacterium comprising an unmodified YenR gene, such as a wild type YenR gene.

The invention also provides a *Yersinia entomophaga* bacterium comprising an increased YenR expression.

The invention also provides a *Yersinia entomophaga* bacterium, wherein the expression of the YenR gene or gene products thereof is increased.

The invention also provides a *Yersinia entomophaga* bacterium comprising a modified YenR gene, wherein the expression of the YenR gene or gene products thereof is increased in an inducible manner.

The invention also provides a *Yersinia entomophaga* bacterium comprising a modified YenR gene, wherein the expression of the YenR gene or gene products thereof can be conditionally increased by an inducible system.

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a difference over what is generally understood in the art. The techniques and procedures described or referenced herein are generally well understood and commonly employed using conventional methodologies by those skilled in the art. As appropriate, procedures involving the use of commercially available kits and reagents are generally carried out in accordance with manufacturer-defined protocols and conditions unless otherwise noted.

The disclosures in context of the methods described herein are disclosed as corresponding use *mutatis mutandis.* The disclosures in context of the use described herein are disclosed as corresponding methods *mutatis mutandis.*

As used herein, the singular forms "a," "an," and "the" include the plural referents unless the context clearly indicates otherwise. The terms "include," "such as," and the like are intended to convey inclusion without limitation, unless otherwise specifically indicated.

As used herein, the term "or" is generally employed in its usual sense including "and/or" unless the content clearly dictates otherwise. The term "and/or" means one or all of the listed elements or a combination of any two or more of the listed elements.

As used herein, the term "comprising" also specifically includes embodiments "consisting of' and "consisting essentially of' the recited elements, unless specifically indicated otherwise.

As used herein, the term "about" indicates and encompasses an indicated value and a range above and below that value. In certain embodiments, the term "about" indicates the designated value ± 10%, ± 5%, or ± 1%. In certain embodiments, where applicable, the term "about" indicates the designated value(s) ± one standard deviation of that value(s).

The terms "YenDF" and "T10SS" as used herein mean *Yersinia entomophaga* death factor and type 10 secretion system, respectively. The terms are used interchangeably herein. The components of YenDF are described herein above. When it is referred herein to the "YenDF operon" this term may also include the YenR gene.

As used herein "pesticide" can refer to the active agent(s) having pesticidal activity, such as the *Yersinia entomophaga* bacteria, population of soldier cells, toxin compositions and/or YenTc toxin and the like, as provided herein. A pesticide is preferably a substance having the ability to deter, retard the growth, incapacitate, kill, or otherwise discourage pests. A pesticide is preferably a biopesticide. The term "pesticide" as used herein can also refer to "pesticide composition". These explanations apply mutatis mutandis to "insecticide" and "insecticide composition"

As used herein the term "biopesticide" refers to a biologically derived substance having the ability to deter, retard the growth, incapacitate, kill, or otherwise discourage pests. In particular, a biopesticide of the present invention is capable of deter, retard the growth, incapacitate, kill, or otherwise discourage insects and/or the larvae thereof.

As used herein the term "insecticide" is a pesticide that has the ability to deter, retard the growth, incapacitate, kill, or otherwise discourage insects and/or the larvae thereof. Thus, an insecticide is a pesticide specific for administration to insects. The term "insecticide" as used herein can also refer to "insecticide composition".

As used herein "culture" refers to a population of bacteria comprised in a growth medium. A culture preferably comprises a *Yersinia entomophaga* bacterium comprising a modified YenR gene in a growth medium.

As used herein "buffer" refers to a composition used to process or store a composition or a bacterium of the invention. In the sense of the present invention a buffer can be used to process or store the *Yersinia entomophaga* bacterium of the invention instead of a culture.

As used herein "insecticidal" refers to the ability to kill or harm insects and/or larvae thereof.

As used herein "toxin" refers to a substance/compound or substances/compounds derived from a *Yersinia entomophaga* bacterium comprising a modified YenR gene of the invention. A toxin may also refer to a mixture of toxins embodying different activities. A toxin may also refer to a YenTc toxin. In the sense of the invention a toxin is a substance that is toxic to insects. Thus, a toxin can be used to deter, retard the growth, incapacitate, kill, or otherwise discourage insects and/or the larvae thereof. A toxin can be comprised in an insecticide or a pesticide.

As used herein "increased expression" means that the expression of a gene or gene products thereof is increased compared to a control or a wild type. For example, increased expression of a modified YenR gene relates to an increase compared to an unmodified YenR gene. This can be the case when expression of the YenR gene or gene products thereof of a *Yersinia entomophaga* bacterium comprising a modified YenR gene of the invention is compared to a *Yersinia entomophaga* bacterium comprising an unmodified YenR gene, such as a wild type YenR gene. Such a *Yersinia entomophaga* bacterium comprising an unmodified YenR gene or a wild type YenR gene can be the *Yersinia entomophaga* strain MH96 (available via the DSMZ-German Collection of Microorganisms and Cell Cultures GmbH; DSM No.: 22339 or from ATCC under accession number ATCC BAA-1678). In general, any increase in expression is detected over a multitude of cells, such as a population of bacteria. Accordingly, an increase in expression can be detected on a cell population level. For example, in the sense of the invention the expression of the YenR gene can be compared between a wild type population, such as a population of *Yersinia entomophaga* cells of the strain MH96 and a population of *Yersinia entomophaga* cells comprising a modified YenR gene as described herein.

As used herein "increased expression of a gene" means that the transcription of the gene is increased compared to a control or a wild type gene such as a gene in the *Yersinia entomophaga* strain MH96. In the sense of the present invention the expression of a gene is preferably increased by an inducible system.

As used herein "increased expression of a gene product" means that the transcription of an RNA encoded by a gene, such as an mRNA, or the translation of a RNA, such as a mRNA is increased, which results in an increased level of said gene products. Thus, "increased expression of a gene product" can mean an increased level of the gene product, such as an increased level of mRNA or protein.

As used herein "gene products" relate to any nucleic acid or protein encoded by a gene. Exemplary gene products can be RNAs and polypeptides, such as mRNA, or proteins.

SEQ ID NO: 1 encodes the genome of a *Yersinia entomophaga* bacterium comprising a modified YenR gene comprising an arabinose inducible promoter.

SEQ ID NO: 2 encodes a modified YenR gene comprising an arabinose inducible promoter. SEQ ID NO: 3 encodes a plasmid comprising a YmoA gene under the control of an anhydrotetracycline-inducible promoter.

SEQ ID NO: 4 encodes an unmodified YeHln polypeptide as found for example in the *Yersinia entomophaga* strain MH96.

SEQ ID NO: 5 encodes an unmodified YeEln polypeptide as found for example in the *Yersinia entomophaga* strain MH96.

SEQ ID NO: 6 encodes an unmodified YeIspn polypeptide as found for example in the *Yersinia entomophaga* strain MH96.

SEQ ID NO: 7 encodes an unmodified YeOspn polypeptide as found for example in the *Yersinia entomophaga* strain MH96.

SEQ ID NO: 8 encodes a modified YenDF operon comprising a YenR gene with an arabinose inducible promoter and a non-functional YenDF.

SEQ ID NO: 9 encodes a modified YenA2 polypeptide. In particular, SEQ ID NO: 9 encodes a YenA2 polypeptide comprising a C-terminal His-tag.

SEQ ID NO: 10 encodes an unmodified YenR polypeptide as found for example in the *Yersinia entomophaga* strain MH96.

SEQ ID NO: 11 encodes an editing plasmid comprising λ-RED recombineering proteins, an IPTG-inducible I-SceI endonuclease, a rhamnose-inducible Cas9 protein and a constitutively expressed gRNA.

SEQ ID NO: 12 encodes a YenA2 comprising a C-terminal His-tag donor plasmid with an excision option.

SEQ ID NO: 13 encodes a YenA2 comprising a C-terminal His-tag donor plasmid without an excision option.

SEQ ID NO: 14 encodes an arabinose inducible YenR donor plasmid without an excision option.

SEQ ID NO: 15 encodes an arabinose inducible YenR and non-functional YenDF donor plasmid without an excision option.

SEQ ID NO: 16 encodes the genome of a *Yersinia entomophaga* bacterium comprising a modified YenR gene comprising an arabinose inducible promoter, further comprising a non-functional YenDF, further comprising a YenA2 comprising a C-terminal His-tag.

SEQ ID NO: 17 encodes the genome of a *Yersinia entomophaga* bacterium comprising a modified YenR gene comprising an arabinose inducible promoter, further comprising a functional YenDF, further comprising a YenA2 comprising a C-terminal His-tag.

SEQ ID NO: 18 encodes an unmodified YeHln gene as found for example in the *Yersinia entomophaga* strain MH96.

SEQ ID NO: 19 encodes an unmodified YeEln gene as found for example in the *Yersinia entomophaga* strain MH96.

SEQ ID NO: 20 encodes an unmodified YeIspn gene as found for example in the *Yersinia entomophaga* strain MH96.

SEQ ID NO: 21 encodes an unmodified YeOspn gene as found for example in the *Yersinia entomophaga* strain MH96.

SEQ ID NO: 22 encodes an unmodified YenR gene as found for example in the *Yersinia entomophaga* strain MH96.

SEQ ID NO: 23 encodes an unmodified YenR mRNA as found for example in the *Yersinia entomophaga* strain MH96.

SEQ ID NO: 24 encodes an unmodified YmoA gene as found for example in the *Yersinia entomophaga* strain MH96.

SEQ ID NO: 25 encodes an unmodified YmoA mRNA as found for example in the *Yersinia entomophaga* strain MH96.

SEQ ID NO: 26 encodes an unmodified YmoA polypeptide as found for example in the *Yersinia entomophaga* strain MH96.

SEQ ID NO: 27 encodes a modified YenA2 gene comprising a C-terminal His-tag.

SEQ ID NO: 28 encodes an arabinose-inducible promoter.

SEQ ID NO: 29 encodes an arabinose-inducible promoter with ribosome binding site.

SEQ ID NO: 30 encodes an arabinose-inducible promoter with ribosome binding site and L-arabinose regulatory protein.

SEQ ID NO: 31 encodes an unmodified YenDF locus (with YenR) as found for example in the *Yersinia entomophaga* strain MH96.

SEQ ID NO: 32 encodes an unmodified YenDF locus (only structural components) as found for example in the *Yersinia entomophaga* strain MH96.

The invention is illustrated in the following examples.

### Brief description of the drawings:

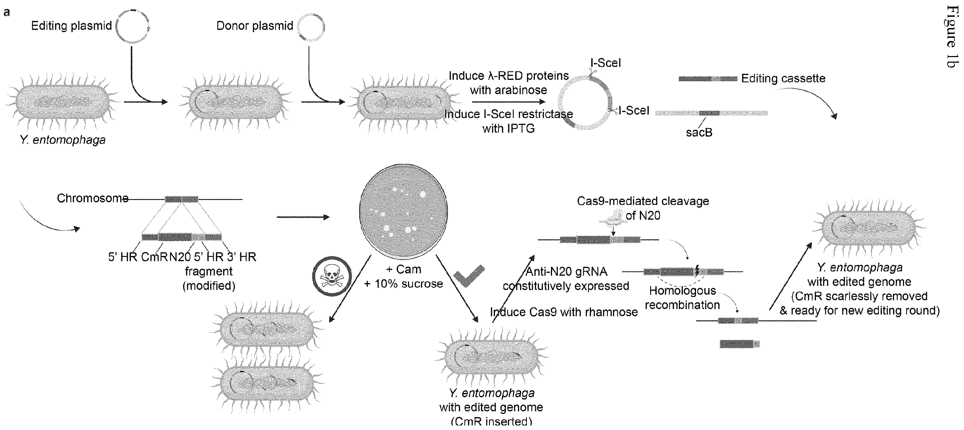

**Figure 1****: Fundamentals of the controlled setup used to find and characterize the YenTc secretion system. a,** Secreted protein fractions from *Y. entomophaga* cultured in various growth media. Secretion is inhibited in media that acidifies during cell growth. Bands corresponding to YenAl, YenA2 and YenB are boxed for clarity. **b,** Schematic representation of the targeted scarless genomic editing protocol established for *Y. entomophaga.*

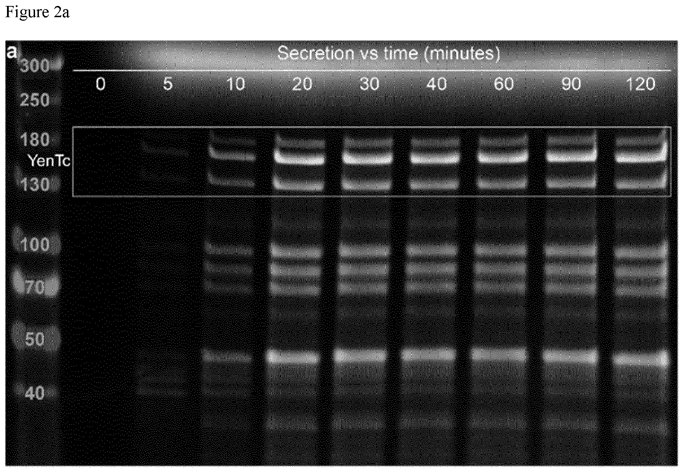

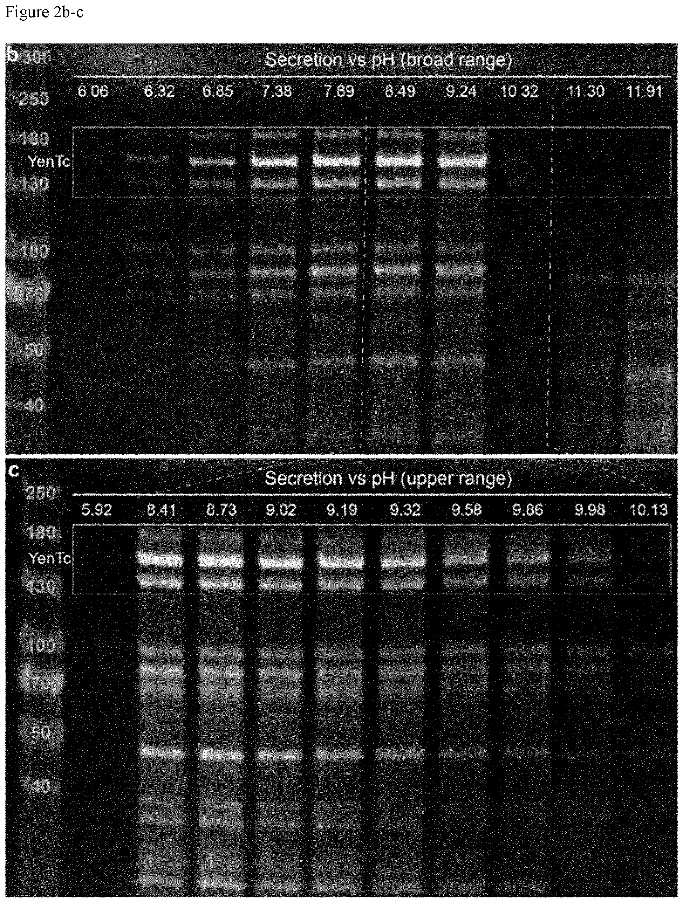

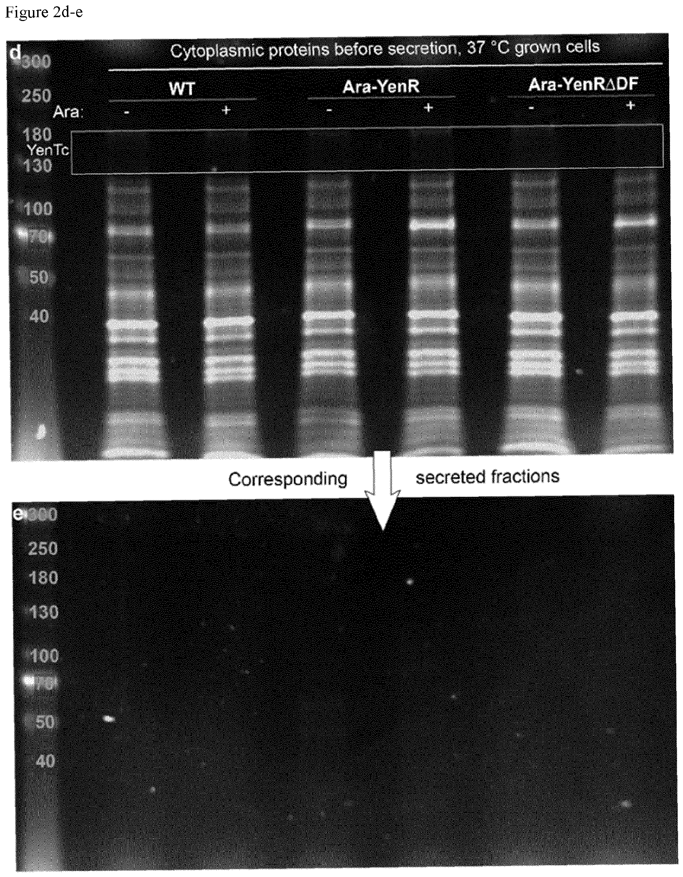

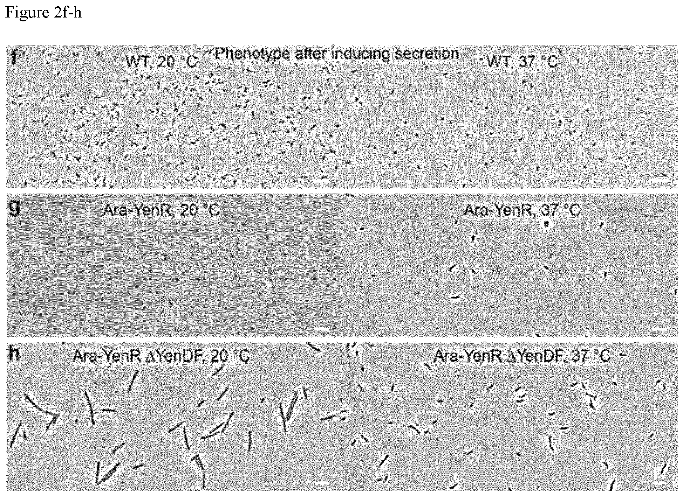

**Figure 2****: Soldier cells secrete YenTc within a few minutes and at near-neutral to slightly alkaline pH, only at lower temperatures. a,** *Y. entomophaga* cells grown in acidified SOC media rapidly secrete when pH is adjusted to 8.0. Bands corresponding to YenAl, YenA2 and YenB are boxed for clarity. **b,** Broad range screening of the pH dependence of secretion, assessed by adjusting the pH of acidified SOC media to the values indicated. Non-specific cell lysis seen in the two most alkaline samples demonstrates the upper limit of *Y. entomophaga* pH tolerance. **c,** Narrow range screening of the pH dependence of secretion to determine the upper pH limit of soldier cell specific secretion. **d-e,** *Y. entomophaga* does not produce or secrete toxins when grown at 37 °C as opposed to the usual 20 °C, even when YenR is induced. **f-g**, The phenotype of WT and Ara-YenR strain cells grown at 20 °C and 37 °C after pH-induced secretion.
**Figure 3****: *Y. entomophaga* soldier cells release YenTc and other toxins using YenDF, a T10SS. a,** Volcano plot of the MS analysis showing significant enrichment (t-test p-value < 0.05) of toxic proteins and a T10SS component in the secreted protein fraction compared to the non-secreted fraction. YenTc components: Chi2, YenAl, YenA2, YenB, YenC1 and YenC2. Other secreted toxins and virulence factors: Cbp, Chi3, NucA, Pil36, PirA, PirB, StcE and Tlh. YenDF structural components: YeEln. Other dark grey dots in the top right section of panel a: cytoplasmic proteins enriched in the secreted fraction. The relevant UniProt accession numbers are provided in the Methods section. n = 3 biological replicates. **b,** Only a minor fraction of an isogenic *Y. entomophaga* population produces YenTc-sfGFP (arrowheads). Scale bar: 20 µm. **c,** Timelapse of YenTc-sfGFP release from soldier cells (arrowheads) after the pH of acidified growth media was raised to 8.0. Scale bars: 5 µm. **d,** Knockout of YenDF components blocks secretion of *Y. entomophaga* soldier cells upon raising the pH of acidified growth media. The bands corresponding to YenAl, YenA2 and YenB are boxed for clarity. Inset: structure of the YenDF operon. Scale bar: 200 bp.

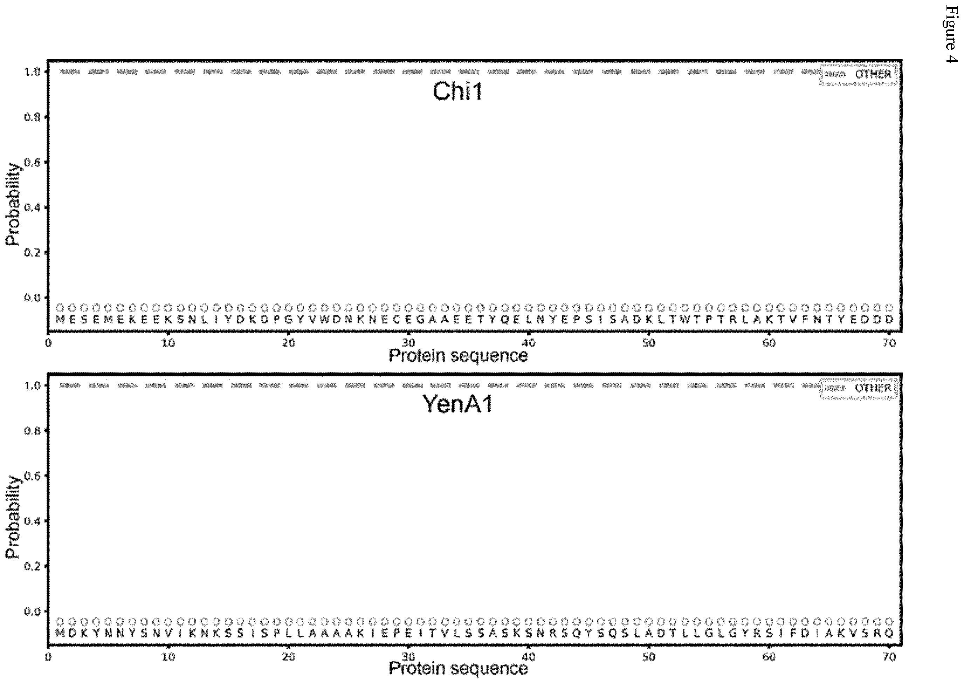

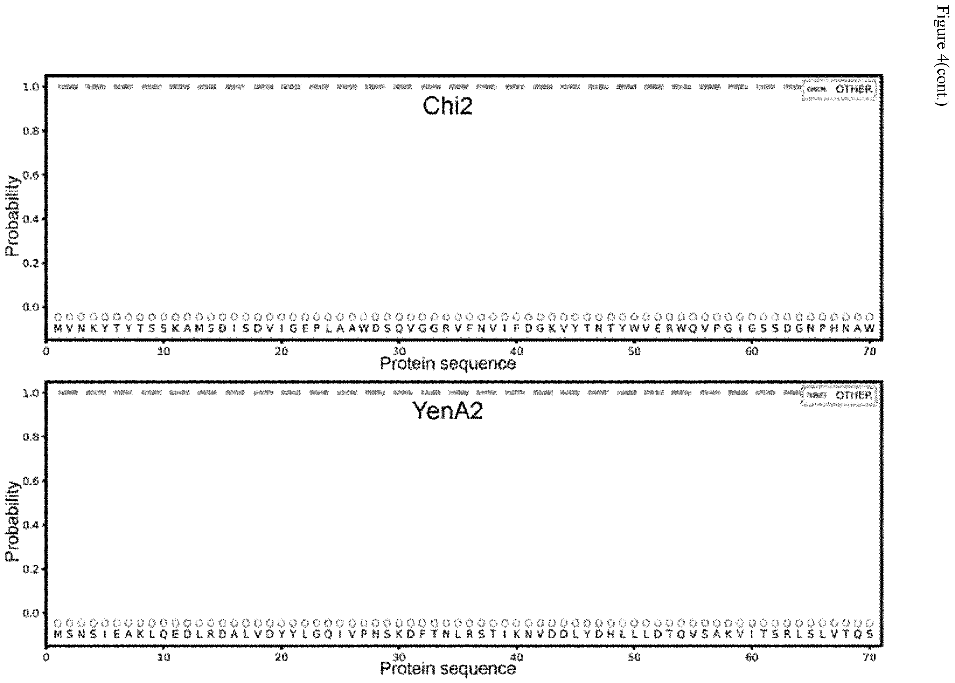

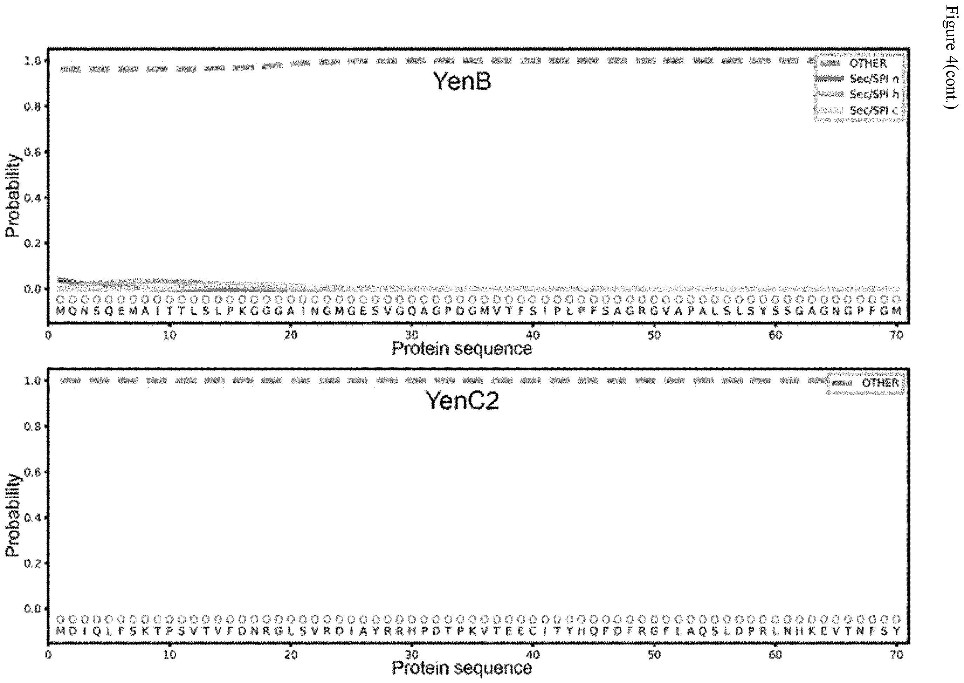

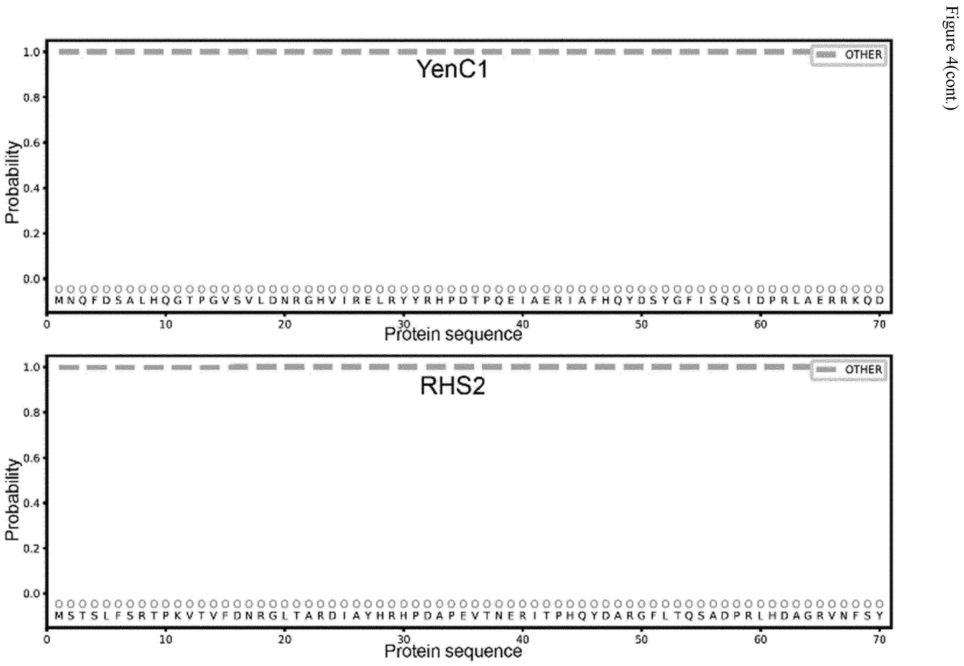

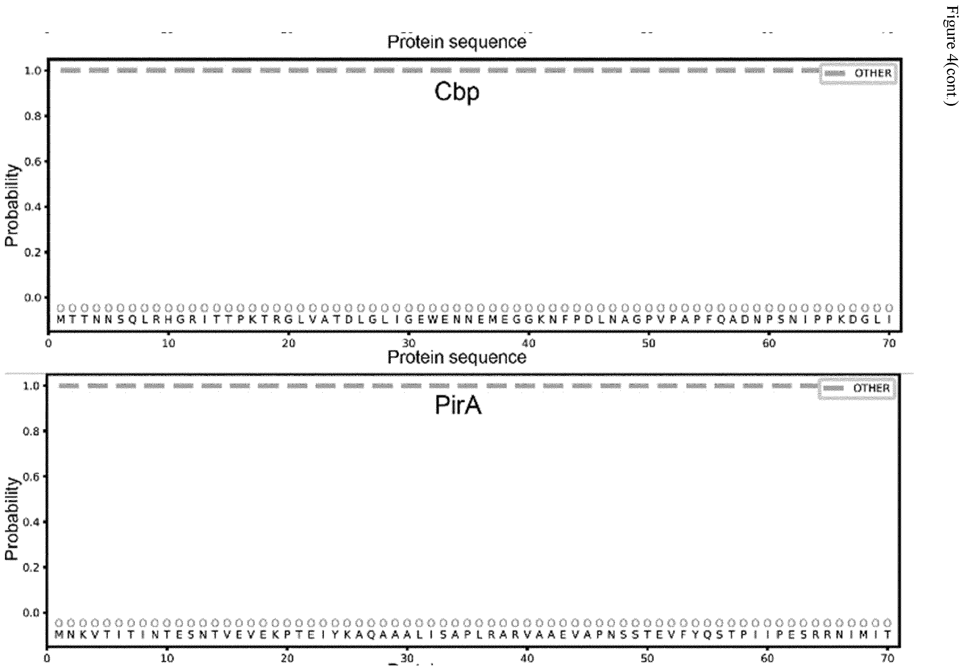

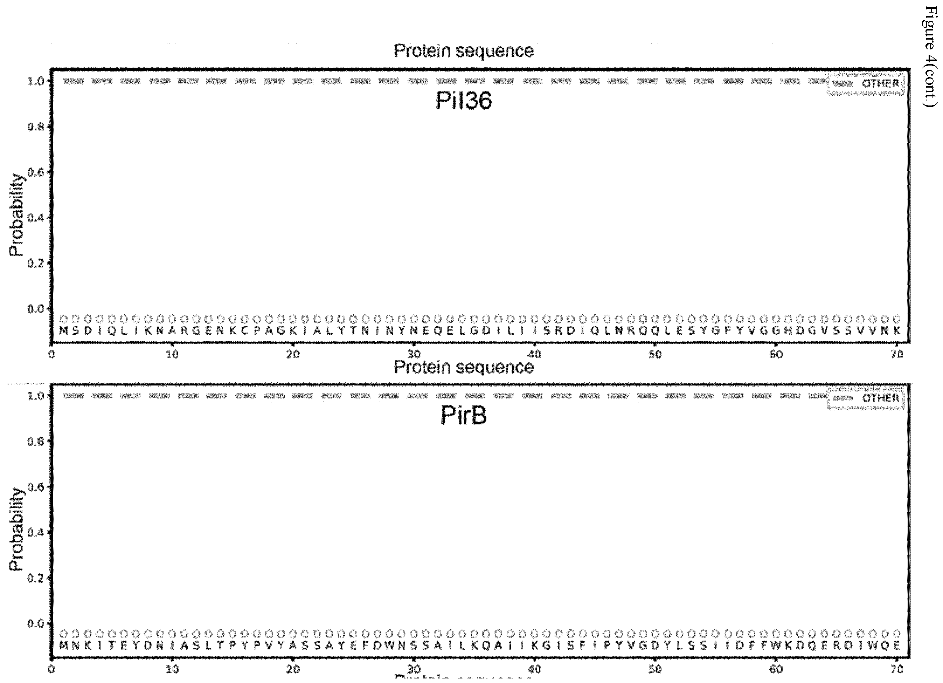

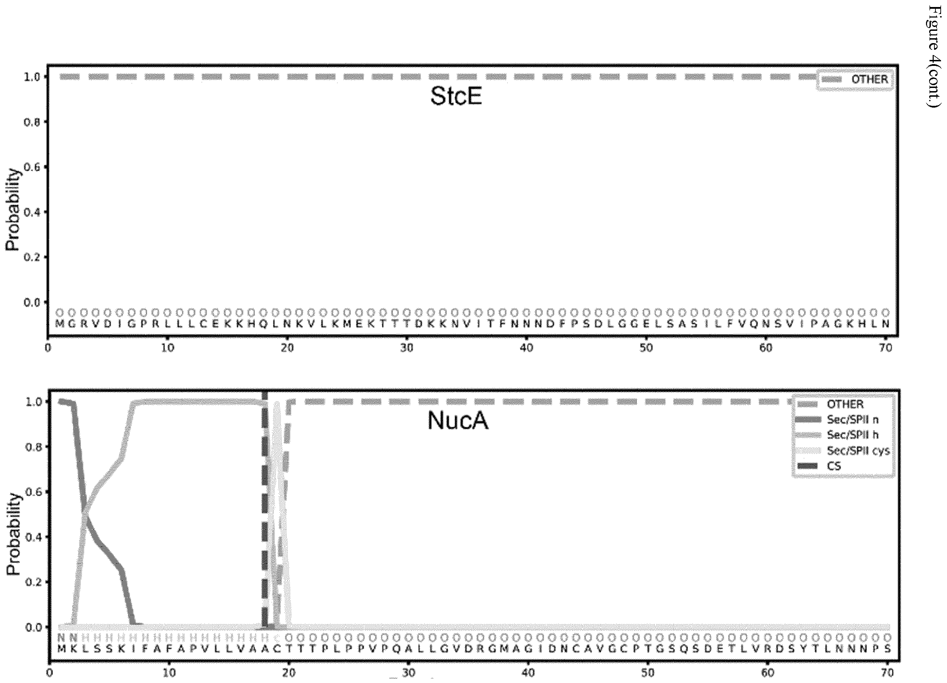

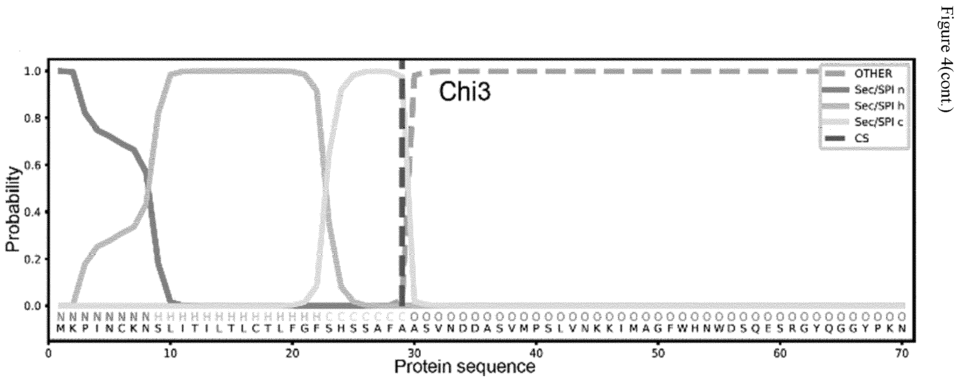

**Figure 4****: Nearly all YenR-controlled toxins and virulence factors lack an established signal sequence.** Predictions of secretion signal sequences for YenR-controlled toxins and virulence factors show that nearly all are unable to utilize alternative export pathways due to lack of a secretion signal sequence. The relevant UniProt accession numbers are provided in the Methods section.

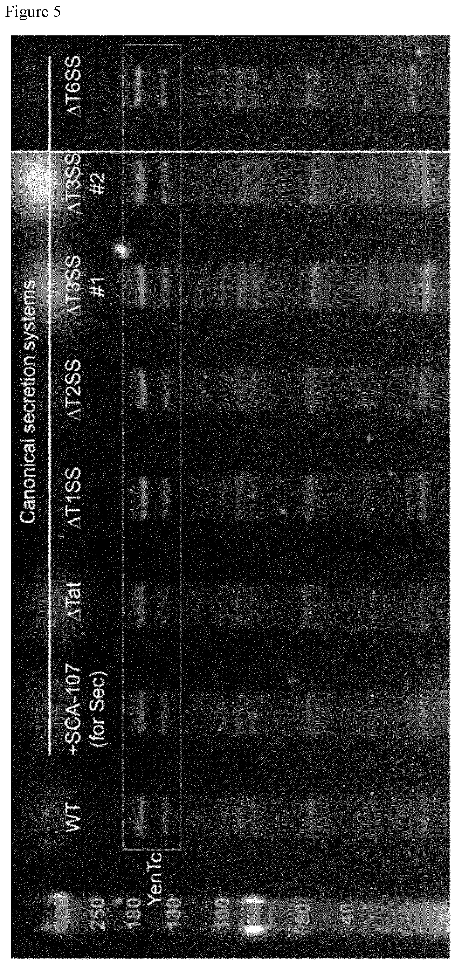

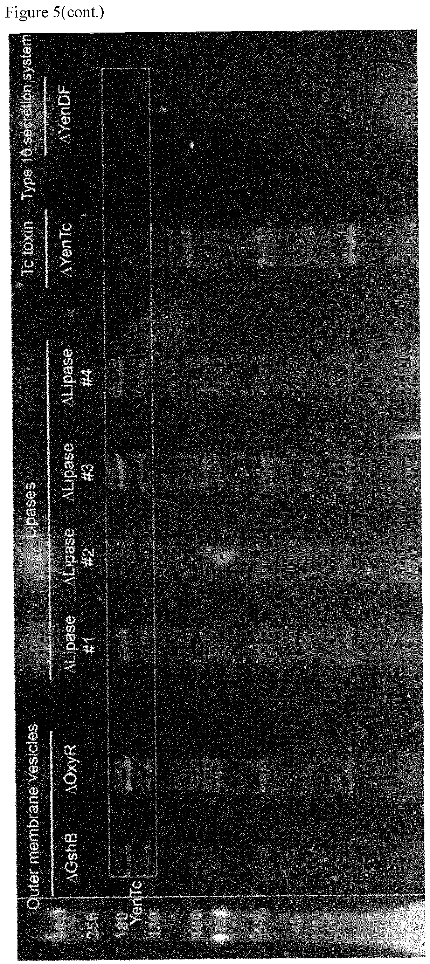

**Figure 5****: A T10SS was identified as the major pathway for *Y. entomophaga* protein secretion.** *Y. entomophaga* knockout strains of established anti-eukaryotic toxin secretion pathways or previously proposed Tc toxin export pathways have WT-like secretion levels, while the knockout of YenDF, a novel T10SS, abolished protein export completely. The Sec export pathway is essential and non-removable and was therefore blocked by the specific chemical inhibitor SCA-107⁷⁶. While no single gene controlling OMV formation is known, absence of OxyR and GshB very strongly decreased *E. coli* OMV formation levels⁷⁷. *Y. entomophaga* does not encode a direct Pdl1 lipase homologue that was proposed to be a *Photorhabdus* Tc toxin release factor, so four proteins with lipase-like domains that might serve as a proxy were targeted, as was YenTc itself to see if it has autotransporter-like capabilities. Bands corresponding to YenAl, YenA2 and YenB are boxed for clarity.

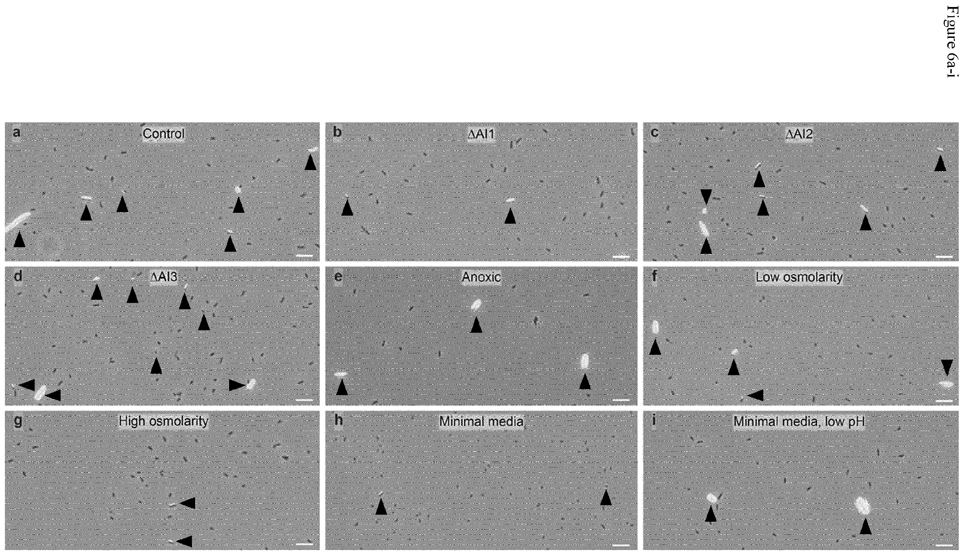

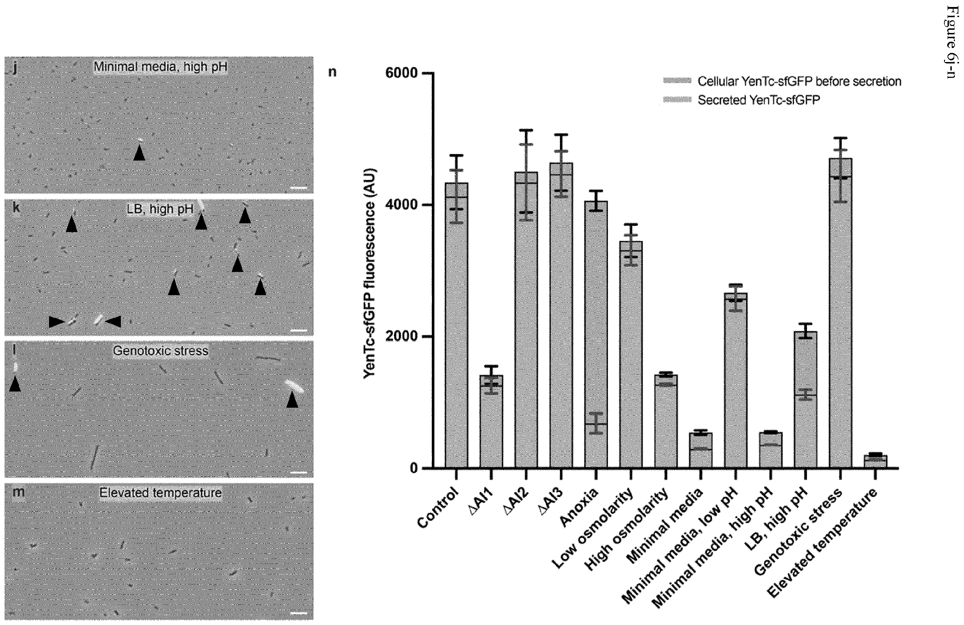

**Figure 6****: Differentiation into soldier cells is primarily controlled by thermosensing, nutrient availability, and autoinducer-1 quorum sensing molecules. a,** A genomic YenTc-sfGFP fusion was used as a qualitative marker of differentiation into soldier cells (arrowheads). *Y. entomophaga* was grown in the acidifying growth media SOC at 20 °C unless noted otherwise. **b-d**, Knockout of the acyl-homoserine-lactone synthase (UniProt accession number: A0A3S6EWZ2), the S-ribosylhomocysteine lyase (UniProt accession number: A0A3S6F351), and the L-threonine 3-dehydrogenase (UniProt accession number: A0A3S6EZE1) to test for the essentiality of autoinducer-1, -2 and -3 type quorum sensing molecules in differentiation, respectively. Absence of autoinducer-1 molecules decreases the number of soldier cells noticeably. **e,** Growth in a pure nitrogen atmosphere to test the effect of anoxia on differentiation. Interestingly, anoxically grown cells demonstrate defective YenTc secretion, but unimpaired YenTc production **(n). f-g**, Growth in SOC media prepared with 0 or 570 mM NaCl to test the effect of decreased and increased osmolarity on differentiation. Normal SOC media contains 190 mM NaCl. **h,** Growth in M9 minimal media to test for the essentiality of complex organic molecules for differentiation. In the absence of complex organic molecules, the cells tend to adopt minimal dimensions and strongly reduce differentiation into soldier cells. **i-j,** Same as in **(e)** but pre-adjusted to pH 6.10 / pH 7.70 to respectively test for the influence of acidic and alkaline pH on differentiation in absence of complex organic compounds. Acidic, but not alkaline pH, stimulates production of YenTc **(n). k**, Growth in LB media, which increases its pH value to 7.50 after cell growth, to test how alkaline pH in presence of complex organic compounds affects differentiation compared to **(a)** and **(j). l**, Growth in media supplemented with 200 ng/mL mitomycin C to test the effect of genotoxin stress on differentiation and the relevance of the SOS response pathway, which tends to generate enlarged cells by inhibition of cell division. **m,** Growth at 37 °C to test the effect of elevated temperature on differentiation. Given the lack of observed YenTc-sfGFP expression, of all factors tested here high temperature affects the propensity of naive cells to differentiate the most. Scale bars: 10 µm. **n**, Differentiation into soldier cells (light grey bars) and their secretory ability (dark grey bars) in conditions from **(a-m)** was quantified by measuring fluorescence of YenTc-sfGFP in the pre-secretion and secreted fractions, respectively, of density-normalized cells. Data is shown as mean ± standard deviation, n = 3 biological replicates.

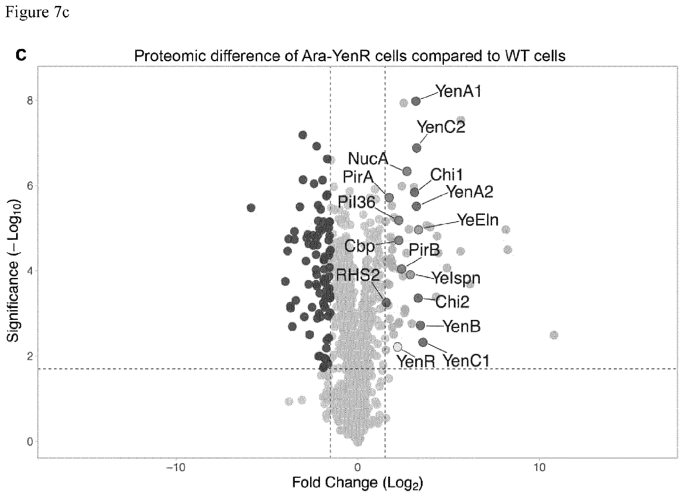

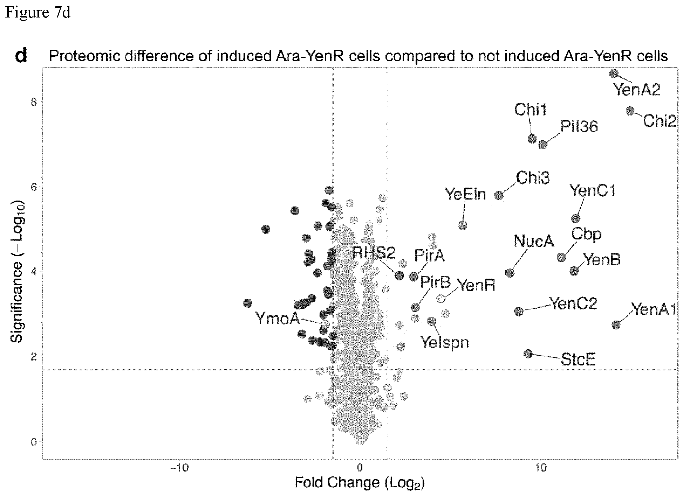

**Figure 7****: Soldier cells use YenR and YmoA to synchronize the expression of YenDF with that of a deadly cocktail of toxins. a,** Arabinose induction of the Ara-YenR strain causes a massive increase in toxin production and secretion compared to wild type (WT) cells, while absence of induction abolishes these completely. **b,** Induction of YenR in an Ara-YenR YenTc-sfGFP strain causes all cells to convert into a soldier cell phenotype confirmed by GFP expression in all cells, while in absence of induction no soldier cells appear and no GFP expression was detected. Scale bar: 20 µm. **c-d,** Volcano plot showing significant enrichment (t-test p-value < 0.02) of toxic proteins and YenDF components in the total protein fraction of induced Ara-YenR cells compared to WT cells (c) or non-induced Ara-YenR cells (d). YenTc components: Chi1, Chi2, RHS2, YenAl, YenA2, YenB, YenC1 and YenC2. Other secreted toxins and virulence factors: Cbp, Chi3, NucA, Pil36, PirA, PirB and StcE. YenDF structural components: YeEln and Yelspn, additionally the regulatory proteins YenR and YmoA are marked. n = 3 biological replicates each. **e,** Anhydrotetracycline-driven expression of additional plasmid-borne YmoA in a YenTc-sfGFP strain causes cells to convert to the soldier cell phenotype. Scale bar: 20 µm. **f**, Expression of additional plasmid-borne YmoA does not rescue the non-toxic, non-secretory phenotype of non-induced Ara-YenR cells. **g**, Expression of additional plasmid-borne YmoA boosts the production of mCherry-tagged YeHln in induced Ara-YenR cells. Data is shown as mean ± standard deviation, n = 3 biological replicates.

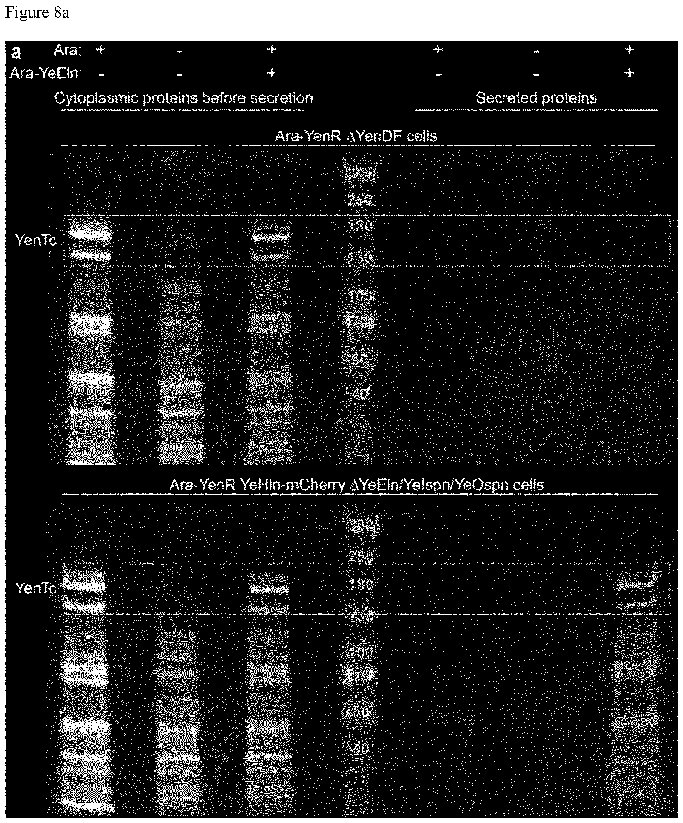

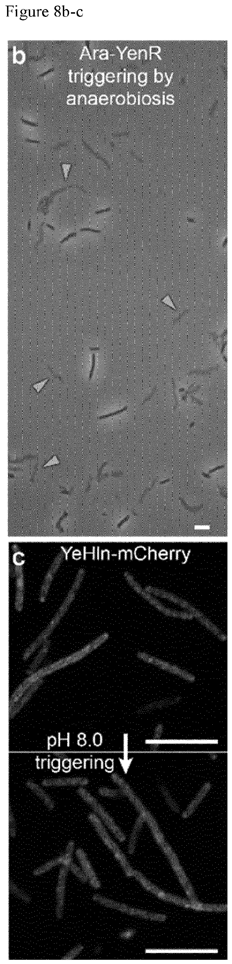

**Figure 8****: pH change triggers YeHln, which concentrates into numerous small foci. a,** YeHln-mCherry enables protein release in response to elevation of pH, in Ara-YenR ΔYeEln/YeIspn/YeOspn cells expressing Ara-YeEln from a plasmid. Bands corresponding to YenAl, YenA2 and YenB are boxed for clarity. **b,** Aerobic shaking cultures of Ara-YenR cells can be stimulated to undergo YenDF-mediated secretion by immobility-induced anaerobiosis. Arrowheads denote examples of cells that already underwent YenDF-mediated secretion. See **Fig. 7b** for an intact reference. **c,** Confocal fluorescence microscopy of Ara-YenR YeHln-mCherry ΔYeEln/YeIspn/YeOspn cells shows that small YeHln rafts are abundantly distributed across the bacterial membrane both prior to and following pH-induced triggering of YenDF secretion, in contrast to the few large lesions observed for triggered phage holin S105⁴¹.

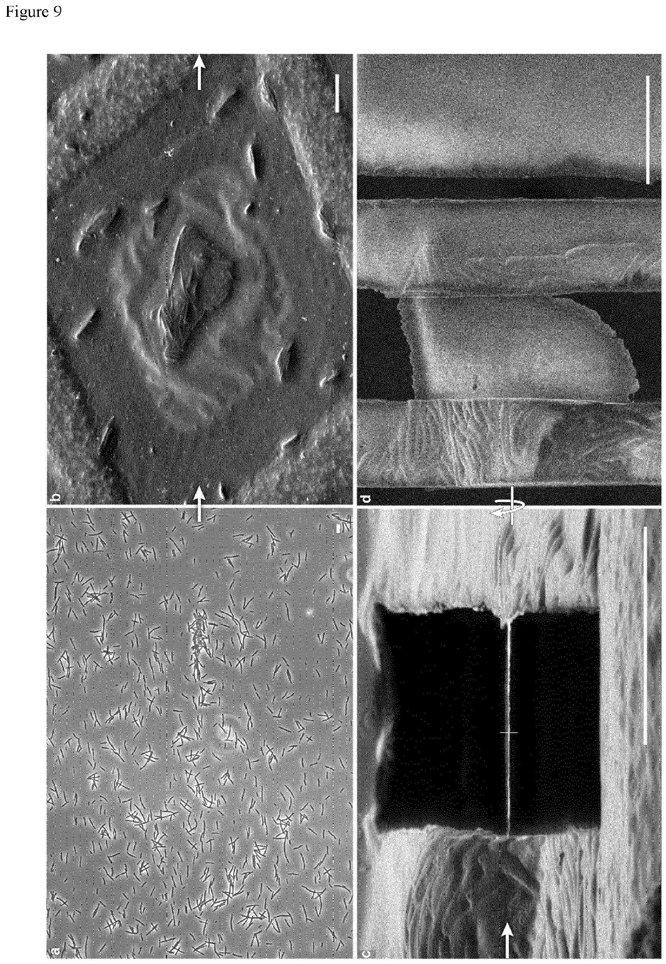

**Figure 9****: Bacterial sample preparation by cryo-focused ion beam milling. a,** Induced Ara-YenR ΔYenDF cells used to investigate the pre-secretion state of soldier cells. Scale bar: 10 µm. **b,** SEM micrograph of plunge-frozen, pH-triggered Ara-YenR ΔYenDF cells on an EM grid, prior to lamella generation. Scale bar: 10 µm. **c-d,** SEM micrograph of an Ara-YenR ΔYenDF cell lamella milled with a focused ion beam, side and top view. Scale bars: 10 µm.

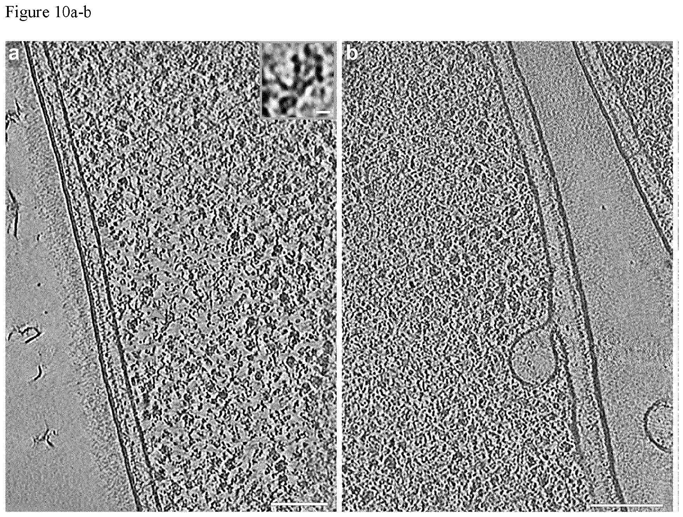

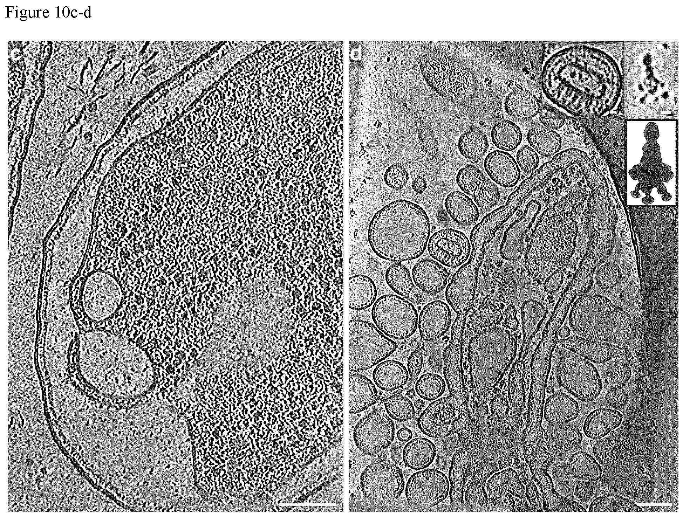

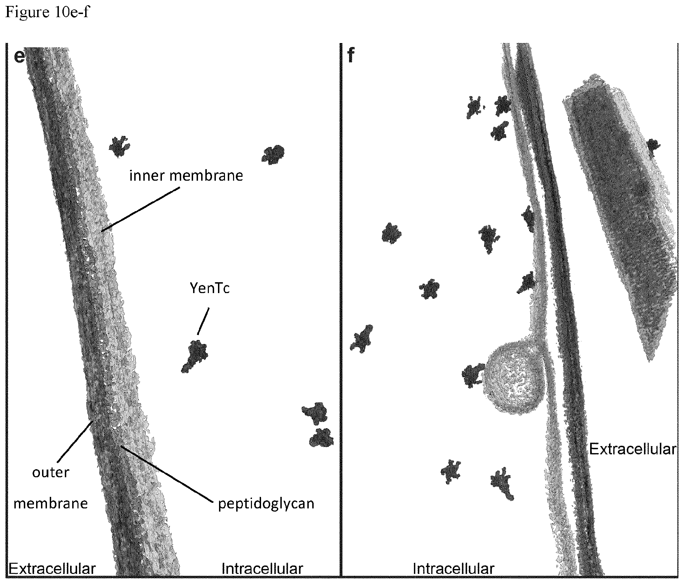

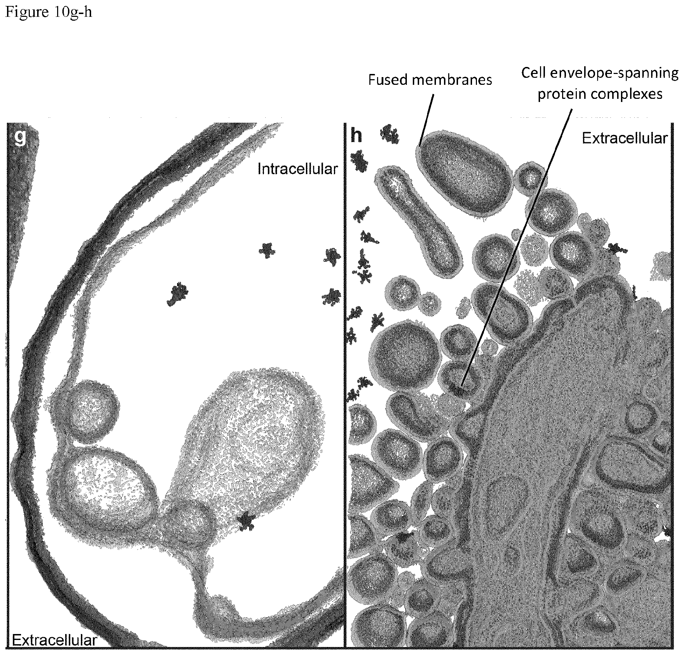

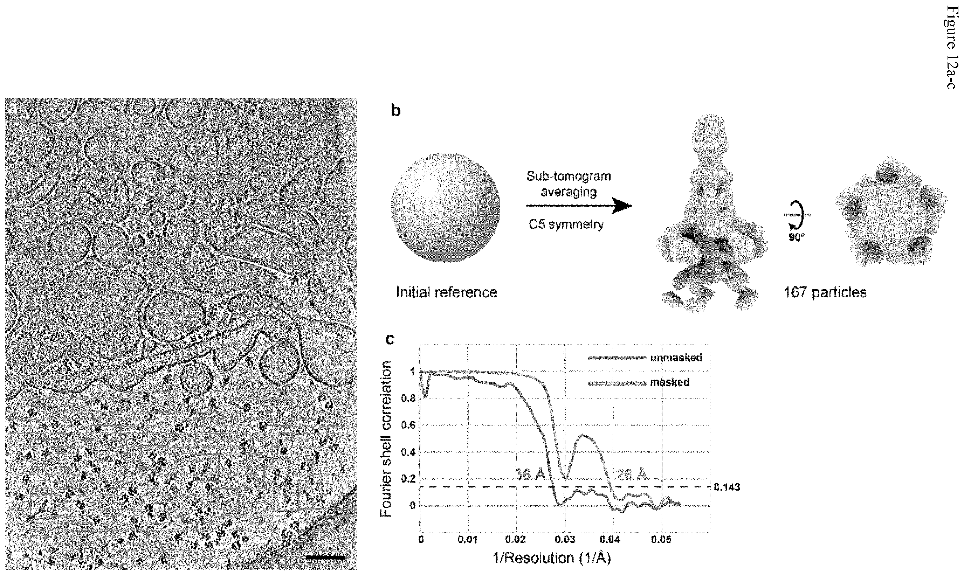

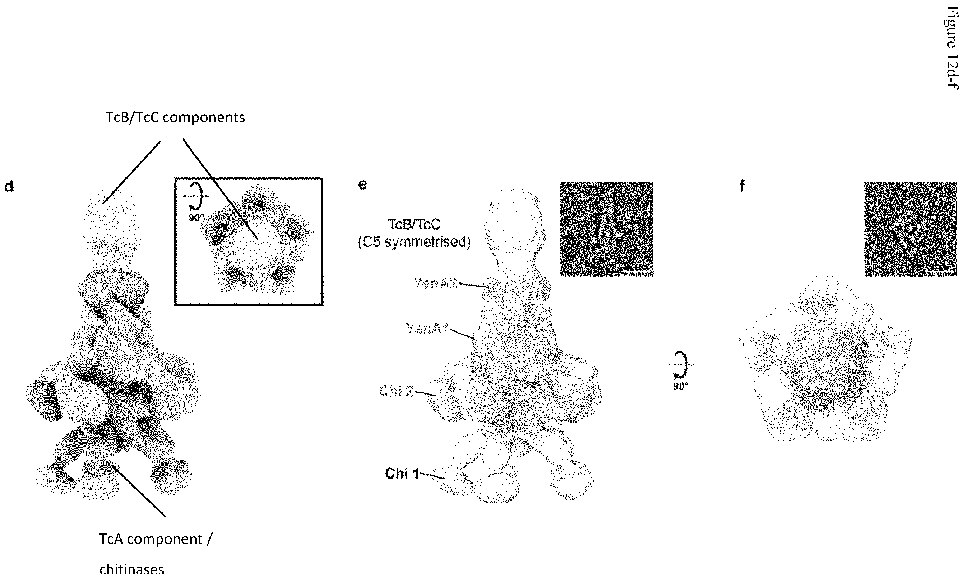

**Figure 10****: Action of YenDF and release of YenTc visualized by cryo-ET. a**, A single slice from an Ara-YenR ΔYenDF cell tomogram, representing the pre-secretion state. Inset: a fully assembled cytoplasmic YenTc holotoxin. An arrowhead denotes its position in the tomogram slice. Scale bar: 10 nm. **b**, Ara-YenR ΔYenEln/YeIspn/YeOspn cell tomogram slice, representing the state of secretion after holin action. **c,** Ara-YenR ΔYeIspn/YeOspn cell tomogram slice, representing the state of secretion after endolysin action. **d,** Ara-YenR cell tomogram slice, representing the state of secretion after spanin action. Left inset: unfused vesicle derived from an area of the cell envelope that contained proteins spanning the entire cell wall. A red arrowhead denotes its position in the tomogram slice. Scale bar: 10 nm. Right inset: A YenTc holotoxin secreted to the external environment. An arrowhead denotes its position in the tomogram slice. Scale bar: 10 nm. Lower inset: Structure of the secreted YenTc holotoxin complex determined by subtomogram averaging. See **Fig. 12** for more details. Scale bars in all tomogram slice views: 100 nm. **e-g,** Annotated densities from the tomograms shown in **(a-d). h,** Annotated densities from the tomogram shown in **(d),** presented as a diagonally sectioned view to demonstrate the internal structure that arises after spanin action. dark blue: cell envelope-spanning protein complexes.

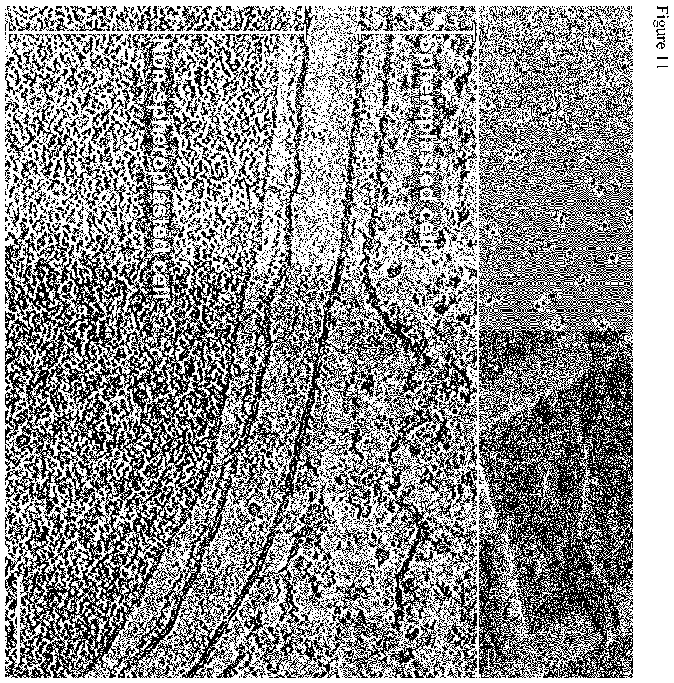

**Figure 11****: Spanin knockout soldier cells form spheroplasts with attractive qualities for *in situ* tomographic protein analysis. a,** Ara-YenR ΔYeIspn/YeOspn strain cells form spheroplasts after pH-induced activation. Scale bar: 10 µm. **b,** SEM micrograph of plunge-frozen AraYenR ΔYeIspn/YeOspn cells that were pH-triggered on an EM grid, prior to lamella generation. Arrowhead: an example spheroplast. Scale bar: 10 µm. **c,** A single slice from a AraYenR ΔYeIspn/YeOspn cell tomogram illustrating the difference in interior protein density of a spheroplasted cell compared to a non-spheroplasted cell. Notice the ultrastructural changes to the spheroplast inner membrane that occur as a result of cell expansion. Arrowheads: an example YenTc holotoxin from a spheroplast (side view) and non-spheroplast (top view). Scale bar: 100 nm.
**Figure 12****: Subtomogram averaging of YenTc released by soldier cells. a,** A 9 nm thick slice of a representative tomogram of Ara-YenR cells. YenTc particles picked for subtomogram averaging are highlighted in boxes. Scale bar: 100 nm. **b,** Reference and final structure from subtomogram averaging. **c,** Gold-standard FSC curve of the YenTc structure. The dip in the FSC curve at 33 Å corresponds to the first zero of the CTF curve at a defocus of -5.5 µm, which is the defoci of the majority of the particles. **d,** The structure of YenTc with individually highlighted protomers of TcA. The TcB/TcC components are also marked. Inset: top view. **e-f,** Side and top view of the YenTc structure fitted with a structural model of the YenTc TcA component (PDB: 6OGD)¹⁴. Insets are cross-sections of the structure. Scale bar: 20 nm.

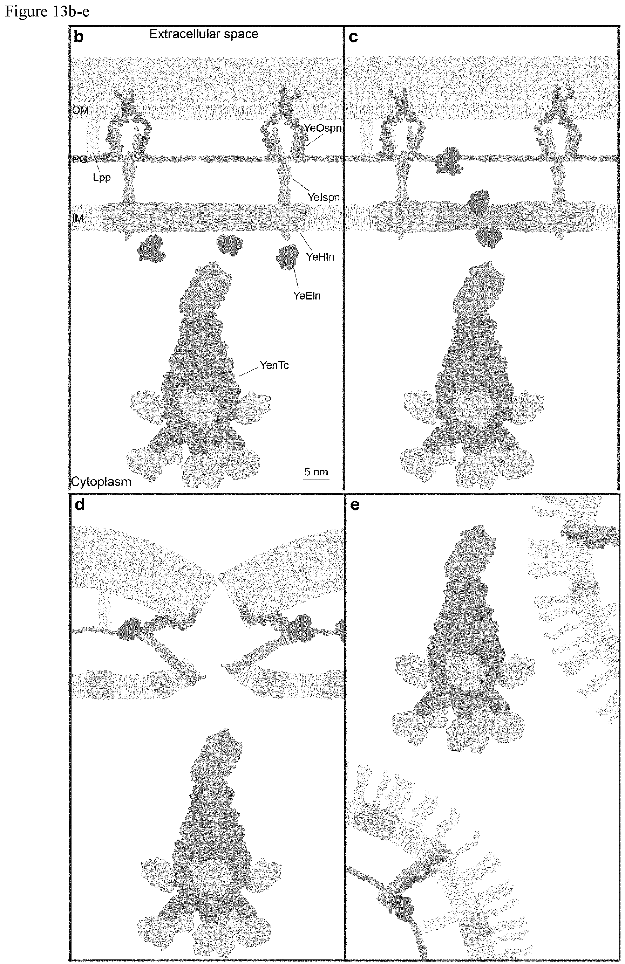

**Figure 13****: Model for soldier cell differentiation and subsequent YenDF-mediated YenTc release, a,** The proposed interplay of the regulatory proteins YenR and YmoA during differentiation into soldier cells, leading to transcription of YenTc components, YenDF components, additional toxins and virulence factors, and the characteristic enlarged phenotype of soldier cells by affecting an as yet undetermined set of genes, as well as shutdown of new YmoA production. H-NS is the histone-like protein that YmoA usually partners with³⁵. **b,** During YenR-mediated differentiation, soldier cells produce numerous toxins including YenTc, which is fully assembled in the cytoplasm. Components of the T10SS YenDF are produced simultaneously. YeEln remains in the cytoplasm, YeHln assembles into oligomeric rafts in the inner membrane, and the inner membrane embedded YeIspn forms a complex with the outer membrane embedded YeOspn. **c,** pH elevation that likely deteriorates the proton motive force triggers the formation/opening of small pores in the YeHln rafts, allowing YeEln to reach the periplasm and cleave peptidoglycan cross-links. **d,** Once enough cross-links have been cleaved, the spanin complex undergoes a conformational change that brings the inner and outer membranes into direct contact, leading to the fusion of the outer and inner membrane. **e,** The fused membranes collapse into loosely bound clusters of vesicles still tethered to peptidoglycan via the protein Lpp. The accompanying release of inner osmotic pressure propels YenTc and other soldier cell toxins into the surrounding environment.

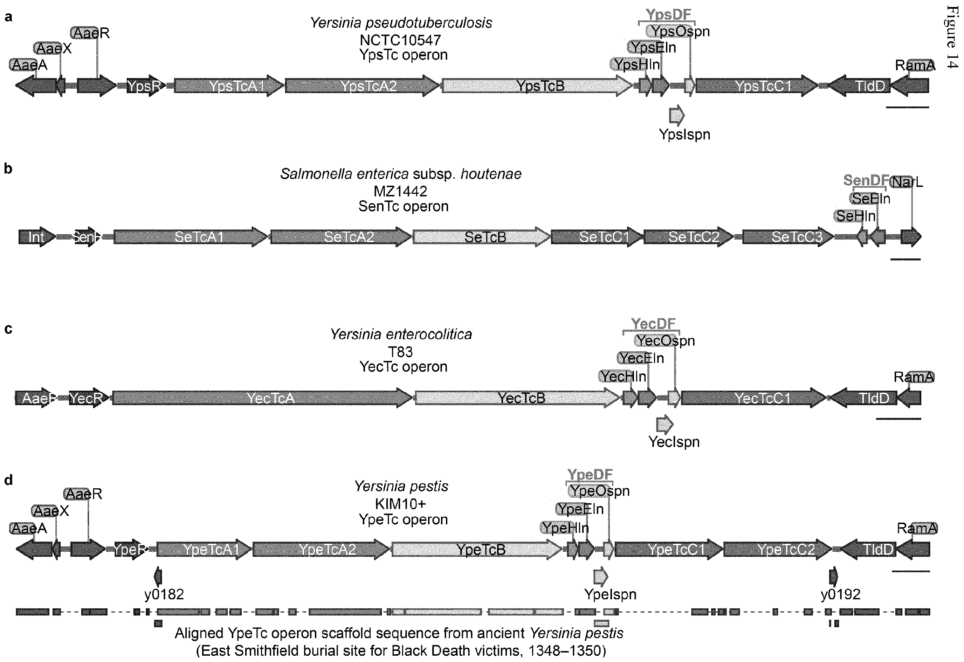

**Figure 14****: Major human pathogens have type 10 secretion systems associated with Tc toxin genomic regions. a-d,** The Tc toxin genomic regions from *Yersinia pseudotuberculosis* (strain NCTC10547), *Salmonella enterica* subsp. *houtenae* (strain MZ1442), *Yersinia enterocolitica* (strain T83) and *Yersinia pestis* (strain KIM10+), with the embedded T10SSs designated as YpsDF, SenDF, YecDF and YpeDF in analogy to YenDF. The holin component is marked with the suffix Hln, endolysin with the suffix Eln, spanins with the suffixes Ispn and Ospn, putative transcriptional regulator of the Tc toxin with the suffix R, TcA components with the suffix TcA/TcA1/TcA2, TcB with the suffix TcB, TcC wit the suffixes TcC1/TcC2/TcC3. All other genes are shown to indicate the genomic context of Tc toxins. Inset of **(d):** the aligned YpeTc operon scaffold sequence from a London victim of the Black Death pandemic, ca. 1348-1350⁵⁰. Regions shown with a dashed line had no available contigs due to degradation of ancient DNA. Scale bars: 1000 bp.

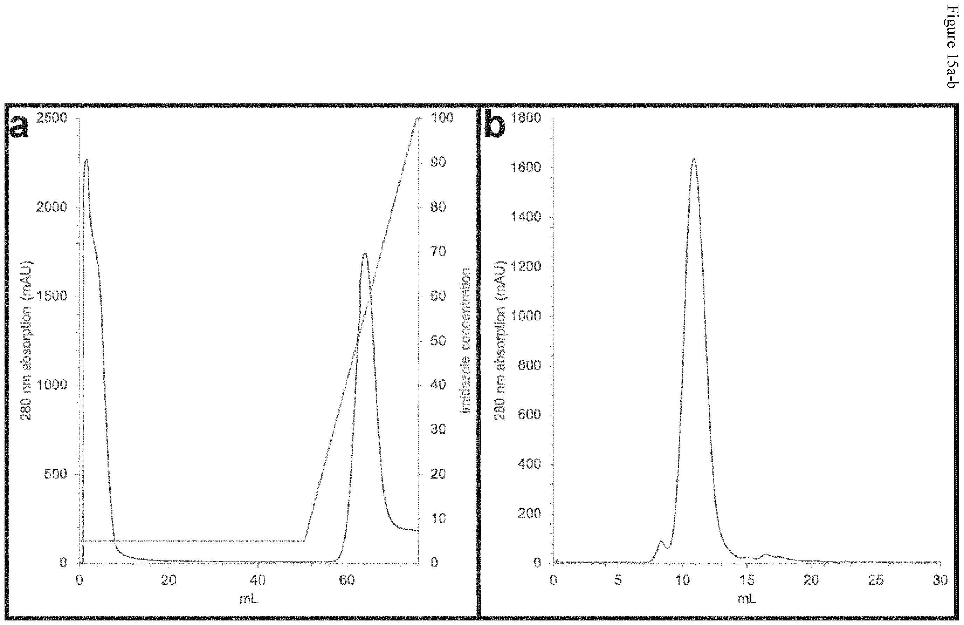

**Figure 15****: High throughput purification of YenTc toxin using Ara-YenR cells. a-b,** YenTc-His purified from the secreted mix of *Y entomophaga* insecticidal toxins, first by Ni²⁺-NTA affinity chromatography and then by size exclusion chromatography, showing monodisperse elution peaks. **c,** Quality control by negative stain transmission electron microscopy confirms that the purified YenTc is homogeneous and properly folded. Inset: class average of multiple YenTc particles. **d,** A comparison of pure YenTc-His yield obtained from 1L of cells with unmodified (WT) YenR and from the Ara-YenR strain.

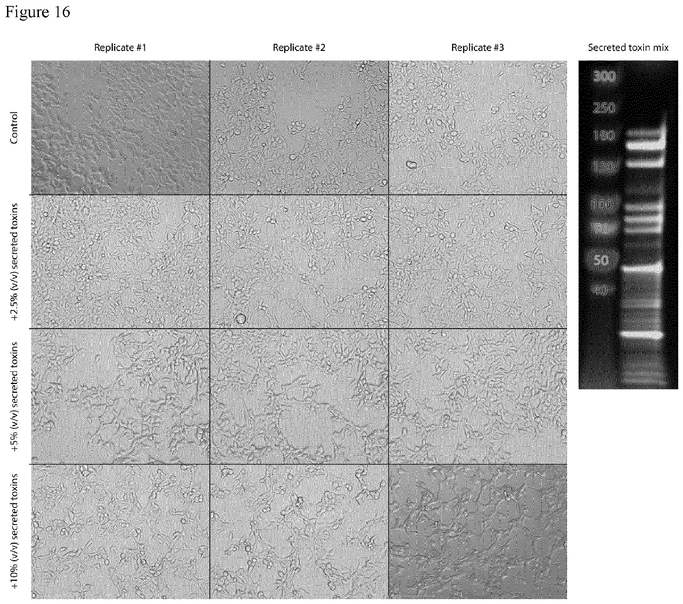

**Figure 16****: Toxicity test of toxin composition on human derived HEK293T cells** The toxin mixture secreted by *Y. entomophaga* (gel image, right) is not harmful to cultured human cells even at high concentration (cell micrographs, left).

### Example 1: Specialized pathogenic cells release Tc toxins using a type 10 secretion system

### Introduction

When attacking eukaryotes, pathogenic bacteria secrete an array of toxic proteins that modulate the defences of the host on a cellular level allowing the pathogen to establish an infection.

Toxins of Gram-negative bacteria use various secretion systems to cross three major barriers when exiting the cell: the phospholipid inner membrane, peptidoglycan sacculus, and lipopolysaccharide outer membrane¹. It has so far been established that type 3, 4 and 6 secretion systems (T3SS, T4SS and T6SS) inject toxins directly into host target cells, while the T1SS, Tat/T2SS, Sec/T2SS, Sec/T5SS and outer membrane vesicle (OMV) pathways eject toxins into extracellular space^{1,2}. Each secretion system introduces unique requirements to its toxin substrates. Those that utilize the Sec, T1SS, T3SS and T4SS machinery must be cytoplasmically unfolded, and nearly all need a signal sequence to direct them to the correct secretion apparatus¹. The vast majority of secreted toxins are limited to tens of kilodaltons (kDa) in size due to the physical dimensions of their secretion systems, although certain larger cargos such as the 900 kDa RTX toxins can still be threaded through a T1SS, a feat afforded by their cytoplasmically unfolded repeat domain architecture³.

In contrast, Tc toxins are an example of megadalton-scale, non-phage-derived proteins with a complex architecture⁴ for which the secretion mechanism remains highly enigmatic⁵. Originally discovered in the insecticidal bacterium *Photorhabdus luminescens⁶,* these protein complexes are now known to be important virulence factors in numerous highly prominent insect and human pathogens⁷. The structure of Tc toxins and the roles of their three core subunits TcA, TcB and TcC have been extensively investigated in the past several years⁸⁻¹⁴. While the details of Tc complex assembly and secretion in a biological context are so far missing⁴, evidence suggests that secreted Tc toxins attach to their target cells via glycan receptors¹⁴⁻¹⁷ and undergo subsequent endocytosis. Endosomal acidification triggers a conformational change by collapsing the linker domains of the five TcA protomers, which drives the central TcA channel into the endocytic membrane¹². The membrane-embedded tip of the channel then opens¹¹, injecting a cytotoxic moiety originally stored in the TcB-TcC cocoon into the target cell cytoplasm⁸, where it modifies substrates such as Rho GTPases and the actin cytoskeleton, ultimately triggering cell death^{8,18}.

Several competing hypotheses exist regarding Tc toxin secretion. TcB-/TcC-driven translocation of a *P*. *luminescens* Tc toxin via an auto-transport mechanism^{19,20} with attachment to the bacterial cell surface²¹ and subsequent lipase-assisted release into the surrounding environment¹⁹ has been proposed on one hand. On the other, secretion of the *Yersinia pestis* and *Yersinia entomophaga* Tc toxins has been suggested to occur via the T3SS and OMVs, respectively^{22,23}.

We aimed to elucidate the Tc toxin secretion mechanism by making use of *Y. entomophaga,* a close relative of the deadly human pathogen *Y. pestis* that has instead evolved to target insects. The virulence factor that makes *Y. entomophaga* extremely lethal to insects is its sole Tc toxin, YenTc, which it secretes into the environment²³. The intricate multi-subunit structure of this toxin¹⁴ gives rise to a complex of roughly 2.4 MDa, which is comparable in size to the 2.5 MDa prokaryotic ribosome.

We use a cutting-edge combination of microscopy, proteomic analyses and targeted genomic knockouts to reveal that a small subset of specialized cells, which we term soldier cells, releases YenTc as well as numerous other virulence factors and toxins using a pH-sensitive type 10 secretion system (T10SS). We then identify the regulatory proteins that control the soldier cell phenotype and couple production of YenTc to the T10SS. Finally, we use cryo-electron tomography (cryo-ET) to visualize the step-by-step action of a T10SS and the release of a Tc toxin for the first time in a cellular context. These results irrefutably resolve the mystery surrounding Tc toxin secretion in insect and human pathogens, and demonstrate how specialized toxin-producing cells confer virulence to an entire bacterial population.

### Results

### pH-dependent secretion of Tc toxins

In order to investigate the secretion of Tc toxins, we started by exploring the influence of growth media composition on YenTc production and secretion. We immediately noticed that *Y. entomophaga* secretes very poorly into media that acidify during cell growth compared to non-acidifying media **(****Fig. 1a****).** This led us to hypothesize that protein secretion from *Y. entomophaga* is pH-dependent, and indeed raising the pH of the acidified medium from 5.5-6.0 to 7.0-8.0 or reconstituting the cells in a buffer with elevated pH caused them to secrete a multitude of proteins within 10 minutes **(****Fig. 2a****).** We systematically screened the pH dependence of secretion and found that it occurs in the range of pH 6.3-10.0, with an optimum between pH 6.9-8.9 **(****Fig. 2b****-c).** Many insect families, including Coleoptera to which the natural *Y. entomophaga* host *Costelytra zealandica* belongs, have an acidic anterior midgut and a more alkaline posterior midgut^{24,25}. The identified pH-sensitivity of secretion would cause release of YenTc near the latter, which is where invading *Y. entomophaga* were found to actively establish themselves²⁶.

We next turned to mass spectrometry to identify the proteins co-secreted with YenTc and compare them to those remaining in the cells. The secreted fraction was highly enriched not only in YenTc, but also in a variety of other toxins and virulence factors such as PirAB, NucA and chitin-modifying proteins **(****Fig. 3a****),** indicating that *Y. entomophaga* produces and secretes a highly devastating protein cocktail. Mysteriously, most lack an established signal sequence for export **(****Fig. 4****).**

### Soldier cells secrete using a T10SS

To identify the pathway these bacteria employ to release YenTc and possibly other constituents of the toxic cocktail, we established a targeted scarless genomic editing protocol for *Y. entomophaga* **(****Fig. 1b****).** After knocking out all established secretion systems in *Y. entomophaga,* as well as components of the non-standard secretion pathway hypothesized for the *P. luminescens* Tc toxin^{19,20}, all resulting strains were fully secretion-competent **(****Fig. 5****),** meaning that neither of the previously proposed mechanisms nor other common secretion systems are used for export of YenTc and other *Y. entomophaga* toxins.

To resolve this mystery, we proceeded by fusing the YenA1 component of YenTc with sfGFP in order to directly monitor secretion of the toxin. Fluorescence microscopy revealed the surprising fact that only a small subpopulation of these isogenic cells expressed the toxin at any given time **(****Fig. 3b****).** Furthermore, the toxin-expressing cells were often morphologically distinct from the rest in the post-log phase **(****Fig. 3c****),** being larger and noticeably less motile. Of all factors tested (quorum sensing, oxygen levels, genotoxic stress, temperature etc.), lower temperature had by far the strongest influence on the propensity of cells to become YenTc producers **(****Fig. 6a****-n),** in line with the observation that *Y. entomophaga* does not secrete this toxin into growth media at temperatures above 30 °C²³. This makes biological sense for such an insect pathogen, since any organism it may encounter with a highly elevated body temperature is unlikely to be a suitable host. Absence of another host marker, complex organic compounds, also strongly disincentivized differentiation into YenTc-producing cells **(****Fig. 6h****,** **n****).** Finally, we reduced the number of YenTc-expressing cells in the population by two thirds when we knocked out production of autoinducer-1 quorum sensing molecules. This showcases the importance of interbacterial communication in the decision to produce YenTc, and interestingly correlates with the reduced secretion of a corresponding hit from a transposon mutagenesis assay³¹.

We then used confocal fluorescence microscopy to visualize the pH-induced secretion of YenTc-expressing cells. While cells not expressing YenTc remained unaltered, the enlarged rod-like morphology of YenTc-expressing cells underwent a striking metamorphosis in a matter of minutes, collapsing into an associated cluster of vesicles and releasing the previously cytoplasmically localized YenTc in the process **(****Fig. 3c****).** Thus, the cells that produce YenTc are sacrificed for the release of the toxin. This demonstrates that the pH-dependent secretion of YenTc is actually not a typical secretion process, but the result of a controlled lysis strictly dedicated to toxin release. This remarkable functional and morphological differentiation of YenTc-producing cells compared to their naive counterparts led us to term them soldier cells. In the light of soldier cells using controlled lysis to release YenTc, we found the exclusive appearance of an M15 family metalloprotease with predicted endolysin activity in the secreted proteome to be particularly intriguing **(****Fig. 3a****),** as holin/endolysin/spanin clusters with notable differences to classical phage lysis cassettes were very recently established as the so-called type 10 secretion system (T10SS)²⁷. Most information on holin/endolysin/spanin function originates from studies on timed cell lysis by bacteriophages²⁸: upon reaching a critical concentration, holins spontaneously form oligomeric pores in the inner membrane that either translocate a cytoplasmic endolysin to the periplasm or stimulate the refolding of a periplasmic membrane-bound endolysin into an active state. This then degrades the peptidoglycan layer, allowing the spanin complex to fuse the inner and outer membranes and thereby enable viral escape. Analyzing the *Y. entomophaga* genome, we found that the metalloprotease indeed belongs to a T10SS distinct from the YenTc pathogenicity island. It is encoded between a holin and bicomponent spanin **(****Fig. 3d****, inset),** flanked by transcription factors and various housekeeping genes. Studies of its *Serratia marcescens* counterpart indicate^{29,30} that this metalloprotease acts as an L-alanyl D-glutamyl endopeptidase that cleaves peptidoglycan cross-links and therefore indeed represents an endolysin. We termed the endolysin YeEln, the holin YeHln, and the spanins YeIspn and YeOspn, respectively. Our suspicion that we were on the right track was strengthened by the results of an independent *Y. entomophaga* transposon mutagenesis assay³¹, where some hits with reduced protein secretion were associated with an intergenic region close to the gene encoding YeHln. Remarkably, we completely eliminated *Y. entomophaga* protein export when we deleted the T10SS fully **(****Fig. 5****)** as well as its YeHln and YeEln components individually **(****Fig. 3d****),** while finding YeIspn and YeOspn to be less crucial in a laboratory setup that involves extreme shearing forces than spanins in a more natural context³². These results show that this T10SS is responsible for the release of not only YenTc by controlled lysis but also all other toxic proteins we found enriched in the secreted fraction, explaining why so many of them lack a dedicated secretion signal sequence **(****Fig. 4****)** and prompting us to name it the *Yersinia entomophaga* death factor (YenDF).

### Synchronization of YenDF and YenTc production

We next aimed to determine how soldier cells activate production of YenDF, with an OmpR/PhoB-type helix-turn-helix fold protein that we termed YenR encoded directly upstream of YeHln drawing our attention as a potential candidate for controlling YenDF expression. To test this, we replaced its native promoter with arabinose-inducible regulatory elements to create the Ara-YenR strain. In the absence of arabinose, this strain completely stopped secreting and - surprisingly - producing YenTc **(****Fig. 7a****).** Adding arabinose to Ara-YenR massively boosted both YenTc production and protein secretion compared to wild type cells, while the control Ara-YenR ΔYenDF strain generated very high levels of intracellular YenTc that it was unable to secrete **(****Fig. 7a****).**

To validate whether YenR indeed controls both YenDF and YenTc production as these phenotypes suggest, we analysed the cytoplasmic contents of induced Ara-YenR cells by mass spectrometry. Remarkably, our comparison with wild type and non-induced Ara-YenR cells **(****Fig. 7c****-d)** revealed that YenR controls production of not only YenDF components and YenTc, but also the numerous other secreted toxins and virulence factors we identified earlier **(****Fig. 3a****).** This demonstrates that YenTc-expressing soldier cells, rather than yet another specialized cell subpopulation, are the source of this lethal protein cocktail.

We then imaged an induced Ara-YenR YenTc-sfGFP strain, and found that the entire bacterial population converted into secretion-competent soldier cells **(****Fig. 7b****).** This means that YenR is the key to both coupling production of YenTc to the production of its secretion system and generating the soldier cell phenotype.

Interestingly, YenR induction did not generate soldier cells when we used a growth temperature of 37 °C instead of 20 °C **(****Fig. 2d****-h).** This is in line with our previous observation that soldier cells are absent in cell cultures grown at elevated temperatures **(****Fig. 6m, n****),** indicating an additional layer of thermosensitive regulation, and is reminiscent of the thermosensitivity of the *Y. enterocolitica* Tc toxin LysR-type transcriptional regulator³³. This led us to investigate YmoA, a histone-like thermosensitive virulence regulator³⁴ which is affected by YenR induction **(****Fig. 7d****)** and which has been previously proposed to negatively control Tc toxin production³⁵ amongst other proteins³⁶. We removed it by targeted genomic editing but this did not result in viable cells, indicating YmoA is essential for *Y. entomophaga* as also observed for certain *Y. enterocolitica* strains³⁷. Therefore, we produced additional plasmid-borne YmoA as an alternative approach to explore its role. Surprisingly and in contrast to *Y. enterocolitica*³⁵*,* anhydrotetracycline-driven overexpression of YmoA stimulated the expression of YenTc-sfGFP in the entire cell population **(****Fig. 7e****).** This effect does not occur in a YenR-nonexpressing background but rather boosts production of YenR-controlled proteins when YenR is present **(****Fig. 7f****-g**), indicating that YmoA either directly interacts with YenR to drive transcription of soldier cell specific genes or controls expression of proteins that affect this process. While the exact nature of this synergy must be explored further, the instability of YmoA at elevated temperatures^{36,38} provides a tempting explanation for how the soldier cell phenotype is suppressed in such conditions, which are incompatible with insect hosts.

### The YenTc release mechanism

We next investigated if the holin YeHln is needed for the endolysin YeEln to reach the periplasm and which role the pH trigger plays in this process. For this, we expressed plasmid-borne endolysin YeEln in Ara-YenR cells with either all YenDF components knocked out or YeHln intact and monitored pH-dependent protein release. Spanins were not considered in these experiments because they do not play a crucial role for protein secretion in our laboratory setup **(****Fig. 3d****).** Only cells containing both YeHln and YeEln were able to release proteins upon elevation of pH **(****Fig. 8a****),** suggesting that YeEln uses YeHln pores to reach the periplasm similarly to the holin/endolysin/spanin systems of bacteriophages²⁸. A logical conclusion would be that elevation of extracellular pH triggers the formation of holin pores in soldier cells, which is in contrast to the situation in phage infections, where holin pore formation is spontaneously triggered by a local decrease in the proton motive force (PMF) when holins reach a critical allele-timed concentration in the bacterial inner membrane^{39,40,41}. However, increasing external pH can also decrease the PMF between the cytoplasm and periplasm⁴², so that the triggering of YeHln pore formation by elevation of external pH could also be explained by an accompanying decrease in the PMF⁴². This interpretation is also supported by our observation of controlled lysis in Ara-YenR cells under sudden anaerobiotic stress **(****Fig. 8b****),** which causes a loss in PMF due to decrease of cytoplasmic pH⁴³. This means that although the initial triggers are different, the ultimate cause of holin pore formation is likely to be similar in phage holins and YenDF. In order to visualize the individual steps of YenDF-mediated YenTc release from soldier cells following pH triggering, we produced derivatives of the Ara-YenR strain blocked in the pre-secretion state as well as the post-holin, post-endolysin and post-spanin states by knockout of the corresponding YenDF components. We then vitrified the cells by plunge-freezing into liquid ethane. For the first three, we then prepared 50-100 nm thin lamellae ideally suited for cryo-ET using cryo-focused ion beam milling⁴⁴ **(****Fig. 9****),** while the cells in the post-spanin state could be imaged directly. The tomograms revealed that the cell envelope is fully intact in the pre-secretion state **(****Fig. 10a** **and e).** Strikingly, we found many fully assembled YenTc holotoxins composed of all three subunits (TcA, TcB, and TcC) in the cytoplasm **(****Fig. 10a****, inset)** which finally establishes the bacterial cytoplasm as the location of holotoxin assembly for Tc toxins.

After the pH trigger, the cells in the post-holin state demonstrate that YeHln action leads to subtle perturbations of the inner membrane in the form of invaginations **(****Fig. 10b** **and f).** However, we did not observe micron-scale lesions with accompanying expulsion of cytoplasmic material into the periplasm that have been described for the archetypal phage holin S105³⁹. Due to its small size, the YeHln holin cannot be directly visualized in the tomograms. Therefore, we used confocal microscopy to determine the distribution of fluorescently tagged YeHln before and after pH triggering. We observed that in both cases YeHln distributes throughout the cell surface in nearly ubiquitous small raft-like foci **(****Fig. 8c****).**

Next, we analyzed the tomograms of the cells in the post-endolysin state to understand the cellular effects of the enzymatic action of YeEln. The inner membrane of these cells is largely detached from the peptidoglycan layer and outer membrane, resulting in major inward invaginations of the inner membrane particularly near the cell poles **(****Fig. 10c** **and g).** In a spanin-free situation, the action of YeEln eventually leads to such significant weakening of the peptidoglycan sacculus that internal osmotic pressure⁴⁵ drives the cell to expand into a spheroplast **(Fig. 11a-b).** Our tomograms show that these spheroplasts have significantly lower protein density compared to pre-spheroplast cells due to their larger volume, making them a highly attractive target for future *in situ* structural proteomics studies **(Fig. 11c).**

When the spanins YenIspn and YenOspn are however present, the bacterium undergoes a dramatic transformation that converts it into a loosely bound cluster of membrane vesicles **(****Fig. 10d** **and h).** Membranes in areas with cell envelope-spanning protein complexes stay discrete after this secretion step **(****Fig. 10d**, **left inset)** and the cell wall of the vesicles appears intact, indicating that this process does not disrupt the lipoprotein Lpp tether of peptidoglycan to the former outer membrane **(****Fig. 10d****).** Importantly, this spanin-driven metamorphosis releases YenTc holotoxins **(****Fig. 10d**, **right and bottom insets,** **Fig. 12****),** indicating that the spanins not only trigger the fusion of the outer and inner membrane of soldier cells, leading to formation of unimembrane vesicles, but that this process also releases the cytoplasmic contents into the external environment.

### Discussion

Based on our observations, we propose the following model for soldier cell differentiation and subsequent YenDF-mediated protein release. In a small percentage of isogenic cells, thermosensing combined with nutrient sensing, quorum sensing and further unknown factors cause an imbalance in the default levels of YenR or YmoA **(****Fig. 13a****).** This enables them to drive transcription of soldier cell genes, resulting in the production of the YenDF components YeHln, YeEln, YeIspn and YeOspn, as well as a cytoplasmic cocktail of toxins including YenTc in a fully assembled holotoxin state **(****Fig. 13b****).** Upon an environmentally pH-triggered decrease of the PMF, small oligomeric holin YeHln rafts in the inner membrane open pores sufficiently large to translocate the 15 kDa cytoplasmic endolysin YeEln into the periplasm **(****Fig. 13c****),** where it cleaves peptidoglycan cross-links. Once YeEln has modified enough of the peptidoglycan sacculus that this no longer sterically hinders the spanins³², the inner membrane anchored spanin YenIspn and the outer membrane anchored spanin YenOspn fold towards each other and ultimately fuse these membranes **(****Fig. 13d****),** which remain bound to the peptidoglycan layer by tethering Lpp proteins. The accompanying release of inner osmotic pressure⁴⁵ fires the cocktail of soldier cell toxins including YenTc into the external environment **(****Fig. 13e****),** where they can proceed to locate and act on their cellular targets.

In this study, we identify the type 10 secretion system YenDF as the machinery used by the Gram-negative insect pathogen *Y. entomophaga* to export the Tc toxin YenTc and numerous other virulence factors into the surrounding environment. This is the first example of anti-eukaryotic toxins using this type of novel secretion system²⁷, which is particularly apt for this case since the nearly ribosome-sized YenTc is fully assembled in the cytoplasm and would not fit through any of the previously described toxin secretion systems. These findings therefore establish a new avenue for export of toxins and other virulence factors previously not associated with any known secretion system, with virtually no upper or lower size limitation.

Since cells pay the ultimate price for exporting proteins via YenDF, namely death, only a small specialized subset of what we term soldier cells employ it at any given time. How the decision to differentiate into a soldier cell is made has not yet been determined, although nutrient and quorum sensing play an important role and temperature sensing is crucial **(****Figs. 2** **and** **6****).** We discovered that the transcription factor YenR serves as the central switch of differentiation, ensuring that the production of numerous toxins and their secretion system is synchronized **(Fig. 7h).** The net result is a *Y. entomophaga* population that demonstrates traits such as differentiation and altruism reminiscent of eusocial systems, a far cry from the "bags of enzymes" that bacteria were once considered to be⁴⁶. From the perspective of costs and benefits to the entire bacterial population, it is rational that its few soldier cells are sacrificed to release as many host damaging factors as possible. This not only explains why soldier cells produce such a variety of virulence factors and toxins, but also why they tend to grow much larger than their non-differentiated isogenic brethren **(****Figs. 3c** **and** **7b****):** this increases the total toxin carrying capacity of each specialized cell.

This also means that in a natural setting, production of YenTc and its secretion machinery is likely initiated only upon ingestion in response to the presence of host nutrients **(****Fig. 6****),** a situation mimicked by complex growth media. Since vomiting clears a significant portion of invading bacteria from the acidic anterior midgut²⁶, having a pH sensitive secretion mechanism helps prevent release of the toxic cocktail from soldier cells until they have reached the alkaline posterior midgut, their major theater of operations. At this point, chitinases and chitin-binding proteins of the cocktail would breach the chitinous peritrophic membrane⁴⁷ to enable access of YenTc and the other released toxins to the underlying midgut epithelial layer, leading to its complete dissolution and successful colonization of the insect²⁶ by the non-differentiated *Y. entomophaga* population.

*Y. entomophaga* serves as an excellent proxy for dangerous human pathogens that employ Tc toxins. Analysis of Tc toxin operons from e.g. *Yersinia pseudotuberculosis, Salmonella enterica* subsp. *houtenae, Yersinia enterocolitica,* and *Yersinia pestis* show that they also encode T10SSs similar to YenDF **(****Fig. 14a****-d),** indicating that these bacteria employ the same secretion mechanism as *Y. entomophaga.* Although the presence of a T10SS in *Y. enterocolitica* has been noticed before⁴⁸, its biological relevance has now been preliminarily validated by demonstrating that this pathogen loses its ability to establish an infection in insects when left without a T10SS⁴⁹. Given that our reconstruction of the ancient *Y. pestis* Tc toxin operon from a Black Death victim⁵⁰ shows that it also encodes a T10SS **(****Fig. 14d****, inset),** this raises questions to its role in the deadliest pandemic of human history⁵¹.

The T10SS-mediated release of Tc toxins also fits well into the emerging paradigm of toxin export by phage-derived proteins. Notable examples include the secretion of chitinolytic proteins by a *Serratia marcescens* T10SS²⁹, release of the *Clostridium difficile* toxins A and B via a holin and endolysin⁵²⁻⁵⁴, the holin-dependent export of large clostridial glucosylating toxins^{55,56} and the endolysin-dependent secretion of typhoid toxin⁵⁷. The potential use of soldier cell subpopulations also by these pathogens is a fascinating possibility.

Finally, combining the observation that YenTc deletion causes full loss of *Y. entomophaga* virulence²³ with our discovery that Tc toxins are produced by only few cells in a population leads to the startling insight that pathogen virulence can be determined by a small number of specialized soldier cells. If this is indeed found to be a more widespread phenomenon, then medical interventions that specifically target such specialized cells may represent a promising treatment strategy for many bacterial diseases.

### Example 2: Production of insecticidal toxin mix and high throughput purification of YenTc toxin using Ara-YenR cells

In addition to the increased production of toxins by the Ara-YenR strain as described above, we have also determined that the extremely dense growth of these bacteria in SOC media causes acidification that prevents release of the toxin mix until the pH is raised, for example by resuspending in a small volume of pH 8.0 buffer. During an incubation time as brief as 15 minutes, the Ara-YenR cells release the toxin mix directly into the buffer, with no laborious cell lysis steps required. The cell debris can then be spun down and the supernatant filtered using a 0.22 µm filter, to obtain a highly concentrated and ready-to-use mix of insecticidal toxins. Alternatively, if secretion is not immediately desired, the bacteria can be stored in pH 5.5 buffer until later use.

Furthermore, if a particular insecticidal toxin of interest is needed rather than a more complex mixture, targeted genomic editing can be applied to the Ara-YenR strain to fuse an in-frame affinity tag to the toxin, which can then be purified in a high-throughput manner. We demonstrated the validity of this approach by adding a C-terminal His-tag to the YenA2 subunit of the orally active YenTc toxin, and purifying the latter in a single step using Ni²⁺-NTA beads from the toxin mix obtained from Ara-YenR cells in the manner described above **(****Fig. 15a****).** Size exclusion chromatography and negative stain electron microscopy **(****Fig. 15b****-c)** confirm the purified YenTc is a homogeneous, well-folded complex. Using this purification strategy, 270 mg of pure YenTc was obtained from 1 liter of Ara-YenR cells (compared to 21 mg using a strain with unmodified YenR, **Fig. 15d****).** This demonstrates how the approaches described above can be used synergistically to produce individually purified insecticidal toxins, which can then be applied to crops for pest control.

### Example 3: Y. entomophaga toxin composition does not show toxicity towards human cells

50 mL of *Y. entomophaga* cells were grown overnight in SOC media and harvested by centrifugation at 4000g. Secretion was induced by resuspending the cells in 100 mM Tris-HCl pH 8.0, 100 mM NaCl buffer and incubating for 20 minutes. The cells were then removed by centrifugation and the supernatant containing toxin mixture was filtered through a 0.22 µm filter. The resulting protein concentration in the supernatant was determined to be 10.0 mg/mL, and 10 µL were used for SDS-PAGE analysis. Then, 2.5%, 5.0%, or 10.0% volume:volume of this toxin mixture was added to wells containing adherent human-derived HEK293T cells, resulting in final toxin mixture concentrations of 0.25 mg/mL, 0.50 mg/mL and 1.0 mg/mL respectively. As a control, a 10.0% volume:volume ratio of 100 mM Tris-HCl pH 8.0, 100 mM NaCl buffer was added. All cell experiments were done in triplicate.

As shown in **Fig. 16****,** the toxin mixture secreted by *Y. entomophaga* (gel image) is not harmful to cultured human cells even at high concentration (cell micrographs).

### Methods of Examples 1 and 2

### Bacterial strains and constructs

*Y. entomophaga* strains and plasmids used in this study are provided in **Table 1.**

**Table 1a: Y. entomophaga strains used in this study**

| ***Y. entomophaga* strain** | **Description** | **Source** |
|---|---|---|
| MH96 | Wild type strain used as the basis for all other strains listed here | German Collection of Microorganisms and Cell Cultures GmbH (DSMZ) |
| YenTc-sfGFP | YenA1 (37- to sfGFP) fusion | This work |
| ΔYeHln | YeHln to CmR | This work |
| ΔYeEln | YeEln to CmR | This work |
| ΔYeIspn/YeOspn | YeIspn and YeOspn to CmR | This work |
| ΔYenDF | YeHln, YeEln, YeIspn and YeOspn to CmR | This work |
| Ara-YenR | araC and AraBAD promoter inserted directly before YenR | This work |
| Ara-YenR ΔYenDF | Ara-YenR and ΔYenDF combination strain | This work |
| Ara-YenR ΔYeHln | Ara-YenR and ΔYeHln combination strain | This work |
| Ara-YenR ΔYeEln | Ara-YenR and ΔYeEln combination strain | This work |
| Ara-YenR ΔYeIspn/YeOspn | Ara-YenR and ΔYeIspn/YeOspn combination strain | This work |
| Ara-YenR YeHln-mCherry ΔYeEln/YeIspn/YeOspn | Ara-YenR combination strain with YeHln an mCherry fusion after a 30 aa linker, plus ΔYeEln/YeIspn/YeOspn | This work |
| Ara-YenR YenAl-sfGFP | Ara-YenR and YenAl-sfGFP combination strain | This work |
| ΔTat | PL78_07045 - PL78_07060 to CmR | This work |
| ΔT1SS | PL78_09880 to CmR | This work |
| ΔT2SS | PL78_08935 - PL78_08965 to CmR | This work |
| ΔT3SS #1 | PL78_14535 - PL78_14625 to CmR | This work |
| ΔT3SS #2 | PL78 18085 - PL78_18230 to CmR | This work |
| ΔT6SS | PL78_00895 - PL78_01045 to CmR | This work |
| ΔGshB | PL78_09425 to CmR | This work |
| ΔOxyR | PL78_07700 to CmR | This work |
| ΔLipase #1 | PL78_09600 to CmR | This work |
| ΔLipase #2 | PL78_18430 to CmR | This work |
| ΔLipase #3 | PL78_18000 to CmR | This work |
| ΔLipase #4 | PL78_00825 to CmR | This work |
| ΔYenTc | PL78 03740 - PL78 03770 to CmR | This work |
| YenTc-sfGFP ΔAI1 | YenTc-sfGFP combination strain with PL78_03850 to TcR | This work |
| YenTc-sfGFP ΔAI2 | YenTc-sfGFP combination strain with PL78_14700 to TcR | This work |
| YenTc-sfGFP ΔAI3 | YenTc-sfGFP combination strain with PL78_07960 to TcR | This work |

**Table 1b: Plasmids used in this study**

| **Plasmid** | **Description** | **Source** |
|---|---|---|
| aTc-YmoA | Anhydrotetracyline-inducible YmoA | This work |
| Ara-YeEln | Arabinose-inducible YeEln | This work |
| pMultiEdit-v4 | Helper plasmid encoding arabinose-inducible λ-RED proteins, IPTG-inducible I-SceI restrictase, rhamnose-inducible Cas9 and constitutively expressed anti-N20 gRNA. The N20 is a sequence that has minimal off-target specificity in *Y. entomophaga.* | This work |
| pMultiDonor | SacB-containing donor backbone plasmid for targeted genomic editing of regions of interest. | This work |

### Cell growth and secretion assay conditions

*Y. entomophaga* strain MH96⁵⁸ was obtained from the German Collection of Microorganisms and Cell Cultures GmbH (DSMZ). To test secretion in different growth media, 10 mL of these were inoculated with 500 µL exponential phase culture grown in LB, then grown for 16 h at 16 °C. The media was separated from the cells by 5 min of centrifugation at 4000*g* and 0.22 µm filtration. Amicon concentrators (Merck) were used for concentrating the media to 250 µL. All samples were then normalized with 1× PBS to a volume equivalent to a cell OD₆₀₀ of 4.0 and analyzed by stain-free SDS-PAGE. For secretion assays, 20 mL cells were grown in SOC growth medium for 16 h at 20 °C. Then the pH was elevated either by drop-wise addition of NaOH / a fixed volume of 1 M Tris-HCl pH 8.0, or resuspension of spun-down cells in 100 mM Tris-HCl pH 8.0, 100 mM NaCl buffer. Ara-YenR containing strains and the Ara-YeEln plasmid were induced by addition of 0.5% L-arabinose during inoculation, while the aTc-YmoA plasmid was induced by addition of 200 ng/mL anhydrotetracycline to OD₆₀₀ 0.5 cells. For anaerobiosis-induced secretion, a 50 mL induced and acidified Ara-YenR cell culture was left stationary for 20 minutes after vigorously shaking during growth.

### Plasmid construction

To construct donor plasmids, the region from the plasmid origin until the post-SacB sequence of plasmid pCM433kanT (Addgene #61262) was PCR amplified and used as a donor plasmid backbone. I-SceI restrictase cleavage sites were encoded by the amplification primers and used to add this feature to the 5' and 3' ends of the backbone. An antibiotic resistance marker, typically chloramphenicol, was PCR amplified from any chloramphenicol-resistant plasmid. ~300 bp homology regions (HR) to the 5' and 3' of the targeted genes were PCR amplified from *Y. entomophaga* cells. If CRISPR-mediated excision for a scarless genomic edit was desired, the 20 bp Cas9-gRNA targeting region with minimal off-target specificity in *Y. entomophaga,* followed by the final 40 bp of the 5' HR sequence, followed by any features to be retained after CRISPR-mediated excision (such as a His-tag) were encoded on the reverse primer used to amplify the antibiotic resistance marker. Donor plasmids were then assembled from these four fragments using the Gibson Assembly Master Mix (New England Biolabs) with subsequent transformation into NEB 10-beta *E. coli* cells for amplification. To construct the editing plasmid, the following fragments were combined using the Gibson Assembly Master Mix: the fragment from the plasmid origin until the antibiotic resistance marker of pREDTAI (Addgene #51627), the rhaR-rhaS-rhaB encoding fragment from pLEMO, the antibiotic resistance marker-araBAD-araC-Cas9 encoding fragment of pCAGO with the 20 bp Cas9-gRNA targeting region that has minimal off-target specificity in *Y. entomophaga* inserted before the gRNA scaffold sequence, and a kanamycin antibiotic resistance marker from any kanamycin-resistant plasmid. Additionally, the anhydrotetracycline-inducible aTc-YmoA plasmid for YmoA expression was constructed in several steps using the Gibson Assembly Master Mix. An intermediate plasmid was made from a fragment of pET28a spanning from the T7 terminator until before the post-rop sequence of the lacI gene, a fragment from a pre-made intermediate plasmid encoding (from 5' to 3') a chloramphenicol resistance marker, a gentamicin resistance marker, an araC gene and an araBAD promoter with subsequent ribosome binding site, and a fragment containing the YmoA sequence from *Y. entomophaga* cells. This intermediate plasmid was then amplified from YmoA until the region between the beginning of the gentamicin resistance marker and the end of araC, and then assembled using the Gibson Assembly Master Mix with a fragment containing the tetR gene and a tetr/tetA promoter from any suitable plasmid.

### Targeted genomic editing of Y. entomophaga

Cells were initially electroporated **(****Figure 1b****)** with a low-copy kanamycin-resistant editing plasmid encoding arabinose-inducible λ-RED recombineering proteins, an IPTG-inducible I-SceI endonuclease, as well as a rhamnose-inducible Cas9 protein plus a constitutively expressed gRNA that has minimal off-target specificity in this species as determined by Cas-OFFinder⁵⁹. The transformed cells were grown on LB agar plates containing 50 µg/mL kanamycin overnight at 30 °C and then in SOC media containing 50 µg/mL kanamycin overnight at 30 °C with shaking. Cells were then transformed by electroporation with a typically chloramphenicol-resistant donor plasmid encoding an I-SceI cleavable fragment containing (in 5' to 3' order) a ~300 bp sequence homologous to the 5'of the targeted area, an antibiotic resistance marker, (optionally) a 20 bp Cas9-gRNA targeting region, (optionally) the last 40 bp of the 3' sequence from the 5' homology region, and a ~300 bp sequence homologous to the 3'of the targeted area. The donor plasmid backbone contains a high copy number plasmid origin and a SacB counterselection marker. This setup allows modification of targeted genomic areas with optional subsequent Cas9-mediated excision of the antibiotic resistance marker⁶⁰, facilitating multiple sequential targeted genomic edits. The cells were then plated onto LB agar plates containing antibiotics for selection of the editing plasmid antibiotic resistance marker (50 µg/mL kanamycin), the donor cassette antibiotic resistance marker (25 µg/mL chloramphenicol), 2% glucose and grown overnight at 30 °C. A picked colony was then used to inoculate 1 mL of antibiotic-free LB media containing 1.5% L-arabinose and 1 mM IPTG for λ-RED and I-SceI induction, respectively, which was grown overnight at 30 °C with shaking. 100 µL of the resulting culture was then plated on LB agar plates containing 50 µg kanamycin, 25 µg/mL chloramphenicol and 10% sucrose as a counterselection agent, and grown overnight at 30 °C. Several surviving colonies were then re-streaked onto an identical plate to eliminate background colonies, and the success of the genomic editing was validated by colony PCR and sequencing. If CRISPR-mediated excision of an antibiotic resistance marker was desired, a validated colony was used to inoculate 1 mL of LB containing 50 µg/mL kanamycin to maintain selection for the editing plasmid, and 0.2% L-rhamnose to induce Cas9 expression. After growth at 30 °C with shaking for 7 hours, cells were spun down, resuspended in 300 µL LB and plated onto an LB agar plate containing 50 µg/mL kanamycin, which was then grown overnight at 30 °C. The success of the CRISPR-mediated excision was then validated by colony PCR and sequencing.

In order to make the Ara-YenR strain, a non-excisible fragment containing a chloramphenicol resistance marker, araBAD promoter and a ribosome binding site was inserted directly before YenR in the *Y. entomophaga* genome, enabling expression of this gene to be switched on or off in the presence or absence of 0.5% L-arabinose. In order to make the Ara-YenR YenA2-His strain, an excisible fragment was used to scarlessly introduce an 8×His tag to the C-terminus of the YenA2 subunit. The YenR promoter region of this strain was then modified in the same way as the Ara-YenR strain. In order to make the Ara-YenR ΔYenDF YenA2-His strain, the scarless YenA2-His strain of *Y. entomophaga* was modified in the same manner as the Ara-YenR strain, however using the Ara-YenR ΔYenDF donor plasmid instead.

### Non-cryogenic cell imaging

For routine phase contrast and fluorescent imaging, 1 µL of *Y. entomophaga* strain cultures were imaged on a glass slide at 20x using an EVOS M7000 microscope (Thermo Fisher Scientific). Confocal fluorescence microscopy of Ara-YenR YeHln-mCherry Δ YeEln/YeIspn/YeOspn cells before and 30 min after pH 8.0 induced secretion triggering was done on a glass slide at 40x using a LSM800 microscope (Zeiss) equipped with an Airyscan detector module (Zeiss).

### Fluorescence spectroscopy

For measuring the effect of alternate versus simultaneous expression of YenR and YmoA on YeHln production, 3 biological replicates of Ara-YenR YeHln-mCherry ΔYeEln/YeIspn/YeOspn cells transformed with an aTc-YmoA plasmid were induced with either 200 ng/mL anhydrotetracycline, 0.5% L-arabinose, or both of them. Along with an uninduced control, these were harvested and resuspended to OD₆₀₀ 5.70 in 1× PBS. Fluorescence emission spectra were recorded on a Spark spectrophotometer (Tecan) using an excitation wavelength of 556 nm and emission wavelength of 610 nm in a 2 × 2 read mode. The acquired data were then processed using Prism 9 (GraphPad).

For measuring the effect of different environmental conditions and quorum system knockouts on YenTc production, 3 biological replicates of YenTc-sfGFP cells with or without genomic knockouts of the acyl-homoserine-lactone synthase (for autoinducer-1 KO), the S-ribosylhomocysteine lyase (for autoinducer-2 KO), and the L-threonine 3-dehydrogenase (for autoinducer-3 KO) were grown in the conditions specified in **Fig. 6****.** These were harvested, resuspended to OD₆₀₀ 1.0 in 1× PBS, and used to measure the pre-secretion content of cellular YenTc-sfGFP. After 20 minutes incubation time, the cells were spun down and the supernatant was used to measure the content of secreted YenTc-sfGFP. Fluorescence emission spectra were recorded on a Spark spectrophotometer (Tecan) using an excitation wavelength of 470 nm and emission wavelength of 518 nm in a 2 × 2 read mode.

### Proteomic analysis using NanoHPLC-MS/MS

Biological triplicates of either the secreted protein fractions or the pre-secretion cytoplasmic contents from cells normalized to OD₆₀₀ 4.0 were briefly run on a stain-free SDS-PAGE gel (Bio-Rad). After tryptic digestion and purification, the protein fragments were analyzed by nano-HPLC-MS/MS by using an Ultimate^{™} 3000 RSLC nano-HPLC system and a Hybrid-Orbitrap mass spectrometer (Q Exactive^{™} Plus) equipped with a nano-spray source (all from ThermoFisher Scientific). Briefly, the lyophilized tryptic peptides were suspended in 20 µL 0.1% TFA, and 3 µL of the samples were injected onto and enriched on a C18 PepMap 100 column (5 µm, 100 Å, 300 µm ID ^{∗} 5 mm, Dionex, Germany) using 0.1% TFA, at a flow rate of 30 µL/min, for 5 min. Subsequently, the peptides were separated on a C18 PepMap 100 column (3 µm, 100 Å, 75 µm ID ^{∗} 50 cm) using a linear gradient, starting with 95% solvent A/5% solvent B and increasing to 30.0% solvent B in 90 min using a flow rate of 300 nL/min followed by washing and re-equilibration of the column (solvent A: water containing 0.1% formic acid; solvent B: acetonitrile containing 0.1% formic acid). The nano-HPLC apparatus was coupled online with the mass spectrometer using a standard coated Pico Tip emitter (ID 20 µm, Tip-ID 10 µM, New Objective, Woburn, MA, USA). Signals in the mass range of m/z 300 to 1650 were acquired at a resolution of 70,000 for full scan, followed by up to ten high-energy collision-dissociation (HCD) MS/MS scans of the most intense at least doubly charged ions at a resolution of 17,500.

Relative protein quantification was performed by using MaxQuant⁶¹ v.2.0.3.1, including the Andromeda search algorithm and searching the *Y. entomophaga* proteome of the UniProt database (downloaded Jan 2022). Briefly, an MS/MS ion search was performed for enzymatic trypsin cleavage, allowing two missed cleavages. Carbamidomethylation was set as a fixed protein modification, and oxidation of methionine and acetylation of the N-terminus were set as variable modifications. The mass accuracy was set to 20 ppm for the first search, and to 4.5 ppm for the second search. The false discovery rates for peptide and protein identification were set to 0.01. Only proteins for which at least two peptides were quantified were chosen for further validation. Relative quantification of proteins was performed by using the label-free quantification algorithm implemented in MaxQuant and the match-between-runs feature was activated.

Statistical data analysis of samples was performed using Perseus⁶² v.1.6.14.0. Label-free quantification (LFQ) intensities were log-transformed (log2); replicate samples were grouped together. Proteins had to be quantified at least three times in at least one of the groups of a comparison to be retained for further analysis. Missing values were imputed using small normal distributed values (width 0.3, down shift 1.8 for the datasets involving the Ara-YenR strain; width 0.3, down shift 2.0 for the dataset assessing the secreted vs non-secreted protein fractions), and a two-sided *t*-test (significance threshold: -log2 fold change > 1.5 for both datasets; p-value < 0.02 for the datasets involving the Ara-YenR strain; p-value < 0.05 for the dataset assessing the secreted vs non-secreted protein fractions) was performed. Proteins that were statistically significant outliers were considered as hits. Volcano plots were generated using VolcaNoseR⁶³.

UniProt accession numbers (parenthesized) of statistically significant hits according to the above criteria, which are of major biological importance to this study are as follows: YenA1 (B6A877), YenA2 (B6A878), Chi1 (B6A876), Chi2 (B6A879), YenB (B6A880), YenC1 (B6A881), YenC2 (B6A882), RHS2 (A0A2D0TC51), Cbp (A0A3S6EXR6), PirA (A0A3S6F007), PirB (A0A3S6F043), PiI36 (A0A3S6F569), NucA (A0A3S6F4M5), Chi3 (A0A3S6F1Q8), StcE (A0A3 S6EYX4), Tlh (A0A3S6F052), YeEln (A0A3 S6F4L4), YenIspn (A0A3S6F4Q6), YenR (A0A3S6F5G2), YmoA (A0A3S6EV06).

### Bioinformatic analyses

Identification of secretion signal sequences for YenR-controlled toxins and virulence factors was performed using SignalP 6.0⁶⁴. For reconstruction of the YpeTc operon of ancient *Y. pestis* from a Black Death victim, genomic reads from Illumina run SRR341961 of the original study⁵⁰ were assembled into contigs using Ray Meta⁶⁵, which were in turn assembled into a scaffold using CSAR-Web⁶⁶ with the YpeTc operon of *Y. pestis* strain KIM10+ as a reference.

### Bacterial vitrification

Overnight cultures of *Y. entomophaga* strains were spun down for 4 min at 4000*g* and resuspended to an OD₆₀₀ of 20 in 1× PBS buffer containing pre-washed 10 nm BSA-NanoGold tracer (Aurion). In case of the AraYenR strain, 10 µL was directly applied to glow-discharged Quantifoil R2/1 Au-SiO₂ 200 grids and incubated for 10 minutes in a Vitrobot Mark IV plunger (Thermo Fisher Scientific) set to 100% humidity and 22 °C prior to blotting and plunge-freezing. 3 µL of the other three strains were applied to identically treated grids in identical plunger conditions following 1 h incubation in the 1× PBS-NanoGold buffer. After a waiting time of 60 s, all grids were blotted from both sides for 32 s using blot force 5. After 0.5 s drain time, the grids were vitrified by plunge-freezing into liquid ethane. Grids containing Ara-YenR strain cells were clipped with standard AutoGrids (Thermo Fisher Scientific) and directly used for further data acquisition as the cells were thin enough without additional cryo-FIB milling.

### Lamella preparation by cryo-FIB milling

Grids containing Ara-YenR ΔYenDF, Ara-YenR ΔYenEln/YeIspn/YeOspn, or Ara-YenR ΔYeIspn/YeOspn strain cells were clipped in cryo-FIB-specific AutoGrids (Thermo Fisher Scientific) with alignment markers and a cut-out for milling at shallow angles. Clipped grids were transferred to an Aquilos 2 cryo-FIB/SEM dual beam microscope (Thermo Fisher Scientific). Lamella preparation was performed as previously described⁶⁷. Briefly, after platinum sputter coating and deposition of metalloorganic platinum, clusters of bacteria cells were targeted for a four-step milling procedure using decreasing ion beam currents from 0.5 nA to 50 µA. Milling angles of 6-10° relative to the grid were used. Lamellae were milled to a thickness range of 50-100 nm.

### Cryo-ET data acquisition

Once ready for imaging, all grids were transferred into a Titan Krios transmission electron microscope (Thermo Fisher Scientific) operated at 300 kV, equipped with a K3 camera and a BioQuantum energy filter (Gatan). Images were acquired with SerialEM⁶⁸. Overview images were acquired at 6500x nominal magnification to identify regions for cryo-ET data acquisition at higher magnification. Images used as references for batch data acquisition were also acquired at this magnification. Tilt series were acquired at 64,000x (pixel size 1.48Å) for milled Ara-YenR ΔYenDF, Ara-YenR ΔYenEln/YeIspn/YeOspn, and Ara-YenR ΔYeIspn/YeOspn cells and at 42,000x (pixel size 2.32 Å) for Ara-YenR cells using a script based on a dose-symmetric tilt scheme⁶⁹ at defoci ranging from -5 to -8 µm. The stage was tilted from -60 to +60 relative to the lamella plane at 3° increments. Each tilt series was exposed to a total dose of 120-140 e⁻/Å².

### Tomogram reconstruction and sub-tomogram averaging of YenTc

Acquired movie frames were motion-corrected and combined into stacks of tilt series using Warp⁷⁰. The stacks were aligned and reconstructed using IMOD⁷¹. During alignment, patch tracking was used in tilt series of milled cells and fiducial-marker tracking was used in tilt series of non-milled cells. The tomograms were 4x binned and low-pass filtered to 60 Å or 100 Å for better visualization using EMAN2⁷². Alternatively, denoising by an implementation of cryo-CARE⁷³ was used for the same purpose. To obtain a structure of YenTc, 167 particles were manually picked from 12 tomograms of Ara-YenR cells. The sub-tomograms were extracted from 4x binned tomograms with a box size of 100 pixels (928 Å) using RELION 3.0⁷⁴. The sub-tomograms were aligned to a spherical reference and averaged over iterations with C5 symmetry in RELION.

### Tomogram annotation and figure production

In order to visualize the morphological changes accompanying YenDF component action as well as the position and assembly status of YenTc, densities of YenTc, the inner membrane, peptidoglycan layer, outer membrane and fused membranes (in AraYenR tomograms) as well as exemplary cell envelope-spanning proteins (in AraYenR tomograms) were annotated on a slice-to-slice basis in reconstructed tomograms using Dragonfly (Object Research Systems). Weaker densities were masked during this process, which culminated in generation of 3D segmented volumes.

ChimeraX⁷⁵ was used for **Fig. 12b****-f.** **Fig. 13a** and **Fig. 1** were created using Biorender.com.

### Production and purification of YenTc from Ara-YenR cells

*Y. entomophaga* strains containing a scarlessly inserted 8x His-tag at the C-terminus of YenA2 either in presence or absence of the Ara-YenR genomic modification were used for high-yield production and purification of YenTc. For this, bacterial cultures were grown in SOC media containing 0.5% L-arabinose until OD₆₀₀=18.0 at 18 °C while shaking at 180 rpm. The cells were then spun down by 10 min of centrifugation at 4000*g* and resuspended in a volume of 120 mL 100 mM Tris-HCl pH 8.0, 100 mM NaCl buffer to induce secretion. Non-secreted material was then removed by 10 min of centrifugation at 4000*g* and subsequent 0.22 µm filtration. 25 mM imidazole pre-adjusted to pH 8.0 was then added and the sample was loaded onto one or multiple 5 mL pre-packed HisTrap HP immobilized metal affinity chromatography columns (Cytiva) via peristaltic pump. After a 10-column volume wash with 20 mM Tris-HCl pH 8.0, 150 mM NaCl buffer containing 25 mM imidazole, the column-bound protein was eluted with a 6-column volume gradient with the final gradient point being the same buffer containing 500 mM imidazole and YenTc purity was confirmed by SDS-PAGE. For optional additional purification, the eluted protein was concentrated to ~50 mg/mL and injected into a Superose 6 Increase size-exclusion chromatography column (Cytiva) run with 20 mM Hepes pH 7.5, 150 mM NaCl buffer. A fraction of the monodisperse elution peak was diluted to 0.01 mg/mL and analyzed by negative stain electron microscopy using a Tecnai Spirit electron microscope (FEI) operated at 120 kV and equipped with a F416 detector (TVIPS).

### Generation of modified strains

The following plasmids have been generated according to the above-described methods: SEQ ID NO: 11 encoding an editing plasmid encoding λ-RED recombineering proteins, an IPTG-inducible I-SceI endonuclease, a rhamnose-inducible Cas9 protein and a constitutively expressed gRNA that has minimal off-target specificity in *Y. entomophaga.*

SEQ ID NO: 12 encodes a YenA2-His donor plasmid with an excision option.

SEQ ID NO: 13 encodes a YenA2-His donor plasmid without an excision option.

SEQ ID NO: 14 encodes an Ara-YenR donor plasmid without an excision option.

SEQ ID NO: 3 encodes an aTc-YmoA expression plasmid.

SEQ ID NO: 15 encodes an Ara-YenR ΔYenDF donor plasmid without an excision option.

The above plasmids have been used to generate the genetically modified strains of the invention. In particular, transformation of *Y entomophaga strain MH96* with SEQ ID NO: 11 (editing plasmid) and SEQ ID NO: 14 (donor plasmid) resulted in the L-arabinose inducible Ara-YenR strain after carrying out the genomic editing and selection steps described in "Targeted genomic editing of *Y. entomophaga".*

Transformation of the *Y entomophaga* Ara-YenR strain with SEQ ID NO: 3 resulted in the L-arabinose and anhydrotetracycline inducible *Y. entomophaga* Ara-YenR strain + YmoA plasmid.

Transformation of *Y entomophaga strain MH96* with SEQ ID NO: 11 (editing plasmid) and SEQ ID NO: 15 (donor plasmid) resulted in the L-arabinose inducible *Y. entomophaga* Ara-YenR ΔYenDF strain after carrying out the genomic editing and selection steps described in "Targeted genomic editing of *Y. entomophaga".*

Transformation of *Y. entomophaga strain MH96* with SEQ ID NO: 11 (editing plasmid) and SEQ ID NO: 13 (donor plasmid) resulted in the *Y. entomophaga* YenA2-His chloramphenicol resistant strain after carrying out the genomic editing and selection steps described in "Targeted genomic editing of *Y entomophaga".*

Transformation of *Y entomophaga strain MH96* with SEQ ID NO: 11 (editing plasmid) and SEQ ID NO: 12 (donor plasmid) resulted in the *Y. entomophaga* YenA2-His strain after carrying out the genomic editing and selection steps described in "Targeted genomic editing of *Y. entomophaga",* including the CRISPR-mediated excision step of the chloramphenicol resistance marker.

Transformation of *Y. entomophaga* YenA2-His strain with SEQ ID NO: 14 (donor plasmid) resulted in the Ara-YenR + YenA2-His strain after carrying out the genomic editing and selection steps described in "Targeted genomic editing of *Y. entomophaga".Transformation* of *Y. entomophaga* YenA2-His strain with SEQ ID NO: 15 (donor plasmid) resulted in the Ara-YenR ΔYenDF + YenA2-His strain after carrying out the genomic editing and selection steps described in "Targeted genomic editing of *Y. entomophaga".*

### List of references:

1 Green, E. R. & Mecsas, J. Bacterial Secretion Systems: An Overview. Microbiol Spectr 4, doi:10.1128/microbiolspec.VMBF-0012-2015 (2016).
2 Macion, A., Wyszynska, A. & Godlewska, R. Delivery of Toxins and Effectors by Bacterial Membrane Vesicles. Toxins (Basel) 13, doi: 10.3390/toxins13120845 (2021).
3 Bumba, L. et al. Calcium-Driven Folding of RTX Domain beta-Rolls Ratchets Translocation of RTX Proteins through Type I Secretion Ducts. Mol Cell 62, 47-62, doi:10.1016/j.molcel.2016.03.018 (2016).
4 Roderer, D. & Raunser, S. Tc Toxin Complexes: Assembly, Membrane Permeation, and Protein Translocation. Annu Rev Microbiol 73, 247-265, doi: 10.1146/annurev-micro-102215-095531 (2019).
5 McQuade, R. & Stock, S. P. Secretion Systems and Secreted Proteins in Gram-Negative Entomopathogenic Bacteria: Their Roles in Insect Virulence and Beyond. Insects 9, doi: 10.3390/insects9020068 (2018).
6 Blackburn, M., Golubeva, E., Bowen, D. & Ffrench-Constant, R. H. A novel insecticidal toxin from photorhabdus luminescens, toxin complex a (Tca), and its histopathological effects on the midgut of manduca sexta. Appl Environ Microbiol 64, 3036-3041, doi:10.1128/AEM.64.8.3036-3041.1998 (1998).
7 Song, N. et al. Genome-wide dissection reveals diverse pathogenic roles of bacterial Tc toxins. PLoS Pathog 17, e1009102, doi:10.1371/journal.ppat. 1009102 (2021).
8 Roderer, D., Hofnagel, O., Benz, R. & Raunser, S. Structure of a Tc holotoxin pore provides insights into the translocation mechanism. Proc Natl Acad Sci U S A 116, 23083-23090, doi:10.1073/pnas.1909821116 (2019).
9 Leidreiter, F. et al. Common architecture of Tc toxins from human and insect pathogenic bacteria. Sci Adv 5, eaax6497, doi:10.1126/sciadv.aax6497 (2019).
10 Gatsogiannis, C. et al. Tc toxin activation requires unfolding and refolding of a beta-propeller. Nature 563, 209-213, doi:10.1038/s41586-018-0556-6 (2018).
11 Gatsogiannis, C. et al. Membrane insertion of a Tc toxin in near-atomic detail. Nat Struct MolBiol 23, 884-890, doi:10.1038/nsmb.3281 (2016).
12 Meusch, D. et al. Mechanism of Tc toxin action revealed in molecular detail. Nature 508, 61-65, doi:10.1038/nature13015 (2014).
13 Gatsogiannis, C. et al. A syringe-like injection mechanism in Photorhabdus luminescens toxins. Nature 495, 520-523, doi:10.1038/nature11987 (2013).
14 Piper, S. J. et al. Cryo-EM structures of the pore-forming A subunit from the Yersinia entomophaga ABC toxin. Nat Commun 10, 1952, doi:10.1038/s41467-019-09890-8 (2019).
15 Roderer, D. et al. Glycan-dependent cell adhesion mechanism of Tc toxins. Nat Commun 11, 2694, doi:10.1038/s41467-020-16536-7 (2020).
16 Song, N. et al. N-Glycans and sulfated glycosaminoglycans contribute to the action of diverse Tc toxins on mammalian cells. PLoS Pathog 17, e1009244, doi:10.1371/journal.ppat.1009244 (2021).
17 Ng'ang'a, P. N. et al. Involvement of N-glycans in binding of Photorhabdus luminescens Tc toxin. Cell Microbiol 23, e13326, doi: 10.1111/cmi.13326 (2021).
18 Lang, A. E. et al. Photorhabdus luminescens toxins ADP-ribosylate actin and RhoA to force actin clustering. Science 327, 1139-1142, doi:10.1126/science.1184557 (2010).
19 Yang, G. et al. Pdl1 is a putative lipase that enhances Photorhabdus toxin complex secretion. PLoS Pathog 8, e1002692, doi:10.1371/journal.ppat.1002692 (2012).
20 Yang, G. & Waterfield, N. R. The role of TcdB and TccC subunits in secretion of the Photorhabdus Tcd toxin complex. PLoS Pathog 9, e1003644, doi:10.1371/journal.ppat.1003644 (2013).
21 Silva, C. P. et al. Bacterial infection of a model insect: Photorhabdus luminescens and Manduca sexta. Cell Microbiol 4, 329-339, doi:10.1046/j.1462-5822.2002.00194.x (2002).
22 Gendlina, I. et al. Identification and type III-dependent secretion of the Yersinia pestis insecticidal-like proteins. Mol Microbiol 64, 1214-1227, doi:10.1111/j.1365-2958.2007.05729.x (2007).
23 Hurst, M. R. et al. The main virulence determinant of Yersinia entomophaga MH96 is a broad-host-range toxin complex active against insects. J Bacteriol 193, 1966-1980, doi:10.1128/JB.01044-10 (2011).
24 Grayson, J. M. Digestive Tract pH of Six Species of Coleoptera. Annals of the Entomological Society of America 51, 403-405, doi:10.1093/aesa/51.4.403 (1958).
25 Terra, W. R. & Ferreira, C. Biochemistry and Molecular Biology of Digestion. Insect Molecular Biology and Biochemistry, 365-418, doi:10.1016/B978-0-12-384747-8.10011-X (2012).
26 Hurst, M. R., van Koten, C. & Jackson, T. A. Pathology of Yersinia entomophaga MH96 towards Costelytra zealandica (Coleoptera; Scarabaeidae) larvae. J Invertebr Pathol 115, 102-107, doi:10.1016/j.jip.2013.11.004 (2014).
27 Palmer, T., Finney, A. J., Saha, C. K., Atkinson, G. C. & Sargent, F. A holin/peptidoglycan hydrolase-dependent protein secretion system. Mol Microbiol 115, 345-355, doi:10.1111/mmi.14599 (2021).
28 Young, R. Phage lysis: three steps, three choices, one outcome. J Microbiol 52, 243-258, doi:10.1007/s12275-014-4087-z (2014).
29 Hamilton, J. J. et al. A holin and an endopeptidase are essential for chitinolytic protein secretion in Serratia marcescens. J Cell Biol 207, 615-626, doi:10.1083/jcb.201404127 (2014).
30 Owen, R. A. et al. Structure and activity of ChiX: a peptidoglycan hydrolase required for chitinase secretion by Serratia marcescens. Biochem J 475, 415-428, doi:10.1042/BCJ20170633 (2018).
31 Schoof, M., O'Callaghan, M., Sheen, C. R., Glare, T. R. & Hurst, M. R. H. Identification of genes involved in exoprotein release using a high-throughput exoproteome screening assay in Yersinia entomophaga. PLoS One 17, e0263019, doi:10.1371/journal.pone.0263019 (2022).
32 Berry, J., Rajaure, M., Pang, T. & Young, R. The spanin complex is essential for lambda lysis. J Bacteriol 194, 5667-5674, doi: 10.1128/JB.01245-12 (2012).
33 Starke, M., Richter, M. & Fuchs, T. M. The insecticidal toxin genes of Yersinia enterocolitica are activated by the thermolabile LTTR-like regulator TcaR2 at low temperatures. Molecular Microbiology 89, 596-611, doi:10.1111/mmi.12296 (2013).
34 Cornelis, G. R. et al. ymoA, a Yersinia enterocolitica chromosomal gene modulating the expression of virulence functions. Mol Microbiol 5, 1023-1034, doi:10.1111/j.1365-2958.1991.tb01875.x (1991).
35 Starke, M. & Fuchs, T. M. YmoA negatively controls the expression of insecticidal genes in Yersinia enterocolitica. Mol Microbiol 92, 287-301, doi:10.1111/mmi.12554 (2014).
36 Bohme, K. et al. The Small Protein YmoA Controls the Csr System and Adjusts Expression of Virulence-Relevant Traits of Yersinia pseudotuberculosis. Front Microbiol 12, 706934, doi:10.3389/fmicb.2021.706934 (2021).
37 Ellison, D. W., Young, B., Nelson, K. & Miller, V. L. YmoA negatively regulates expression of invasin from Yersinia enterocolitica. J Bacteriol 185, 7153-7159, doi:10.1128/JB.185.24.7153-7159.2003 (2003).
38 Jackson, M. W., Silva-Herzog, E. & Plano, G. V. The ATP-dependent ClpXP and Lon proteases regulate expression of the Yersinia pestis type III secretion system via regulated proteolysis of YmoA, a small histone-like protein. Mol Microbiol 54, 1364-1378, doi:10.1111/j.1365-2958.2004.04353.x (2004).
39 Dewey, J. S. et al. Micron-scale holes terminate the phage infection cycle. Proc Natl Acad Sci U S A 107, 2219-2223, doi:10.1073/pnas.0914030107 (2010).
40 Grundling, A., Manson, M. D. & Young, R. Holins kill without warning. Proc Natl Acad Sci U S A 98, 9348-9352, doi:10.1073/pnas.151247598 (2001).
41 White, R. et al. Holin triggering in real time. Proc Natl Acad Sci U S A 108, 798-803, doi:10.1073/pnas.1011921108 (2011).
42 Kashket, E. R. The proton motive force in bacteria: a critical assessment of methods. Annu Rev Microbiol 39, 219-242, doi:10.1146/annurev.mi.39.100185.001251 (1985).
43 Michels, M. & Bakker, E. P. Generation of a large, protonophore-sensitive proton motive force and pH difference in the acidophilic bacteria Thermoplasma acidophilum and Bacillus acidocaldarius. J Bacteriol 161, 231-237, doi:10.1128/jb.161.1.231-237.1985 (1985).
44 Tacke, S. et al. A streamlined workflow for automated cryo focused ion beam milling. J Struct Biol 213, 107743, doi:10.1016/j.jsb.2021.107743 (2021).
45 Vollmer, W. & Bertsche, U. Murein (peptidoglycan) structure, architecture and biosynthesis in Escherichia coli. Biochim Biophys Acta 1778, 1714-1734, doi:10.1016/j.bbamem.2007.06.007 (2008).
46 Saier, M. H., Jr. Microcompartments and protein machines in prokaryotes. J Mol Microbiol Biotechnol 23, 243-269, doi:10.1159/000351625 (2013).
47 Frederiksen, R. F. et al. Bacterial chitinases and chitin-binding proteins as virulence factors. Microbiology (Reading) 159, 833-847, doi:10.1099/mic.0.051839-0 (2013).
48 Springer, K. et al. Activity of a Holin-Endolysin System in the Insecticidal Pathogenicity Island of Yersinia enterocolitica. J Bacteriol 200, doi:10.1128/JB.00180-18 (2018).
49 Sänger, P.-A., Wagner, S., Liebler-Tenorio, E. & Fuchs, T. M. Dissecting the invasion of Galleria mellonella by Yersinia enterocolitica reveals metabolic adaptations and a role of a phage lysis cassette in insect killing. bioRxiv, 2022.2006.2027.497489, doi:10.1101/2022.06.27.497489 (2022).
50 Bos, K. I. et al. A draft genome of Yersinia pestis from victims of the Black Death. Nature 478, 506-510, doi:10.1038/nature10549 (2011).
51 Haensch, S. et al. Distinct clones of Yersinia pestis caused the black death. PLoS Pathog 6, e1001134, doi:10.1371/journal.ppat.1001134 (2010).
52 Govind, R. & Dupuy, B. Secretion of Clostridium difficile toxins A and B requires the holin-like protein TcdE. PLoS Pathog 8, e1002727, doi:10.1371/journal.ppat.1002727 (2012).
53 Wydau-Dematteis, S. et al. Cwp19 Is a Novel Lytic Transglycosylase Involved in Stationary-Phase Autolysis Resulting in Toxin Release in Clostridium difficile. mBio 9, doi:10.1128/mBio.00648-18 (2018).
54 Mehner-Breitfeld, D. et al. Evidence for an Adaptation of a Phage-Derived Holin/Endolysin System to Toxin Transport in Clostridioides difficile. Front Microbiol 9, 2446, doi:10.3389/fmicb.2018.02446 (2018).
55 Vidor, C. J. et al. A Highly Specific Holin-Mediated Mechanism Facilitates the Secretion of Lethal Toxin TcsL in Paeniclostridiumsordellii. Toxins (Basel) 14, doi:10.3390/toxins14020124 (2022).
56 Saadat, A. & Melville, S. B. Holin-Dependent Secretion of the Large Clostridial Toxin TpeL by Clostridium perfringens. J Bacteriol 203, doi:10.1128/JB.00580-20 (2021).
57 Geiger, T., Pazos, M., Lara-Tejero, M., Vollmer, W. & Galan, J. E. Peptidoglycan editing by a specific LD-transpeptidase controls the muramidase-dependent secretion of typhoid toxin. Nat Microbiol 3, 1243-1254, doi:10.1038/s41564-018-0248-x (2018).
58 Hurst, M. R. H., Becher, S. A., Young, S. D., Nelson, T. L. & Glare, T. R. Yersinia entomophaga sp. nov., isolated from the New Zealand grass grub Costelytra zealandica. IntJ Syst Evol Microbiol 61, 844-849, doi:10.1099/ijs.0.024406-0 (2011).
59 Bae, S., Park, J. & Kim, J. S. Cas-OFFinder: a fast and versatile algorithm that searches for potential off-target sites of Cas9 RNA-guided endonucleases. Bioinformatics 30, 1473-1475, doi:10.1093/bioinformatics/btu048 (2014).
60 Zhao, D. et al. CRISPR/Cas9-assisted gRNA-free one-step genome editing with no sequence limitations and improved targeting efficiency. Sci Rep 7, 16624, doi:10.1038/s41598-017-16998-8 (2017).
61 Cox, J. & Mann, M. MaxQuant enables high peptide identification rates, individualized p.p.b.-range mass accuracies and proteome-wide protein quantification. Nat Biotechnol 26, 1367-1372, doi:10.1038/nbt,1511 (2008).
62 Tyanova, S. et al. The Perseus computational platform for comprehensive analysis of (prote)omics data. Nat Methods 13, 731-740, doi:10.1038/nmeth.3901 (2016).
63 Goedhart, J. & Luijsterburg, M. S. VolcaNoseR is a web app for creating, exploring, labeling and sharing volcano plots. Sci Rep 10, 20560, doi:10.1038/s41598-020-76603-3 (2020).
64 Teufel, F. et al. SignalP 6.0 predicts all five types of signal peptides using protein language models. Nat Biotechnol, doi:10.1038/s41587-021-01156-3 (2022).
65 Boisvert, S., Raymond, F., Godzaridis, E., Laviolette, F. & Corbeil, J. Ray Meta: scalable de novo metagenome assembly and profiling. Genome Biol 13, R122, doi:10.1186/gb-2012-13-12-r122 (2012).
66 Chen, K. T. & Lu, C. L. CSAR-web: a web server of contig scaffolding using algebraic rearrangements. Nucleic Acids Res 46, W55-W59, doi:10.1093/nar/gky337 (2018).
67 Wang, Z. et al. The molecular basis for sarcomere organization in vertebrate skeletal muscle. Cell 184, 2135-2150 e2113, doi:10.1016/j.cell.2021.02.047 (2021).
68 Mastronarde, D. N. Automated electron microscope tomography using robust prediction of specimen movements. J Struct Biol 152, 36-51, doi:10.1016/j.jsb.2005.07.007 (2005).
69 Hagen, W. J. H., Wan, W. & Briggs, J. A. G. Implementation of a cryo-electron tomography tilt-scheme optimized for high resolution subtomogram averaging. J Struct Biol 197, 191-198, doi:10.1016/j.jsb.2016.06.007 (2017).
70 Tegunov, D. & Cramer, P. Real-time cryo-electron microscopy data preprocessing with Warp. Nat Methods 16, 1146-1152, doi:10.1038/s41592-019-0580-y (2019).
71 Kremer, J. R., Mastronarde, D. N. & McIntosh, J. R. Computer visualization of three-dimensional image data using IMOD. J Struct Biol 116, 71-76, doi: 10.1006/jsbi. 1996.0013 (1996).
72 Tang, G. et al. EMAN2: an extensible image processing suite for electron microscopy. J Struct Biol 157, 38-46, doi:10.1016/j.jsb.2006.05.009 (2007).
73 Buchholz, T. O. et al. Content-aware image restoration for electron microscopy. Methods Cell Biol 152, 277-289, doi:10.1016/bs.mcb.2019.05.001 (2019).
74 Bharat, T. A. & Scheres, S. H. Resolving macromolecular structures from electron cryo-tomography data using subtomogram averaging in RELION. Nat Protoc 11, 2054-2065, doi:10.1038/nprot.2016.124 (2016).
75 Pettersen, E. F. et al. UCSF ChimeraX: Structure visualization for researchers, educators, and developers. Protein Sci 30, 70-82, doi:10.1002/pro.3943 (2021).
76 Cui, J. et al. Design, Synthesis and Evaluation of Triazole-Pyrimidine Analogues as SecA Inhibitors. ChemMedChem 11, 43-56, doi: 10.1002/cmdc.201500447 (2016).
77 Kulp, A. J. et al. Genome-Wide Assessment of Outer Membrane Vesicle Production in Escherichia coli. PLoS One 10, e0139200, doi:10.1371/journal.pone.0139200 (2015).
78 Sedlacek JD, Vail PV. Application and Evaluation of Entomopathogens for Managing Lepidoptera in Stored Products. InField Manual of Techniques in Invertebrate Pathology 2000 (pp. 707-720). Springer, Dordrecht.
79 Roh JY, Choi JY, Li MS, Jin BR, Je YH. Bacillus thuringiensis as a specific, safe, and effective tool for insect pest control. Journal of microbiology and biotechnology. 2007;17(4):547-59.
80 Tabashnik BE, Brévault T, Carrière Y. Insect resistance to Bt crops: lessons from the first billion acres. Nature biotechnology. 2013 Jun;31(6):510-21.
81 Bundesanstalt für Arbeitsschutz und Arbeitsmedizin. TRBA 466 "Einstufung von Prokaryonten (Bacteria und Archaea) in Risikogruppen". Ausgabe 2015, GMB1. 2015, Nr. 46-50 vom 25.8.2015, 9. Änderung vom 21.12.2020, GMB1. Nr. 51.
82 Jones SA, Hurst MR. Purification of the Yersinia entomophaga Yen-TC toxin complex using size exclusion chromatography. In: Glare, T., Moran-Diez, M. (eds) Microbial-Based Biopesticides. Methods Mol Biol. 1477, 39-48, doi: 10.1007/978-1-4939-6367-6_4(2016)

## Claims

1. A *Yersinia entomophaga* bacterium comprising a modified YenR gene.

2. The *Yersinia entomophaga* bacterium of claim 1,
wherein the expression of the YenR gene or gene products thereof is increased, optionally wherein the expression of the YenR gene or gene products thereof is directly or indirectly increased, or
wherein the *Yersinia entomophaga* bacterium is capable of having the expression of the YenR gene or gene products increased, optionally wherein the *Yersinia entomophaga* bacterium is capable of having the expression of the YenR gene or gene products thereof directly or indirectly increased.

3. The *Yersinia entomophaga* bacterium of claim 1 or 2, wherein the promotor region of the YenR gene comprises an inducible promotor or a high expressing promotor or regulatory elements, optionally, wherein the YenR gene comprises an arabinose-inducible promotor or regulatory element, a lactose/IPTG-inducible promotor or regulatory element, an anhydrotetracycline-inducible promotor or regulatory element, or a rhamnose-inducible promoter or regulatory element, preferably an arabinose-inducible promotor or regulatory element.

4. The *Yersinia entomophaga* bacterium of any one of claims 1-3, comprising the nucleotide sequence set forth in SEQ ID NO: 1 and/or SEQ ID NO: 2.

5. The *Yersinia entomophaga* bacterium of any one of claims 1-4, further comprising a modified YmoA gene, optionally comprising the nucleotide sequence set forth in SEQ ID NO: 3.

6. The *Yersinia entomophaga* bacterium of any one of claims 1-5, further comprising a disrupted YenDF, wherein the disruption comprises a deletion of the YeHln, YeEln, YeIspn and/or YeOspn gene or within the YeHln, YeEln, YeIspn and/or YeOspn gene.

7. The *Yersinia entomophaga* bacterium of any one of claims 1-6, further comprising a modified gene encoding a YenTc subunit or a modified YenTc subunit, such as a modified YenA2 or a modified YenA2 gene, optionally, wherein the modified YenA2 comprises an internal, N-terminal and/or C-terminal purification tag, and/or, wherein the modified YenA2 gene comprises a sequence encoding a purification tag.

8. A toxin composition comprising Cbp, Chi1, Chi2, Chi3, NucA, Pil36, PirA, PirB, StcE, Tlh, YenAl, YenA2, YenB, YenC1, YenC2, and RHS2.

9. A method to produce a toxin composition comprising a step of culturing a *Yersinia entomophaga* bacterium of any one of claims 1-7.

10. The method of claim 9, further comprising a secretion step, wherein the secretion step comprises
(i) increasing the pH of the initial culture from pH 5.5-6.0 to pH 6.3-10.0 or transferring the bacterium to a pH 6.3-10.0 buffer and/or
(ii) incubating the *Yersinia entomophaga* bacterium at pH 6.3-10.0, and/or optionally
(iii) a lysis step comprising mechanical lysis or enzymatic lysis of the *Yersinia entomophaga* bacterium, optionally wherein the lysis step is performed within an insect host.

11. The method of claim 9 or 10, further comprising a purification step, wherein the purification step comprises
(i) centrifuging the *Yersinia entomophaga* bacterium, and/or optionally
(ii) filtrating the *Yersinia entomophaga* bacterium, and/or optionally
(iii) purifying a toxin composition, and/or optionally
(iv) purifying a YenTc toxin via an affinity purification, optionally comprising a bead-based purification, wherein the beads show high affinity to a modified YenTc subunit.

12. A YenTc toxin or a subunit of a YenTc toxin comprising a purification tag.

13. A population of soldier cells, wherein the soldier cells are *Yersinia entomophaga* bacteria, wherein the expression of the YenR gene or gene products thereof is increased, optionally, wherein the expression of the YmoA gene or gene products thereof is increased and/or wherein the soldier cells comprise the toxin composition and/or one or more of the toxin(s) according to claim 8, preferably, wherein the population comprises the *Yersinia entomophaga* bacterium of any one of claims 1-7,
optionally wherein the expression of the YenR gene or gene products thereof is directly or indirectly increased, further optionally, wherein the expression of the YmoA gene or gene products thereof is directly or indirectly increased.

14. A method to kill pests comprising contacting a pest with a *Yersinia entomophaga* bacterium of any one of claims 1-7, or the population of soldier cells of claim 13, or the toxin composition of claim 8, or a YenTc toxin of claim 12.

15. A modified YenR gene comprising the sequence set forth in SEQ ID NO: 2.
